# EUROPEAN PATENT SPECIFICATION

(11) **EP 0 573 218 B1**
(45) Date of publication and mention of the grant of the patent: **07.03.2001**
(21) Application number: 93304131.1
(22) Date of filing: 27.05.1993
(51) Int. Cl.: C07D 233/90, A61K 31/415, C07D 403/10

(54) **4-Carboxyimidazoles as angiotensin II antagonists and their thrapeutic use**
4-Carboxyimidazolderivate als Angiotensin-II-Antagonisten und ihre therapeutische Verwendung
Dérivés de 4-carboxyimidazole comme antagonistes de l'angiotensine II et leur application en thérapeutique

(30) Priority: 02.06.1992 JP 14116092
(43) Date of publication of application: 08.12.1993
(73) Proprietor: SANKYO COMPANY LIMITED, Chuo-ku, Tokyo 103-0023 (JP)
(72) Inventor: Yanagisawa, Hiroaki, c/o Sankyo Company Limited, Shinagawa-ku, Tokyo 140 (JP); Shimoji, Yasuo, c/o Sankyo Company Limited, Shinagawa-ku, Tokyo 140 (JP); Amomiya, Yoshiya, c/o Sankyo Company Limited, Shinagawa-ku, Tokyo 140 (JP); Kanazaki, Takuro, c/o Sankyo Company Limited, Shinagawa-ku, Tokyo 140 (JP); Koike, Hiroyuki, c/o Sankyo Company Limited, Shinagawa-ku, Tokyo 140 (JP); Sada, Toshio, c/o Sankyo Company Limited, Shinagawa-ku, Tokyo 140 (JP)
(74) Representative: Gibson, Christian John Robert

(56) References cited:
- EP-A- 0 253 310
- EP-A- 0 324 377
- EP-A- 0 465 368
- EP-A- 0 503 785
- EP-A- 0 505 098

## Description

The present invention relates to a series of new imidazole derivatives which are antagonists to angiotensin II (hereinafter abbreviated as "AII" ) and which can therefore be used for the treatment and prophylaxis of diseases arising from hypertension and in the treatment or prohylaxis of cardiovascular diseases. The invention also provides methods and compositions using these new compounds as well as processes for their preparation.

It is known that the renin-angiotensin system provides one of the important mechanisms for maintaining the homeostasis of blood pressure in living animals. When blood pressure is reduced or the sodium ion concentration of the body fluids falls, this system is activated. As a result, the enzyme renin and angiotensin converting enzyme (hereinafter abbreviated, as is conventional, as "ACE") are activated and act on angiotensinogen, which is first decomposed by the renin to produce angiotensin I (hereinafter abbreviated as "AI"). This AI is then converted by ACE to AII. Since AII induces strong contractions of blood vessels and accelerates the secretion of aldosterone (which is a hormone produced by the adrenal glands that controls the excretion of sodium by the kidneys and thereby maintains the balance of salt and water in the body fluids) the activation of the system results in an elevation of blood pressure. Inhibitors or suppressors of the renin-angiotensin system, such as renin inhibitors, ACE inhibitors and AII antagonists, dilate blood vessels, cause lower blood pressure and improve the circulatory function, which is the basis for the use of these agents in the treatment of cardiovascular diseases.

At present only ACE inhibitors are used clinically, although both renin inhibitors and AII antagonists are under investigation for such use. Of these, some peptide type AII antagonists, such as saralasin, have been known for many years, whilst certain non-peptide type antagonists have only recently been discovered (for example, as disclosed in European Patent Publications No. 28 833, 28 834, 245 637, 253 310, 323 841, 324 377 and 492 105, and in Japanese Patent Application Kokai No. Sho 57-98270). However, the closest prior art is believed to be European Patent Publication No. 324 377 and German Patent Publication No. 4 036 706.

European Patent Publication No. 324 377 discloses a series of 1-(substituted phenyl)-, 1-(substituted phenethyl)- or 1-(substituted benzyl)- imidazole derivatives which are said to have the ability to inhibit the activity of AII. Included in the scope of these prior art compounds are a number of 1-biphenylmethylimidazole derivatives, which, however, differ from the compounds of the present invention in the nature of the substituent at the imidazole 4-position.

German Patent Publication No. 4 036 706 also discloses a series of such compounds, differing from the compounds of the present invention in a similar manner. The activities of all of these prior art compounds, however, including those of European Patent Publication No. 324 377 and German Patent Publication No. 4 036 706, are not sufficient and more potent AII antagonists are sought for better clinical results.

We have now discovered a limited series of 1-(biphenylmethyl)imidazole-5-carboxylic acid derivatives having certain specific substituents at the imidazole 4-position and which, as a result, have an excellent All receptor antagonist activity, and are therefore useful as anti-hypertensive drugs and for the therapy and prophylaxis of cardiovascular diseases.

Thus, in accordance with the present invention, there are provided compounds of formula (I): 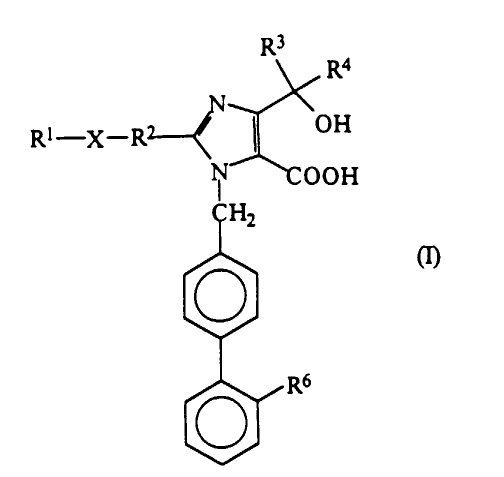 in which:
R¹ represents a hydrogen atom, an alkyl group having from 1 to 6 carbon atoms, a cycloalkyl group having from 3 to 6 ring carbon atoms or an alkanoyl group having from 1 to 6 carbon atoms;
R² represents a single bond or an alkylene or alkylidene group having from 1 to 4 carbon atoms;
R³ and R⁴ are the same or different and each represents an alkyl group having from 1 to 6 carbon atoms;
R⁶ represents a carboxy group or a tetrazol-5-yl group; and
X represents an oxygen or sulphur atom;
and pharmaceutically acceptable salts and esters thereof.

The invention also provides a pharmaceutical composition for the treatment or prophylaxis of hypertension or of a cardiovascular disease, which comprises an effective amount of an anti-hypertensive agent in admixture with a pharmaceutically acceptable carrier or diluent, wherein the anti-hypertensive agent is selected from compounds of formula (I) and pharmaceutically acceptable salts and esters thereof.

The invention further provides compounds of formula (I) and pharmaceutically acceptable salts and esters thereof, for use as a pharmaceutical, particularly for use in the treatment or prophylaxis of hypertension or of a cardiovascular disease.

The invention yet further provides the use of a compound of formula (I), or a pharmaceutically acceptable salt or ester thereof, in the manufacture of a medicament for the prophylaxis or treatment of hypertension and/or cardiovascular diseases.

The invention still further provides processes for the preparation of compounds of formula (I) and pharmaceutically acceptable salts and esters thereof, which are described in more detail hereafter.

Where R¹, R³ or R⁴ represents an alkyl group having from 1 to 6 carbon atoms, this may be a straight or branched chain group having from 1 to 6 carbon atoms, and examples include the methyl, ethyl, propyl, isopropyl, butyl, isobutyl, sec-butyl, t-butyl, pentyl, isopentyl, neopentyl, t-pentyl, 2-methylbutyl, 1-ethylpropyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl, hexyl and isohexyl groups. Of these, we prefer those alkyl groups having from 1 to 4 carbon atoms, preferably the methyl, ethyl, propyl, isopropyl, butyl and isobutyl groups, more preferably the methyl and ethyl groups, and most preferably the methyl group.

Where R¹ represents a cycloalkyl group, this has from 3 to 6 ring carbon atoms, and examples include the cyclopropyl, cyclobutyl, cyclopentyl and cyclohexyl groups, preferably the cyclopropyl group.

Where R¹ represents an alkanoyl group having from 1 to 6 carbon atoms, this may be a straight or branched chain group having from 1 to 6 carbon atoms, and examples include the formyl, acetyl, propionyl, butyryl, isobutyryl, pivaloyl, valeryl, isovaleryl and hexanoyl groups, of which we prefer the acetyl and propionyl groups, most preferably the acetyl group.

Where R² represents an alkylene or alkylidene group, this is a bivalent saturated aliphatic hydrocarbon group having from 1 to 4 carbon atoms. Where the two "free" valencies are on the same carbon atom, the group is generally referred to as an "alkylidene" group; where they are on different carbon atoms, it is commonly referred to as an "alkylene" group. The term "alkylene" is also often used to embrace both types of group. Examples of such groups include the methylene, ethylene, trimethylene, propylene, ethylethylene, tetramethylene, ethylidene, propylidene, butylidene and isobutylidene groups, of which those groups having 1 or 2 carbon atoms are preferred, particularly the methylene group.

The compounds of the present invention contain a carboxy group at the 5-position of the imidazole group and may contain another carboxy group if this is the meaning of R⁶. These groups can, of course, form esters. There is no particular restriction on the nature of the ester group, provided that, where the compound is intended for therapeutic purposes, it is pharmaceutically acceptable (i.e. it is not less active, or unacceptably less active than the free acid, and it is not more toxic, or unacceptably more toxic, than the free acid). Where, however, the compound is intended for non-therapeutic purposes, for example as an intermediate in the preparation of other, and possibly more active, compounds, even this restriction does not apply. In general, however, any protecting group commonly used in the field of synthetic organic chemistry or any ester group capable of conversion to a carboxy group under physiological conditions, to form a pro-drug, may be used.

The compounds of formula (I) and their esters may collectively be represented by the formula (Ia): 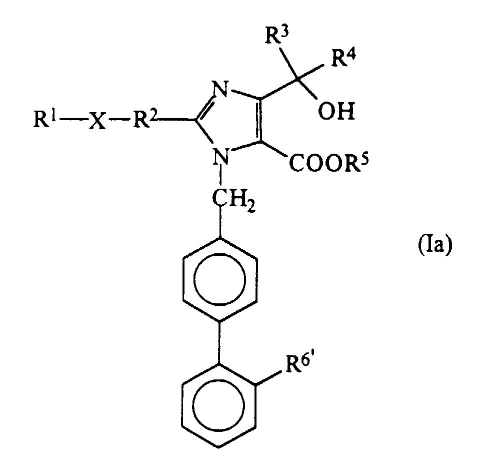 (in which: R¹, R², R³, R⁴ and X are as defined above; R⁵ represents a hydrogen atom or an ester group; and R^{6'} represents a carboxy group, an esterified carboxy group or a tetrazol-5-yl group).

Examples of such ester groups which may be represented by R⁵ or may be included in the esterified carboxy group represented by R^{6'} include:
alkyl groups having from 1 to 6 carbon atoms, such as those exemplified above in relation to R¹ etc.;
haloalkyl groups having from 1 to 6 carbon atoms, such as the fluoromethyl, trifluoromethyl, trichloromethyl, 2,2,2-trifluoroethyl, 2,2,2-trichloroethyl, 2-fluoroethyl, 2-chloroethyl, 2-iodoethyl, 3-chloro- propyl, 4-fluorobutyl and 6-iodohexyl groups, of which we prefer the 2,2,2-trichloroethyl and 2-chloroethyl groups;
hydroxyalkyl groups having from 1 to 6 carbon atoms and having at least one, and preferably 1 or 2, hydroxy groups, such as the 2-hydroxyethyl, 2,3-dihydroxypropyl, 3-hydroxy- propyl, 3,4-dihydroxybutyl and 4-hydroxybutyl groups, of which we prefer the 2-hydroxyethyl group;
alkoxyalkyl and alkoxyalkoxyalkyl groups, in which the or each alkoxy part has from 1 to 6 carbon atoms and the alkyl part has from 1 to 6 carbon atoms, for example the methoxymethyl, 2-methoxyethyl, 2-ethoxyethyl, 3-methoxypropyl, 4-methoxybutyl, propoxymethyl, butoxymethyl and 2-methoxyethoxymethyl groups, of which we prefer the methoxymethyl group;
the phenacyl group;
alkoxycarbonylalkyl groups, in which the alkoxy part has from 1 to 8 carbon atoms and the alkyl part has from 1 to 6 carbon atoms, such as the methoxycarbonylmethyl, ethoxycarbonylmethyl, propoxycarbonylmethyl, isopropoxycarbonylmethyl, butoxycarbonylmethyl, t-butoxycarbonylmethyl, pentyloxycarbonylmethyl, hexyloxycarbonylmethyl, heptyloxycarbonylmethyl, octyloxycarbonylmethyl, 2-methoxycarbonylethyl, 2-ethoxycarbonylethyl, 2-propoxycarbonylethyl, 2-isopropoxycarbonylethyl, 2-butoxycarbonylethyl, 2-t-butoxycarbonylethyl, 2-pentyloxycarbonylethyl, 2-hexyloxycarbonylethyl, 2-heptyloxycarbonylethyl, 2-octyloxycarbonylethyl, 3-methoxycarbonylpropyl, 3-ethoxycarbonylpropyl, 4-methoxycarbonylbutyl, 4-ethoxycarbonylbutyl, 5-methoxycarbonylpentyl, 5-ethoxycarbonylpentyl, 6-methoxycarbonylhexyl and 6-ethoxycarbonylhexyl groups, of which the methoxycarbonylmethyl group is preferred;
cyanoalkyl groups, in which the alkyl part has from 1 to 6 carbon atoms, such as the cyanomethyl, 2-cyanoethyl, 3-cyanopropyl, 4-cyanobutyl, 5-cyanopentyl and 6-cyanohexyl groups, of which the cyanomethyl and 2-cyanoethyl groups are preferred;
alkylthiomethyl groups, in which the alkyl part has from 1 to 6 carbon atoms, such as the methylthiomethyl, ethylthiomethyl, propylthiomethyl, butylthiomethyl, pentylthiomethyl and hexylthiomethyl groups, of which the methylthiomethyl and ethylthiomethyl groups are preferred;
arylthiomethyl groups, in which the aryl part is a carbocyclic aromatic ring having from 6 to 10 ring carbon atoms and is unsubstituted or substituted, preferably unsubstituted, for example the phenylthiomethyl and naphthylthiomethyl groups;
alkanesulphonylalkyl groups, in which each alkyl part (which may be the same as each other or different from each other) has from 1 to 6 carbon atoms and in which the alkane part is unsubstituted or substituted by at least one halogen atom, for example the 2-methanesulphonylethyl and 2-trifluoromethanesulphonylethyl groups;
arylsulphonylalkyl groups, in which the aryl part has from 6 to 10 ring carbon atoms and the alkyl part has from 1 to 6 carbon atoms, and where the aryl part is unsubstituted or is substituted, preferably by at least one alkyl group, for example the 2-benzenesulphonylethyl, 2-(1-naphthalenesulphonyl)ethyl, 2-p-toluenesulphonylethyl, 3-benzenesulphonylpropyl, 3-(1-naphthalenesulphonyl)-propyl, 3-p-toluenesulphonylpropyl, 6-benzenesulphonylhexyl, 6-(1-naphthalenesulphonyl)hexyl, 6-p-toluenesulphonylhexyl, benzenesulphonylmethyl and p-toluenesulphonylmethyl groups, and preferably the 2-benzenesulphonylethyl and 2-p-toluenesulphonylethyl groups;
aralkyl groups, in which an alkyl group having from 1 to 6 carbon atoms is substituted by at least one (and preferably from 1 to 3) aryl groups which have from 6 to 10 ring carbon atoms and which are unsubstituted or are substituted, preferably unsubstituted; examples include the benzyl, diphenylmethyl, triphenylmethyl, 1-naphthylmethyl, 2-naphthylmethyl, phenethyl, 1-phenylethyl, 3-phenylpropyl, 2-phenylpropyl, 1-phenylpropyl, 4-phenylbutyl, 5-phenylpentyl and 6-phenylhexyl groups, of which the benzyl, diphenylmethyl and 1-naphthylmethyl groups are preferred and the benzyl group is most preferred;
aryl groups having from 6 to 10, preferably 6 or 10, ring carbon atoms, which may be unsubstituted or substituted (preferably unsubstituted), for example the phenyl and naphthyl groups, of which the phenyl group is preferred;
alkanoyloxyalkyl groups, in which the alkanoyl and alkyl parts both have from 1 to 6 carbon atoms, for example the formyloxymethyl, acetoxymethyl, propionyloxymethyl, butyryloxymethyl, pivaloyloxymethyl, valeryloxymethyl, isovaleryloxymethyl, hexanoyloxymethyl, 1-formyloxyethyl, 1-acetoxyethyl, 1-propionyloxyethyl, 1-butyryloxyethyl, 1-pivaloyloxyethyl, 1-valeryloxyethyl, 1-isovaleryloxyethYl, 1-hexanoyloxyethyl, 2-formyloxyethyl, 2-acetoxyethyl, 2-propionyloxyethyl, 2-butyryloxyethyl, 2-pivaloyloxyethyl, 2-valeryloxyethyl, 2-isovaleryloxyethyl, 2-hexanoyloxyethyl, 1-formyloxypropyl, 1-acetoxypropyl, 1-propionyloxypropyl, 1-butyryloxypropyl, 1-pivaloyloxypropyl, 1-valeryoxypropyl, 1-isovaleryloxypropyl, 1-hexanonyloxypropyl, 1-acetoxybutyl, 1-propionyloxybutyl, 1-butyryloxybutyl, 1-pivaloyloxybutyl, 1-acetoxypentyl, 1-propionyloxypentyl, 1-butyryloxypentyl, 1-pivaloyloxypentyl and 1-pivaloyloxyhexyl groups, of which we prefer the formyloxymethyl, acetoxymethyl, propionyloxymethyl, butyryloxymethyl, pivaloyloxymethyl, 1-formyloxyethyl, 1-acetoxyethyl, 1-propionyloxyethyl, 1-butyryloxyethyl and 1-pivaloyloxyethyl groups and more prefer the acetoxymethyl, propionyloxymethyl, butyryloxymethyl, pivaloyloxymethyl, 1-acetoxyethyl, 1-propionyloxyethyl, 1-butyryloxyethyl and 1-pivaloyloxyethyl groups, the pivaloyloxymethyl and 1-pivaloyloxyethyl groups being most preferred;
cycloalkanecarbonyloxyalkyl groups, in which the cycloalkane part has 5 or 6 ring carbon atoms and the alkyl part has from 1 to 6 carbon atoms, for example the cyclopentanecarbonyloxymethyl, cyclohexanecarbonyloxymethyl, 1-cyclopentanecarbonyloxyethyl, 1-cyclohexanecarbonyloxyethyl, 1-cyclopentanecarbonyloxypropyl, 1-cyclohexanecarbonyloxypropyl, 1-cyclopentanecarbonyloxybutyl and 1-cyclohexanecarbonyloxybutyl groups, preferably the cyclopentanecarbonyloxymethyl, cyclohexanecarbonyloxymethyl, 1-cyclopentanecarbonyloxyethyl and 1-cyclohexanecarbonyloxyethyl groups;
alkoxycarbonyloxyalkyl groups, in which the alkoxy and alkyl parts both have from 1 to 6 carbon atoms, for example the methoxycarbonyloxymethyl, ethoxycarbonyloxymethyl, propoxycarbonyloxymethyl, isopropoxycarbonyloxymethyl, butoxycarbonyloxymethyl, isobutoxycarbonyloxymethyl, pentyloxycarbonyloxymethyl, hexyloxycarbonyloxymethyl, 1-methoxycarbonyloxyethyl, 1-ethoxycarbonyloxyethyl, 1-propoxycarbonyloxyethyl, 1-isopropoxycarbonyloxyethyl, 1-butoxycarbonyloxyethyl, 1-isobutoxycarbonyloxyethyl, 1-pentyloxycarbonyloxyethyl, 1-hexyloxycarbonyloxyethyl, 2-methoxycarbonyloxyethyl, 2-ethoxycarbonyloxyethyl, 2-propoxycarbonyloxyethyl, 2-isopropoxycarbonyloxyethyl, 2-butoxycarbonyloxyethyl, 2-isobutoxycarbonyloxyethyl, 2-pentyloxycarbonyloxyethyl, 2-hexyloxycarbonyloxyethyl, 1-methoxycarbonyloxypropyl, 1-ethoxycarbonyloxypropyl, 1-propoxycarbonyloxypropyl, 1-isopropoxycarbonyloxypropyl, 1-butoxycarbonyloxypropyl, 1-isobutoxycarbonyloxypropyl, 1-pentyloxycarbonyloxypropyl, 1-hexyloxycarbonyloxypropyl, 1-methoxycarbonyloxybutyl, 1-ethoxycarbonyloxybutyl, 1-propoxycarbonyloxybutyl, 1-isopropoxycarbonyloxybutyl, 1-butoxycarbonyloxybutyl, 1-isobutoxycarbonyloxybutyl, 1-methoxycarbonyloxypentyl, 1-ethoxycarbonyloxypentyl, 1-methoxycarbonyloxyhexyl and 1-ethoxycarbonyloxyhexyl groups, of which we prefer the methoxycarbonyloxymethyl, ethoxycarbonyloxymethyl, propoxycarbonyloxymethyl, isopropoxycarbonyloxymethyl, butoxycarbonyloxymethyl, isobutoxycarbonyloxymethyl, 1-methoxycarbonyloxyethyl, 2-pentyloxycarbonyloxyethyl, 1-propoxycarbonyloxyethyl, 1-isopropoxycarbonyloxyethyl, 1-butoxycarbonyloxypropyl, 1-isobutoxycarbonyloxyethyl, 1-methoxycarbonyloxypropyl, 1-ethoxycarbonyloxypropyl, 1-propoxycarbonyloxypropyl, 1-isopropoxycarbonyloxypropyl, 1-butoxycarbonyloxypropyl, 1-isobutoxycarbonyloxypropyl, 1-methoxycarbonyloxybutyl, 1-ethoxycarbonyloxybutyl, 1-propoxycarbonyloxybutyl, 1-isopropoxycarbonyloxybutyl, 1-butoxycarbonyloxybutyl and 1-isobutoxycarbonyloxybutyl groups, and more prefer the methoxycarbonyloxymethyl, ethoxycarbonyloxymethyl, propoxycarbonyloxymethyl, isopropoxycarbonyloxymethyl, butoxycarbonyloxymethyl, isobutoxycarbonyloxymethyl, 1-methoxycarbonyloxyethyl, 1-ethoxycarbonyloxyethyl, 1-propoxycarbonyloxyethyl, 1-isopropoxycarbonyloxyethyl, 1-butoxycarbonyloxyethyl and 1-isobutoxycarbonyloxyethyl groups, the methoxycarbonyloxymethyl, ethoxycarbonyloxymethyl, isopropoxycarbonyloxymethyl, 1-methoxycarbonyloxyethyl, 1-ethoxycarbonyloxyethyl and 1-isopropoxycarbonyloxyethyl groups being most preferred;
cycloalkyloxycarbonyloxyalkyl groups, in which the cycloalkyl part has 5 or 6 ring carbon atoms and the alkyl part has from 1 to 6 carbon atoms, for example the cyclopentyloxycarbonyloxymethyl, cyclohexyloxycarbonyloxymethyl, 1-cyclopentyloxycarbonyloxyethyl, 1-cyclohexyloxycarbonyloxyethyl, 1-cyclopentyloxycarbonyloxypropyl, 1-cyclohexyloxycarbonyloxypropyl, 1-cyclopentyloxycarbonyloxybutyl and 1-cyclohexyloxycarbonyloxybutyl groups, of which we prefer the cyclopentyloxycarbonyloxymethyl, cyclohexyloxycarbonyloxymethyl, 1-cyclopentyloxycarbonyloxyethyl and 1-cyclohexyloxycarbonyloxyethyl groups;
[5-(aryl or alkyl)-2-oxo-1,3-dioxolen-4-yl]methyl groups, in which the aryl group is a carbocyclic aromatic group having from 6 to 10, preferably 6 or 10, ring carbon atoms (and is substituted, preferably with a halogen atom, an alkyl group or an alkoxy group, or unsubstituted, preferably unsubstituted), and the alkyl group has from 1 to 6 carbon atoms, for example the (5-phenyl-2-oxo-1,3-dioxolen-4-yl)methyl, [5-(4-methylphenyl)-2-oxo-1,3-dioxolen-4-yl]methyl, [5-(4-methoxyphenyl)-2-oxo-1,3-dioxolen-4-yl]methyl, [5-(4-fluorophenyl)-2-oxo-1,3-dioxolen-4-yl]methyl, [5-(4-chlorophenyl)-2-oxo-1,3-dioxolen-4-yl]methyl, (5-methyl-2-oxo-1,3-dioxolen-4-yl)methyl, (5-ethyl-2-oxo-1,3-dioxolen-4-yl)methyl, (5-propyl-2-oxo-1,3-dioxolen-4-yl)-methyl, (5-isopropyl-2-oxo-1,3-dioxolen-4-yl)methyl and (5-butyl-2-oxo-1,3-dioxolen-4-yl)methyl groups, of which we prefer the (5-phenyl-2-oxo-1,3-dioxolen-4-yl)methyl, (5-methyl-2-oxo-1,3-dioxolen-4-yl)-methyl and (5-ethyl-2-oxo-1,3-dioxolen-4-yl)methyl groups, and more prefer the (5-methyl-2-oxo-1,3-dioxolen-4-yl)methyl group; and
the phthalidyl group.

In the above groups, where an aryl group is referred to as substituted, examples of suitable substituents include:
alkyl groups having from 1 to 6 carbon atoms, such as those exemplified above in relation to R¹ etc.;
alkoxy groups having from 1 to 6 carbon atoms, such as the methoxy, ethoxy, propoxy, isopropoxy, t-butoxy, pentyloxy and hexyloxy groups;
halogen atoms, such as the fluorine, chlorine, bromine and iodine atoms;
preferably alkyl groups having from 1 to 4 carbon atoms, alkoxy groups having from 1 to 4 carbon atoms, and florine, chlorine or bromine atoms, most preferably a methyl, ethyl, methoxy or ethoxy group, or a fluorine or chlorine atom.

Examples of such preferred ester groups include:
alkyl groups having from 1 to 4 carbon atoms;
phenyl groups which are unsubstituted or are substituted by at least one substituent selected from methyl groups, ethyl groups, methoxy groups, ethoxy groups, fluorine atoms and chlorine atoms;
naphthyl groups;
benzyl groups which are unsubstituted or are substituted by at least one substituent selected from methyl groups, ethyl groups, methoxy groups, ethoxy groups, fluorine atoms and chlorine atoms;
diphenylmethyl groups;
naphthylmethyl groups;
alkanoyloxyalkyl groups in which the alkanoyl part has from 1 to 5 carbon atoms and the alkyl part has from 1 to 4 carbon atoms;
cycloalkanecarbonyloxyalkyl groups in which the cycloalkane part has 5 or 6 ring carbon atoms and the alkyl part has from 1 to 4 carbon atoms;
alkoxycarbonyloxyalkyl groups in which the alkoxy and alkyl parts both have from 1 to 4 carbon atoms;
cycloalkyloxycarbonyloxyalkyl groups in which the cycloalkyl part has 5 or 6 ring carbon atoms and the alkyl part has from 1 to 4 carbon atoms;
[5-phenyl- or 5-alkyl- 2-oxo-1,3-dioxolen-4-yl]-methyl groups in which the alkyl part has from 1 to 4 carbon atoms; and
the phthalidyl group.

Still more preferred ester groups include:
alkyl groups having from 1 to 4 carbon atoms;
the benzyl group;
alkanoyloxyalkyl groups in which the alkanoyl part has from 1 to 5 carbon atoms and the alkyl part has 1 or 2 carbon atoms;
cycloalkanecarbonyloxyalkyl groups in which the cycloalkane part has 5 or 6 ring carbon atoms and the alkyl part has 1 or 2 carbon atoms;
alkoxycarbonyloxyalkyl groups in which the alkoxy part has from 1 to 4 carbon atoms and the alkyl part has 1 or 2 carbon atoms;
cycloalkyloxycarbonyloxyalkyl groups in which the cycloalkane part has 5 or 6 ring carbon atoms and the alkyl part has 1 or 2 carbon atoms;
[5-phenyl-, 5-methyl- or 5-ethyl- 2-oxo-1,3-dioxolen-4-yl]methyl groups; and
the phthalidyl group.

The most preferred ester groups include the pivaloyloxymethyl, ethoxycarbonyloxymethyl, 1-(ethoxy-carbonyloxy)ethyl, isopropoxycarbonyloxymethyl, 1-(isopropoxycarbonyloxy)ethyl, (5-methyl-2-oxo-1,3-dioxolen-4-yl)methyl and phthalidyl groups.

The compounds of the present invention can also form salts. Examples of such salts include: salts with an alkali metal, such as sodium, potassium or lithium; salts with an alkaline earth metal, such as magnesium, barium or calcium; salts with another metal, such as aluminium; ammonium salts; organic base salts, such as a salt with triethylamine, diisopropylamine, guanidine or dicyclohexylamine; and salts with a basic amino acid, such as lysine or arginine. Also, since the compound of the present invention contains a basic nitrogen atom in its molecule, it can form acid addition salts. Examples of such acid addition salts include: salts with mineral acids, especially hydrohalic acids (such as hydrofluoric acid, hydrobromic acid, hydroiodic acid or hydrochloric acid), nitric acid, carbonic acid, sulphuric acid or phosphoric acid; salts with lower alkylsulphonic acids, such as methanesulphonic acid, trifluoromethanesulphonic acid or ethanesulphonic acid; salts with arylsulphonic acids, such as benzenesulphonic acid or p-toluenesulphonic acid; salts with organic carboxylic acids, such as acetic acid, fumaric acid, tartaric acid, oxalic acid, maleic acid, malic acid, succinic acid, benzoic acid, mandelic acid, ascorbic acid, lactic acid, gluconic acid or citric acid; and salts with acidic amino acids, such as glutamic acid or aspartic acid.

The compounds of the present invention may contain one or more asymmetric carbon atoms in their molecules, and can, in such a case, form optical isomers. Although these are all represented herein by a single molecular formula, the present invention includes both the individual, isolated isomers and mixtures, including racemates thereof. Where stereospecific synthesis techniques are employed or optically active compounds are employed as starting materials, individual isomers may be prepared directly; on the other hand, if a mixture of isomers is prepared, the individual isomers may be obtained by conventional resolution techniques.

Of the compounds of the present invention, we prefer those compounds of formula (I) or (Ia) and salts and (where appropriate) esters thereof, in which:
(A) R¹ represents a hydrogen atom, a methyl group, an ethyl group, a cyclopropyl group or an acetyl group, particularly a methyl or ethyl group;
(B) R² represents a single bond, a methylene group, an ethylene group or an ethylidene group;
(C) R³ and R⁴ are the same or different and each represents a methyl group or an ethyl group,
(D) R⁵ represents
   a hydrogen atom,
   an alkyl group having from 1 to 4 carbon atoms,
   a phenyl group,
   a phenyl group substituted by at least one substituent selected from methyl groups, ethyl groups, methoxy groups, ethoxy groups, fluorine atoms and chlorine atoms,
   a naphthyl group,
   a benzyl group,
   a benzyl group substituted by at least one substituent selected from methyl groups, ethyl groups, methoxy groups, ethoxy groups, fluorine atoms and chlorine atoms,
   a diphenylmethyl group,
   a naphthylmethyl group,
   an alkanoyloxyalkyl group in which the alkanoyl part has from 1 to 5 carbon atoms and the alkyl part has from 1 to 4 carbon atoms,
   a cycloalkanecarbonyloxyalkyl group in which the cycloalkane part has 5 or 6 carbon atoms and the alkyl part has from 1 to 4 carbon atoms,
   an alkoxycarbonyloxyalkyl group in which the alkoxy and alkyl parts each have from 1 to 4 carbon atoms,
   a cycloalkyloxycarbonyloxyalkyl group in which the cycloalkyl part has 5 or 6 carbon atoms and the alkyl part has from 1 to 4 carbon atoms,
   a (5-phenyl-2-oxo-1,3-dioxolen-4-yl)methyl group,
   a (5-alkyl-2-oxo-1,3-dioxolen-4-yl)methyl group,
   in which the alkyl part has from 1 to 4 carbon atoms, or
   a phthalidyl group;
(E) R^{6'} represents a carboxy group or a tetrazol-5-yl group.

We particularly prefer those compounds of formula (Ia) and salts and esters thereof in which R¹ is as defined in (A) above, R² is as defined in (B) above, R³ and R⁴ are as defined in (C) above, R⁵ is as defined in (D) above and R^{6'} is as defined in (E) above.

More preferred compounds of the present invention are those compounds of formula (I) or (Ia) and salts and (where appropriate) esters thereof, in which:
(F) the group of formula R¹-X-R²- represents a methoxymethyl group, an ethoxymethyl group, a 1-methoxyethyl group, a 2-methoxyethyl group, a 2-ethoxyethyl group, a methylthiomethyl group, an ethylthiomethyl group, a 1-methylthioethyl group, 2-methylthioethyl, a 2-ethylthioethyl group, a methylthio group or an ethylthio group;
(G) R³ and R⁴ are the same or different and each represents a methyl or ethyl group;
(H) R⁵ represents a hydrogen atom, an alkyl group having from 1 to 4 carbon atoms, a benzyl group, an alkanoyloxyalkyl group in which the alkanoyl part has from 1 to 5 carbon atoms and the alkyl part has 1 or 2 carbon atoms, a cycloalkanecarbonyloxyalkyl group in which the cycloalkane part has 5 or 6 carbon atoms and the alkyl part has 1 or 2 carbon atoms, an alkoxycarbonyloxyalkyl group in which the alkoxy part has from 1 to 4 carbon atoms and the alkyl part has 1 or 2 carbon atoms, a cycloalkyloxycarbonyloxyalkyl group in which the cycloalkyl part has 5 or 6 carbon atoms and the alkyl part has 1 or 2 carbon atoms, a (5-phenyl-, 5-methyl- or 5-ethyl- 2-oxo-1,3-dioxolen-4-yl)methyl group, or a phthalidyl group.

We particularly prefer those compounds of formula (Ia) and salts and esters thereof in which R¹-X-R² is as defined in (F) above, R³ and R⁴ are as defined in (G) above, R⁵ is as defined in (H) above and R^{6'} is as defined in (E) above.

The most preferred compounds of the present invention are those compounds of formula (I) or (Ia) and salts and (where appropriate) esters thereof, in which:
(I) the group of formula R¹-X-R²- represents a methoxymethyl group, an ethoxymethyl group, a methylthiomethyl group, a methylthio group or an ethylthio group;
(J) R³ and R⁴ both represent methyl groups; and
(K) R⁵ represents a hydrogen atom, a pivaloyloxymethyl group, an ethoxycarbonyloxymethyl group, a 1-(ethoxycarbonyloxy)ethyl group, an isopropoxycarbonyloxymethyl group, a 1-(isopropoxycarbonyloxy)ethyl group, a (5-methyl-2-oxo-1,3-dioxolen-4-yl)methyl group or a phthalidyl group.

We particularly prefer those compounds of formula (Ia) and salts and esters thereof in which R¹-X-R² is as defined in (I) above, R³ and R⁴ are as defined in (J) above, R⁵ is as defined in (K) above and R^{6'} is as defined in (E) above.

Specific examples of compounds of the present invention are those compounds of formula (Ia), shown above, in which R¹-X-R²-, R³, R⁴, R⁵ and R^{6'} are as defined in the following Table 1. In the Table, the following abbreviations are employed:

| | |
|---|---|
| Bu | butyl |
| Et | ethyl |
| Etc | ethoxycarbonyl |
| Me | methyl |
| Mod | (5-methyl-2-oxo-1,3-dioxolen-4-yl)methyl |
| Phth | 3-phthalidyl |
| Pom | pivaloyloxymethyl |
| Pr | propyl |
| iPr | isopropyl |
| iPrc | isopropoxycarbonyl |
| Tz | tetrazol-5-yl |

**Table 1**

| Compound No. | R¹-X-R²- | R³ | R⁴ | R⁵ | R^{6'} |
|---|---|---|---|---|---|
| 1 | MeOCH₂- | Me | Me | H | COOH |
| 2 | MeOCH₂- | Me | Me | H | Tz |
| 3 | EtOCH₂- | Me | Me | H | COOH |
| 4 | EtOCH₂- | Me | Me | H | Tz |
| 5 | PrOCH₂- | Me | Me | H | COOH |
| 6 | PrOCH₂- | Me | Me | H | Tz |
| 7 | BuOCH₂- | Me | Me | H | COOH |
| 8 | BuOCH₂- | Me | Me | H | Tz |
| 9 | iPrOCH₂ - | Me | Me | H | COOH |
| 10 | iPrOCH₂- | Me | Me | H | Tz |
| 11 | 1-(MeO)Et | Me | Me | H | COOH |
| 12 | 1-(MeO)Et | Me | Me | H | Tz |
| 13 | 2-(MeO)Et | Me | Me | H | COOH |
| 14 | 2-(MeO)Et | Me | Me | H | Tz |
| 15 | 2-(EtO)Et | Me | Me | H | COOH |
| 16 | 2-(EtO)Et | Me | Me | H | Tz |
| 17 | MeSCH₂- | Me | Me | H | COOH |
| 18 | MeSCH₂- | Me | Me | H | Tz |
| 19 | EtSCH₂- | Me | Me | H | COOH |
| 20 | EtSCH₂- | Me | Me | H | Tz |
| 21 | 1-(MeS)Et | Me | Me | H | COOH |
| 22 | 1-(MeS)Et | Me | Me | H | Tz |
| 23 | MeS- | Me | Me | H | COOH |
| 24 | MeS- | Me | Me | H | Tz |
| 25 | EtS- | Me | Me | H | COOH |
| 26 | EtS- | Me | Me | H | Tz |
| 27 | PrS- | Me | Me | H | COOH |
| 28 | PrS- | Me | Me | H | Tz |
| 29 | MeOCH₂- | Me | Et | H | COOH |
| 30 | MeOCH₂- | Me | Et | H | Tz |
| 31 | EtOCH₂- | Me | Et | H | COOH |
| 32 | EtOCH₂- | Me | Et | H | Tz |
| 33 | PrOCH₂- | Me | Et | H | COOH |
| 34 | PrOCH₂- | Me | Et | H | Tz |
| 35 | BuOCH₂- | Me | Et | H | COOH |
| 36 | BuOCH₂- | Me | Et | H | Tz |
| 37 | iPrOCH₂- | Me | Et | H | COOH |
| 38 | iPrOCH₂- | Me | Et | H | Tz |
| 39 | 1-(MeO)Et | Me | Et | H | COOH |
| 40 | 1-(MeO)Et | Me | Et | H | Tz |
| 41 | 2-(MeO)Et | Me | Et | H | COOH |
| 42 | 2-(MeO)Et | Me | Et | H | Tz |
| 43 | 2-(EtO)Et | Me | Et | H | COOH |
| 44 | 2-(EtO)Et | Me | Et | H | Tz |
| 45 | MeSCH₂- | Me | Et | H | COOH |
| 46 | MeSCH₂- | Me | Et | H | Tz |
| 47 | EtSCH₂- | Me | Et | H | COOH |
| 48 | EtSCH₂- | Me | Et | H | Tz |
| 49 | 1-(MeS)Et | Me | Et | H | COOH |
| 50 | 1-(MeS)Et | Me | Et | H | Tz |
| 51 | MeS- | Me | Et | H | COOH |
| 52 | MeS- | Me | Et | H | Tz |
| 53 | EtS- | Me | Et | H | COOH |
| 54 | EtS- | Me | Et | H | Tz |
| 55 | PrS- | Me | Et | H | COOH |
| 56 | PrS- | Me | Et | H | Tz |
| 57 | MeOCH₂- | Et | Et | H | COOH |
| 58 | MeOCH₂- | Et | Et | H | Tz |
| 59 | EtOCH₂- | Et | Et | H | COOH |
| 60 | EtOCH₂- | Et | Et | H | Tz |
| 61 | PrOCH₂- | Et | Et | H | COOH |
| 62 | PrOCH₂- | Et | Et | H | Tz |
| 63 | BuOCH₂- | Et | Et | H | COOH |
| 64 | BuOCH₂- | Et | Et | H | Tz |
| 65 | iPrOCH₂- | Et | Et | H | COOH |
| 66 | iPrOCH₂- | Et | Et | H | Tz |
| 67 | 1-(MeO)Et | Et | Et | H | COOH |
| 68 | 1-(MeO)Et | Et | Et | H | Tz |
| 69 | 2-(MeO)Et | Et | Et | H | COOH |
| 70 | 2-(MeO)Et | Et | Et | H | Tz |
| 71 | 2-(EtO)Et | Et | Et | H | COOH |
| 72 | 2-(EtO)Et | Et | Et | H | Tz |
| 73 | MeSCH₂- | Et | Et | H | COOH |
| 74 | MeSCH₂- | Et | Et | H | Tz |
| 75 | EtSCH₂- | Et | Et | H | COOH |
| 76 | EtSCH₂- | Et | Et | H | Tz |
| 77 | 1-(MeS)Et | Et | Et | H | COOH |
| 78 | 1-(MeS)Et | Et | Et | H | Tz |
| 79 | MeS- | Et | Et | H | COOH |
| 80 | MeS- | Et | Et | H | Tz |
| 81 | EtS- | Et | Et | H | COOH |
| 82 | EtS- | Et | Et | H | Tz |
| 83 | PrS- | Et | Et | H | COOH |
| 84 | PrS- | Et | Et | H | Tz |
| 85 | MeOCH₂- | Me | Me | Pom | COOH |
| 86 | MeOCH₂- | Me | Me | Pom | Tz |
| 87 | EtOCH₂- | Me | Me | Pom | COOH |
| 88 | EtOCH₂- | Me | Me | Pom | Tz |
| 89 | MeSCH₂- | Me | Me | Pom | COOH |
| 90 | MeSCH₂- | Me | Me | Pom | Tz |
| 91 | MeS- | Me | Me | Pom | COOH |
| 92 | MeS- | Me | Me | Pom | Tz |
| 93 | EtS- | Me | Me | Pom | COOH |
| 94 | EtS- | Me | Me | Pom | Tz |
| 95 | MeOCH₂- | Me | Me | Mod | COOH |
| 96 | MeOCH₂- | Me | Me | Mod | Tz |
| 97 | EtOCH₂- | Me | Me | Mod | COOH |
| 98 | EtOCH₂- | Me | Me | Mod | Tz |
| 99 | MeSCH₂- | Me | Me | Mod | COOH |
| 100 | MeSCH₂- | Me | Me | Mod | Tz |
| 101 | MeS- | Me | Me | Mod | COOH |
| 102 | MeS- | Me | Me | Mod | Tz |
| 103 | EtS- | Me | Me | Mod | COOH |
| 104 | EtS- | Me | Me | Mod | Tz |
| 105 | MeOCH₂- | Me | Me | EtcOCH₂- | COOH |
| 106 | MeOCH₂- | Me | Me | EtcOCH₂- | Tz |
| 107 | EtOCH₂- | Me | Me | EtcOCH₂- | COOH |
| 108 | EtOCH₂- | Me | Me | EtcOCH₂- | Tz |
| 109 | MeSCH₂- | Me | Me | EtcOCH₂- | COOH |
| 110 | MeSCH₂- | Me | Me | EtcOCH₂- | Tz |
| 111 | MeS- | Me | Me | EtcOCH₂- | COOH |
| 112 | MeS- | Me | Me | EtcOCH₂- | Tz |
| 113 | EtS- | Me | Me | EtcOCH₂- | COOH |
| 114 | EtS- | Me | Me | EtcOCH₂- | Tz |
| 115 | MeOCH₂- | Me | Me | iPrcOCH₂- | COOH |
| 116 | MeOCH₂- | Me | Me | iPrcOCH₂- | Tz |
| 117 | EtOCH₂- | Me | Me | iPrcOCH₂- | COOH |
| 118 | EtOCH₂- | Me | Me | iPrcOCH₂- | Tz |
| 119 | MeSCH₂- | Me | Me | iPrcOCH₂- | COOH |
| 120 | MeSCH₂- | Me | Me | iPrcOCH₂- | Tz |
| 121 | MeS- | Me | Me | iPrcOCH₂- | COOH |
| 122 | MeS- | Me | Me | iPrcOCH₂- | Tz |
| 123 | EtS- | Me | Me | iPrcOCH₂- | COOH |
| 124 | EtS- | Me | Me | iPrcOCH₂- | Tz |
| 125 | MeOCH₂- | Me | Me | 1-(EtcO)Et | COOH |
| 126 | MeOCH₂- | Me | Me | 1-(EtcO)Et | Tz |
| 127 | EtOCH₂- | Me | Me | 1-(EtcO)Et | COOH |
| 128 | EtOCH₂- | Me | Me | 1-(EtcO)Et | Tz |
| 129 | MeSCH₂- | Me | Me | 1-(EtcO)Et | COOH |
| 130 | MeSCH₂- | Me | Me | 1-(EtcO)Et | Tz |
| 131 | MeS- | Me | Me | 1-(EtcO)Et | COOH |
| 132 | MeS- | Me | Me | 1-(EtcO)Et | Tz |
| 133 | EtS- | Me | Me | 1-(EtcO)Et | COOH |
| 134 | EtS- | Me | Me | 1-(EtcO)Et | Tz |
| 135 | MeOCH₂- | Me | Me | 1-(iPrcO)Et | COOH |
| 136 | MeOCH₂- | Me | Me | 1-(iPrcO)Et | Tz |
| 137 | EtOCH₂- | Me | Me | 1-(iPrcO)Et | COOH |
| 138 | EtOCH₂- | Me | Me | 1-(iPrcO)Et | Tz |
| 139 | MeSCH₂- | Me | Me | 1-(iPrcO)Et | COOH |
| 140 | MeSCH₂- | Me | Me | 1-(iPrcO)Et | Tz |
| 141 | MeS- | Me | Me | 1-(iPrcO)Et | COOH |
| 142 | MeS- | Me | Me | 1-(iPrcO)Et | Tz |
| 143 | EtS- | Me | Me | 1-(iPrcO)Et | COOH |
| 144 | EtS- | Me | Me | 1-(iPrcO)Et | Tz |
| 145 | MeOCH₂- | Me | Me | Phth | COOH |
| 146 | MeOCH₂- | Me | Me | Phth | Tz |
| 147 | EtOCH₂- | Me | Me | Phth | COOH |
| 148 | EtOCH₂- | Me | Me | Phth | Tz |
| 149 | MeSCH₂- | Me | Me | Phth | COOH |
| 150 | MeSCH₂- | Me | Me | Phth | Tz |
| 151 | MeS- | Me | Me | Phth | COOH |
| 152 | MeS- | Me | Me | Phth | Tz |
| 153 | EtS- | Me | Me | Phth | COOH |
| 154 | EtS- | Me | Me | Phth | Tz |
| 155 | MeOCH₂- | Me | Me | Me | COOH |
| 156 | MeOCH₂- | Me | Me | Me | Tz |
| 157 | EtOCH₂- | Me | Me | Et | COOH |
| 158 | EtOCH₂- | Me | Me | Et | Tz |
| 159 | PrOCH₂- | Me | Me | Pr | COOH |
| 160 | PrOCH₂- | Me | Me | Pr | Tz |
| 161 | iPrOCH₂- | Me | Me | iPr | COOH |
| 162 | iPrOCH₂- | Me | Me | iPr | Tz |
| 163 | 1-(MeO)Et | Me | Me | Me | COOH |
| 164 | 1-(MeO)Et | Me | Me | Me | Tz |
| 165 | MeSCH₂- | Me | Me | Et | COOH |
| 166 | MeSCH₂- | Me | Me | Et | Tz |
| 167 | MeS- | Me | Me | Et | COOH |
| 168 | MeS- | Me | Me | Et | Tz |
| 169 | EtS- | Me | Me | Et | COOH |
| 170 | EtS- | Me | Me | Et | Tz |
| 171 | PrS- | Me | Me | Et | COOH |
| 172 | PrS- | Me | Me | Et | Tz |
| 173 | 1-(EtO)Et | Me | Me | H | COOH |
| 174 | 1-(EtO)Et | Me | Me | H | Tz |
| 175 | 1-(EtO)Et | Me | Me | Pom | COOH |
| 176 | 1-(EtO)Et | Me | Me | Pom | Tz |
| 177 | 1-(EtO)Et | Me | Me | Mod | COOH |
| 178 | 1-(EtO)Et | Me | Me | Mod | Tz |
| 179 | 1-(EtO)Et | Me | Me | Et | COOH |
| 180 | 1-(EtO)Et | Me | Me | Et | Tz |
| 181 | HOCH₂- | Me | Me | H | COOH |
| 182 | HOCH₂- | Me | Me | H | Tz |
| 183 | HOCH₂- | Me | Me | Et | COOH |
| 184 | HOCH₂- | Me | Me | Et | Tz |
| 185 | MeOCH₂- | Me | Et | Pom | COOH |
| 186 | MeOCH₂- | Me | Et | Pom | Tz |
| 187 | MeSCH₂- | Me | Et | Pom | COOH |
| 188 | MeSCH₂- | Me | Et | Pom | Tz |
| 189 | MeS- | Me | Et | Pom | COOH |
| 190 | MeS- | Me | Et | Pom | Tz |
| 191 | MeOCH₂- | Me | Et | Mod | COOH |
| 192 | MeOCH₂- | Me | Et | Mod | Tz |
| 193 | MeSCH₂- | Me | Et | Mod | COOH |
| 194 | MeSCH₂- | Me | Et | Mod | Tz |
| 195 | MeS- | Me | Et | Mod | COOH |
| 196 | MeS- | Me | Et | Mod | Tz |
| 197 | EtS- | Me | Et | Mod | COOH |
| 198 | EtS- | Me | Et | Mod | Tz |
| 199 | MeOCH₂- | Me | Et | EtcOCH₂- | COOH |
| 200 | MeOCH₂- | Me | Et | EtcOCH₂- | Tz |
| 201 | MeSCH₂- | Me | Et | EtcOCH₂- | COOH |
| 202 | MeSCH₂- | Me | Et | EtcOCH₂- | Tz |
| 203 | MeS- | Me | Et | EtcOCH₂- | COOH |
| 204 | MeS- | Me | Et | EtcOCH₂- | Tz |
| 205 | MeOCH₂- | Me | Et | iPrcOCH₂- | COOH |
| 206 | MeOCH₂- | Me | Et | iPrcOCH₂- | Tz |
| 207 | MeSCH₂- | Me | Et | iPrcOCH₂- | COOH |
| 208 | MeSCH₂- | Me | Et | iPrcOCH₂- | Tz |
| 209 | MeS- | Me | Et | iPrcOCH₂- | COOH |
| 210 | MeS- | Me | Et | iPrcOCH₂- | Tz |
| 211 | EtS- | Me | Et | iPrcOCH₂- | Tz |
| 212 | MeOCH₂- | Me | Et | 1-(EtcO)Et | COOH |
| 213 | MeOCH₂- | Me | Et | 1-(EtcO)Et | Tz |
| 214 | MeSCH₂- | Me | Et | 1-(EtcO)Et | COOH |
| 215 | MeSCH₂- | Me | Et | 1-(EtcO)Et | Tz |
| 216 | MeS- | Me | Et | 1-(EtcO)Et | COOH |
| 217 | MeS- | Me | Et | 1-(EtcO)Et | Tz |
| 218 | MeOCH₂- | Me | Et | 1-(iPrcO)Et | COOH |
| 219 | MeOCH₂- | Me | Et | 1-(iPrcO)Et | Tz |
| 220 | MeSCH₂- | Me | Et | 1-(iPrcO)Et | COOH |
| 221 | MeSCH₂- | Me | Et | 1-(iPrcO)Et | Tz |
| 222 | MeS- | Me | Et | 1-(iPrcO)Et | COOH |
| 223 | MeS- | Me | Et | 1-(iPrcO)Et | Tz |
| 224 | MeOCH₂- | Me | Et | Phth | COOH |
| 225 | MeOCH₂- | Me | Et | Phth | Tz |
| 226 | MeSCH₂- | Me | Et | Phth | COOH |
| 227 | MeSCH₂- | Me | Et | Phth | Tz |
| 228 | MeS- | Me | Et | Phth | COOH |
| 229 | MeS- | Me | Et | Phth | Tz |

Of the compounds illustrated above, preferred compounds are Compounds No. 1, 2, 3, 4, 5, 6, 9, 10, 11, 12, 17, 18, 19, 20, 23, 24, 25, 26, 27, 28, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152 and 153; and more preferred compounds are Compounds No. 1, 2, 3, 4, 17, 18, 19, 20, 23, 24, 25, 26, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123 and 124.

The most preferred specific compounds are Compounds No.:
2. 4-(1-hydroxy-1-methylethyl)-2-methoxymethyl-1-{4-[2-(tetrazol-5-yl)phenyl]phenyl}methylimidazole-5-carboxylic acid;
4. 2-ethoxymethyl-4-(1-hydroxy-1-methylethyl)-1-{4-[2-(tetrazol-5-yl)phenyl]phenyl}methylimidazole-5-carboxylic acid;
26. 2-ethylthio-4-(1-hydroxy-1-methylethyl)-1-{4-[2-(tetrazol-5-yl)phenyl]phenyl)methylimidazole-5-carboxylic acid;
86. pivaloyloxymethyl 4-(1-hydroxy-1-methylethyl)-2-methoxymethyl-1-{4-[2-(tetrazol-5-yl)phenyl]phenyl}-methylimidazole-5-carboxylate;
88. pivaloyloxymethyl 2-ethoxymethyl-4-(1-hydroxy-1-methylethyl)-1-{4-[2-(tetrazol-5-yl)phenyl]phenyl}-methylimidazole-5-carboxylate;
94. pivaloyloxymethyl 2-ethylthio-4-(1-hydroxy-1-methylethyl)-1-{4-[2-(tetrazol-5-yl)phenyl]phenyl}-methylimidazole-5-carboxylate;
96. (5-methyl-2-oxo-1,3-dioxolen-4-yl)methyl 4-(1-hydroxy-1-methylethyl)-2-methoxymethyl-1-{4-[2-(tetrazol-5-yl)phenyl]phenyl}methylimidazole-5-carboxylate;
98. (5-methyl-2-oxo-1,3-dioxolen-4-yl)methyl 2-ethoxymethyl-4-(1-hydroxy-1-methylethyl)-1-{4-[2-(tetrazol-5-yl)phenyl]phenyl}methylimidazole-5-carboxylate;
104. (5-methyl-2-oxo-1,3-dioxolen-4-yl)methyl 2-ethyl-thio-4- (1-hydroxy-1-methylethyl)-1-{4-[2-(tetrazol-5 -yl)phenyl]phenyl)methylimidazole-5-carboxylate;
106. ethoxycarbonyloxymethyl 4-(1-hydroxy-1-methyl-ethyl)-2-methoxymethyl-1-{4-[2-(tetrazol-5-yl)phenyl]-phenyl}methylimidazole-5-carboxylate;
108. ethoxycarbonyloxymethyl 2-ethoxymethyl-4-(1-hydroxy-1-methylethyl)-1-{4-[2-(tetrazol-5-yl)phenyl]-phenyl}methylimidazole-5-carboxylate;
114. ethoxycarbonyloxymethyl 2-ethylthio-4-(1-hydroxy-1-methylethyl)-1-{4-[2-(tetrazol-5-yl)phenyl]phenyl}-methylimidazole-5-carboxylate;
116. isopropoxycarbonyloxymethyl 4-(1-hydroxy-1-methylethyl)-2-methoxymethyl-1-{4-[2-(tetrazol-5-yl)phenyl]-phenyl}methylimidazole-5-carboxylate;
118. isopropoxycarbonyloxymethyl 2-ethoxymethyl-4-(1-hydroxy-1-methylethyl)-1-(4- [2- (tetrazol-5-yl)phenyl]-phenyl}methylimidazole-5-carboxylate and;
124. isopropoxycarbonyloxymethyl 2-ethylthio-4-(1-hydroxy-1-methylethyl)-1-(4- [2- (tetrazol-5-yl)phenyl]-phenyl}methylimidazole-5-carboxylate;
and pharmaceutically acceptable salts and esters thereof.

The compounds of the present invention can be prepared by a variety of processes well known in the art for the preparation of compounds of this type. For example, they may be prepared by reacting a compound of formula (II): 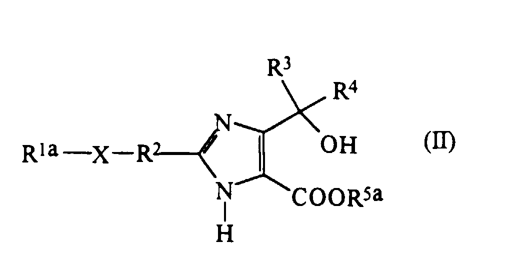 (in which:
R², R³, R⁴ and X are as defined above;
R^{1a} represents
   when X represents an oxygen atom: a hydrogen atom, an alkyl group having from 1 to 6 carbon atoms, a cycloalkyl group having from 3 to 6 carbon atoms or a group of formula R⁷CO-, where R⁷ represents a hydrogen atom, an alkyl group having from 1 to 6 carbon atoms or an aryl group having from 6 to 10 ring carbon atoms; or
   when X represents a sulphur atom: an alkyl group having from 1 to 6 carbon atoms, a cycloalkyl group having from 3 to 6 carbon atoms, a mercapto-protecting group or said group of formula R⁷CO-; and
R^{5a} represents a carboxy-protecting group) with a compound of formula (III): 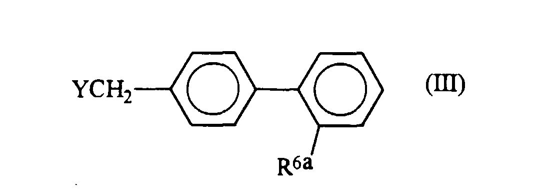 (in which Y represents a halogen atom; and R^{6a} represents a protected carboxy group, a protected tetrazol-5-yl group, a cyano group, a carbamoyl group or an alkylcarbamoyl group in which the alkyl part has from 1 to 6 carbon atoms)
to give a compound of formula (Ib): 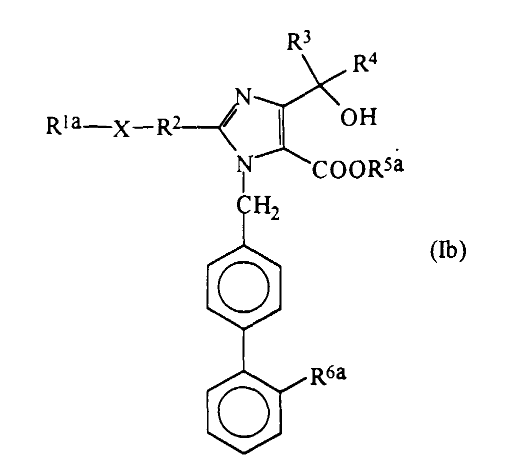
(in which R^{1a}, R², R³, R⁴, R^{5a}, R^{6a} and X are as defined above), and, if necessary, converting any group represented by R^{1a} or R^{6a} to a group represented by R¹ or R⁶, respectively, and, optionally, removing any carboxy-protecting group, salifying and/or esterifying the resulting compound.

In more detail, the compounds of the present invention may be prepared as shown in the following Reaction Schemes A, B and C:

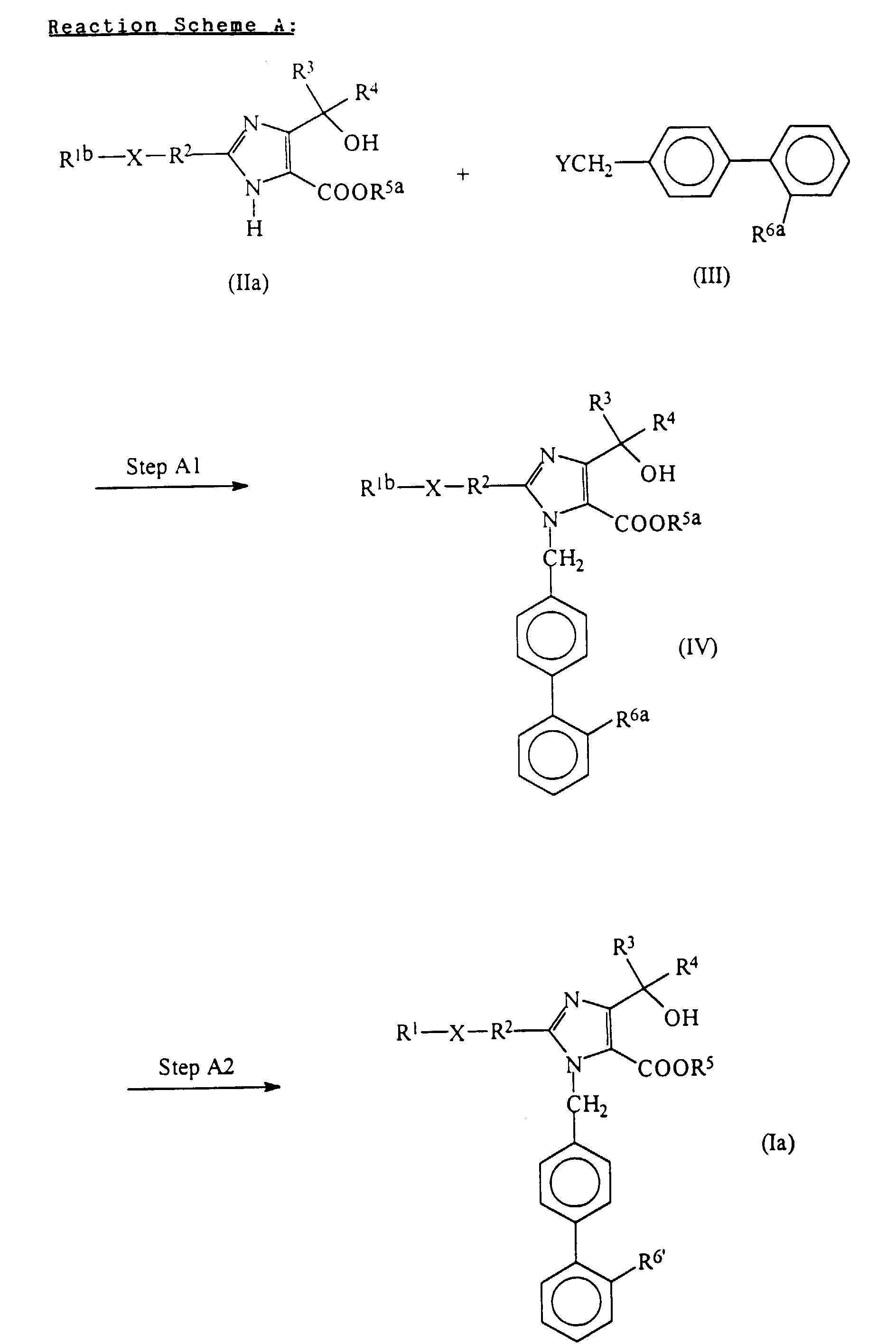

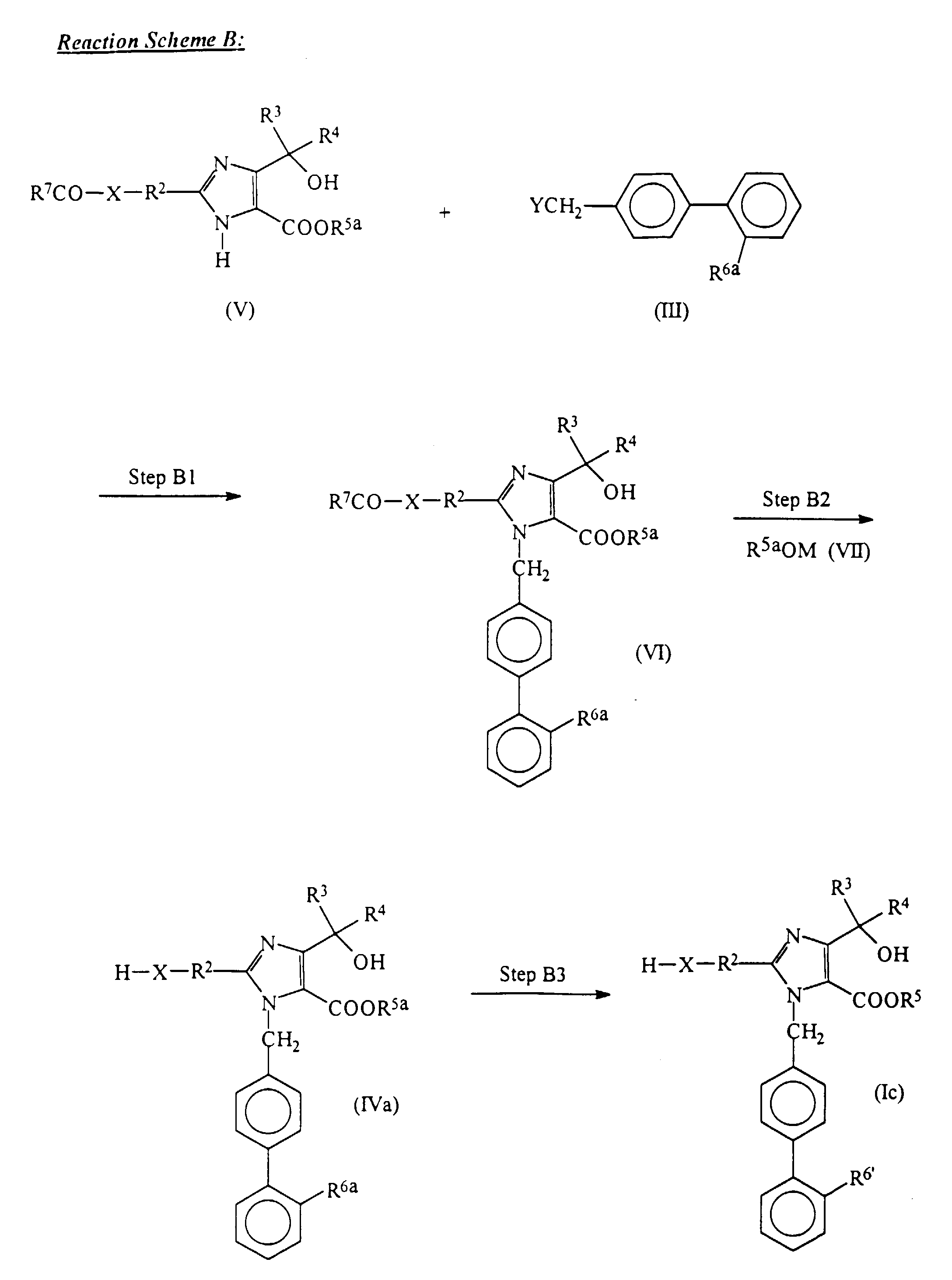

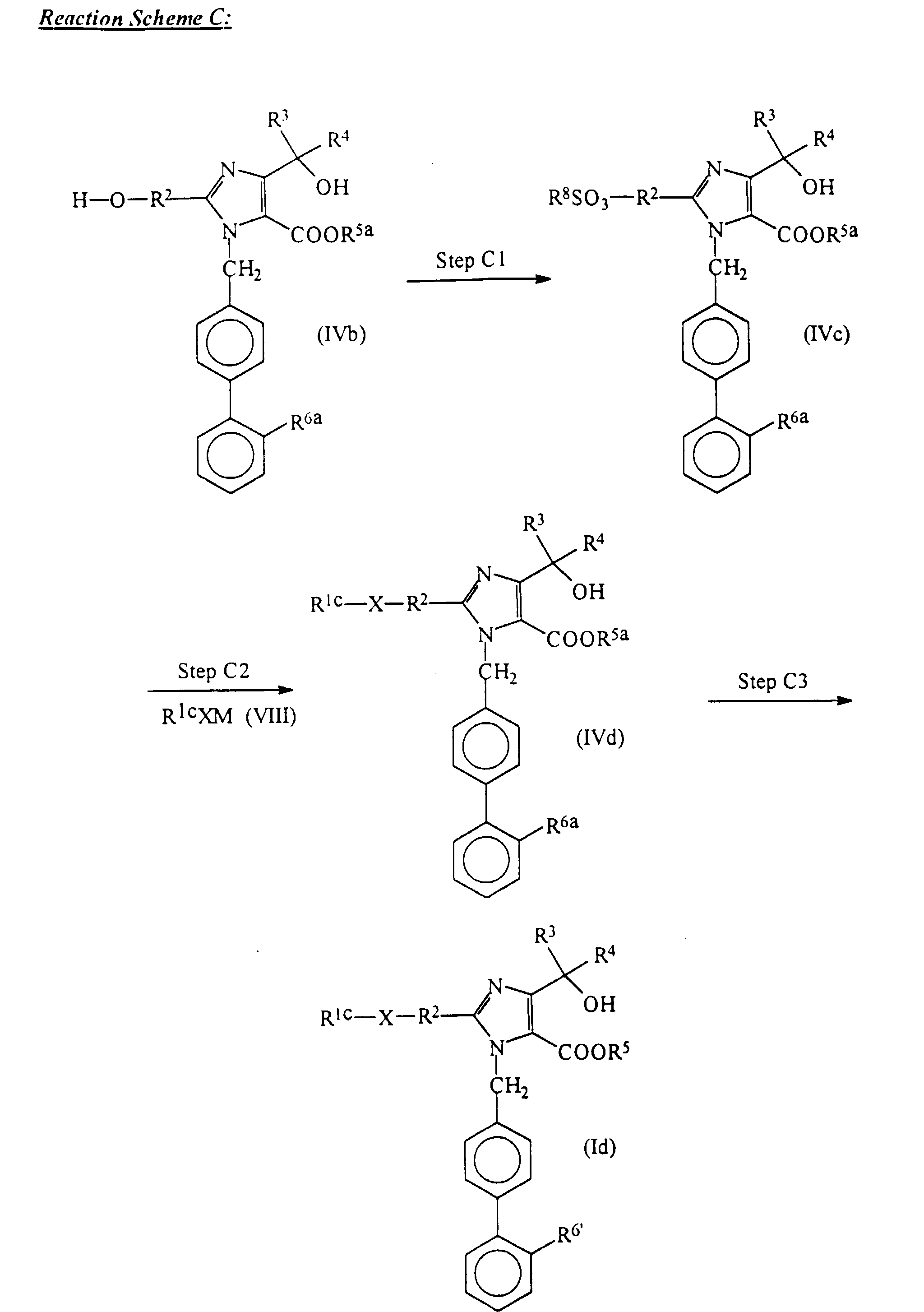

In the above formulae:
R¹, R², R³, R⁴, R⁵, R^{5a}, R⁶', R^{6a}, R⁷, X and Y are as defined above;
R^{1b} represents
   when X represents an oxygen atom, a hydrogen atom, an alkyl group having from 1 to 6 carbon atoms or a cycloalkyl group having from 3 to 6 carbon atoms, or
   when X represents a sulphur atom, an alkyl group having from 1 to 6 carbon atoms group, a cycloalkyl group having from 3 to 6 carbon atoms or a mercapto-protecting group;
R^{1c} represents an alkyl group having from 1 to 6 carbon atoms or a cycloalkyl group having from 3 to 6 carbon atoms;
R⁸ represents an alkyl group having from 1 to 6 carbon atoms or a haloalkyl group having from 1 to 6 carbon atoms or a phenyl group which is unsubstituted or is substituted by at least one substituent selected from halogen atoms, alkyl groups having from 1 to 6 carbon atoms group and nitro groups;
M represents an alkali metal.

Examples of the mercapto-protecting groups which may be represented by R^{1b} include: aralkyl groups in which an alkyl group having from 1 to 4 carbon atoms (such as a methyl, ethyl, propyl, isopropyl, butyl, isobutyl or t-butyl group) is substituted by at least one (and preferably from 1 to 3) aryl groups. The aryl groups are aromatic carbocyclic groups having from 6 to 10, preferably 6 or 10, ring carbon atoms, and are unsubstituted or are substituted by at least one substituent selected from halogen atoms, alkyl groups having from 1 to 4 carbon atoms and alkoxy groups having from 1 to 4 carbon atoms. Examples of such aralkyl groups include the diphenylmethyl, bis(4-methylphenyl)-methyl, bis(4- methoxyphenyl)methyl and trityl (i.e. triphenylmethyl) groups, preferably the trityl group.

Examples of carboxy-protecting groups which are represented by R^{5a} or included in the protected group represented by R^{6a} include the ester groups exemplified above in relation to the groups which may be represented by R⁵.

Examples of the tetrazolyl-protecting groups which may be included in the protected group represented by R^{6a} include aralkyl groups as defined above in relation to the mercapto-protecting groups which may be included in R^{1b}, such as the benzyl, diphenylmethyl and trityl groups, and preferably the trityl group.

The alkyl and cycloalkyl groups which may be represented by R^{1b} and R^{1c} are as defined and exemplified above in relation to the corresponding groups which may be represented by R¹.

Examples of alkylcarbamoyl groups which may be represented by R^{6a} include methylcarbamoyl, ethylcarbamoyl, propylcarbamoyl, butylcarbamoyl, t-butylcarbamoyl, pentylcarbamoyl, t-pentylcarbamoyl and hexylcarbamoyl groups, preferably the t-butylcarbamoyl and t-pentylcarbamoyl groups.

Where R⁷ represents an alkyl group, this may be, for example, a methyl, ethyl, propyl, butyl, t-butyl, pentyl, t-pentyl or hexyl group, preferably a methyl or ethyl group. Where R⁷ represents an aryl group, this is a carbocyclic aromatic group having from 6 to 10, preferably 6 or 10, ring carbon atoms, which may be unsubstituted or substituted as defined generally above, for example a substituted or unsubstituted phenyl or naphthyl group, preferably a phenyl group.

Where R⁸ represents an alkyl group, this has from 1 to 6 carbon atoms and may be any of those alkyl groups exemplified above in relation to R¹, and is most preferably a methyl group. Where R⁸ represents a haloalkyl group, this has from 1 to 6 carbon atoms and may be any of those haloalkyl groups exemplified above in relation to R⁵, for example a trifluoromethyl, trichloromethyl or 2,2,2-trichloroethyl group, preferably a trifluoromethyl group. Where R⁸ represents a phenyl group, this may be unsubstituted or it may be substituted by a halogen atom, an alkyl group having from 1 to 6 carbon atoms or a nitro group, and preferred examples of such groups include the phenyl, p-tolyl, p-chlorophenyl, p-bromophenyl and p-nitrophenyl groups.

Examples of the halogen atoms which may be represented by Y include the chlorine, bromine and iodine atoms.

Examples of the alkali metals which may be represented by M include the lithium, sodium and potassium atoms, of which we prefer the lithium and sodium atoms.

### Reaction Scheme A

This Reaction Scheme illustrates the preparation of compounds of formula (Ia).

### Step A1:

In Step A1, a compound of formula (IV) may be prepared by reacting a compound of formula (IIa) with a compound of formula (III), normally and preferably in an inert solvent and in the presence of a base.

There is no particular restriction on the nature of the solvent to be employed, provided that it has no adverse effect on the reaction or on the reagents involved and that it can dissolve the reagents, at least to some extent. Examples of suitable solvents include: hydrocarbons, especially aromatic hydrocarbons, such as benzene or toluene; ethers, such as tetrahydrofuran or dioxane; alcohols, such as methanol, ethanol or t-butanol; amides, such as N,N-dimethylacetamide, N,N-dimethylformamide or N-methyl-2-pyrrolidinone; ketones, such as acetone or methyl ethyl ketone; nitriles, such as acetonitrile; and sulphoxides, such as dimethyl sulphoxide. Of these, we prefer the amides, ketones, nitriles or sulphoxides.

There is likewise no particular restriction on the nature of the base employed in this reaction, provided that it has no adverse effect on any of the reagents. Preferred examples of bases which may be used include: alkali metal carbonates, such as sodium carbonate or potassium carbonate; alkali metal hydrides, such as sodium hydride, potassium hydride or lithium hydride; alkali metal alkoxides, such as sodium methoxide, sodium ethoxide, potassium t-butoxide or lithium methoxide; and alkali metal hydrogencarbonates, such as sodium hydrogencarbonate or potassium hydrogencarbonate. Of these, we prefer the alkali metal carbonates, alkali metal hydrides or alkali metal alkoxides.

The reaction can take place over a wide range of temperatures, and the precise reaction temperature is not critical to the invention. In general, we find it convenient to carry out the reaction at a temperature of from -10°C to 100°C, more preferably from 0°C to 80°C. The time required for the reaction may also vary widely, depending on many factors, notably the reaction temperature and the nature of the reagents, solvent and base employed. However, provided that the reaction is effected under the preferred conditions outlined above, a period of from 30 minutes to 24 hours, more preferably from 1 to 16 hours, will usually suffice.

After completion of the reaction, the desired compound of formula (IV) can be recovered from the reaction mixture by conventional means. A suitable recovery procedure comprises: distilling off the solvent under reduced pressure; adding water to the residue; extracting the mixture with a water-immiscible organic solvent, such as ethyl acetate; drying the extract, for example over anhydrous magnesium sulphate; and then distilling off the solvent. If necessary, the product can be further purified by conventional means, for example, by recrystallisation, or by the various chromatography techniques, notably by column chromatography.

### Step A2:

Step A2 is optional, and comprises a series of reactions any one or more of which may be carried out, if desired:
Reaction A2(a): in which the carboxy-protecting groups represented by R^{5a} and included in R^{6a} may be deprotected selectively or nonselectively;
Reaction A2(b): in which the tetrazolyl-protecting groups included in R^{6a} may be deprotected;
Reaction A2(c): in which a cyano group, a carbamoyl group or an alkylcarbamoyl group having from 1 to 6 carbon atoms in the alkyl part may be converted to a tetrazolyl group;
Reaction A2(d): in which the carboxy groups in the compound in which R⁵ represents a hydrogen atom or R⁶ represents a carboxy group may be protected;
Reaction A2(e): in which the mercapto-protecting group represented by R^{1b} may be deprotected; and
Reaction A2(f): in those cases where R^{1b} represents a hydrogen atom, the resulting hydroxy or mercapto group may be acylated.

These reactions may be carried out in any appropriate order, and are described in more detail below.

### Reaction A2(a):

The nature of the reaction employed for the deprotection of the carboxy-protecting group in reaction A2(a) will, of course, vary depending upon the nature of the protecting group to be removed, as is well known in the art. The reaction can be carried out using procedures well known in the field of organic synthetic chemistry.

For example, where the carboxy-protecting group is an aralkyl group, such as a benzyl group, it can be removed by catalytic reduction in an atmosphere of hydrogen. The pressure of hydrogen is preferably from 1 to 5 atmospheres. The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: alcohols, such as methanol or ethanol; and carboxylic acids, such as acetic acid. Any catalyst commonly used for catalytic reduction may equally be used in this reaction. Examples include palladium-on-charcoal and platinum oxide.

Where the carboxy-protecting group is a t-butyl or diphenylmethyl group, it can be removed by reaction with an acid (preferably a mineral acid, such as hydrogen chloride or sulphuric acid, or an organic acid, such as trifluoroacetic acid, methanesulphonic acid or p-toluene- sulphonic acid). The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: alcohols, such as methanol and ethanol; ethers, such as tetrahydrofuran and dioxane; water; or a mixture of water and any one or more of the above organic solvents.

The ester residue can be removed by a conventional hydrolysis reaction, using a base, preferably an alkali metal hydroxide, such as lithium hydroxide, sodium hydroxide or potassium hydroxide; or an alkali metal carbonate, such as sodium carbonate or potassium carbonate. The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: alcohols, such as methanol and ethanol; ethers, such as tetrahydrofuran and dioxane; water; or a mixture of water with any one or more of the above organic solvents.

These reactions can take place over a wide range of temperatures, and the precise reaction temperature is not critical to the invention, although the preferred reaction temperature will vary depending upon the deprotecting method and the nature of the solvent. However, in general, we find it convenient to carry out the reaction at a temperature of from 0°C to 100°C, more preferably from about room temperature to 60°C. The time required for the reaction may also vary widely, depending on many factors, notably the reaction temperature and the nature of the reagents and solvent employed. However, provided that the reaction is effected under the preferred conditions outlined above, a period of from 30 minutes to 24 hours, more preferably from 1 to 16 hours, will usually suffice.

After completion of the reaction, the desired product can be recovered from the reaction mixture by conventional means, which will depend on the nature of the deprotection reaction. For example, where deprotection is carried out by catalytic reduction, the product can be recovered by filtering off the catalyst and then distilling off the solvent. Where deprotection is carried out using an acid, the product can be recovered by collecting the crystals which appear in the reaction system by filtration or other suitable means, or by distilling off the solvent. Where deprotection is carried out by alkaline hydrolysis, the product can be recovered by distilling off the solvent, neutralising the residue with an acid, and collecting the crystals which appear in the aqueous solvent; or by neutralising the mixture with an acid, extracting the product with a water-immiscible organic solvent, such as ethyl acetate, and distilling off the solvent. If necessary, the product can be further purified by conventional means, for example, by recrystallisation, or by the various chromatography techniques, notably by column chromatography.

The protecting groups included in R^{5a} and R^{6a} can be selectively removed by appropriate selection of the reaction conditions.

### Reaction A2(b):

The nature of the reaction employed for the deprotection of the tetrazolyl-protecting group included in the protected group represented by R^{6a} in reaction A2(b) will, of course, vary depending upon the nature of the protecting group to be removed, as is well known in the art. The reaction can be carried out using procedures well known in the field of organic synthetic chemistry.

For example, where the protecting group is a trityl group, it may be removed by treating the protected compound with an acid. The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include water and organic solvents, for example: carboxylic acids, such as formic acid and acetic acid; ethers, such as tetrahydrofuran and dioxane; alcohols, such as methanol and ethanol; and mixtures of any two or more of the above solvents.

Examples of acids which may be used in this reaction include: organic carboxylic and sulphonic acids, such as formic acid, acetic acid, oxalic acid, methanesulphonic acid, p-toluenesulphonic acid or trifluoroacetic acid, and inorganic acids, such as hydrochloric acid, hydrobromic acid, sulphuric acid or phosphoric acid. Of these, we prefer acetic acid, trifluoroacetic acid or hydrochloric acid.

The reaction can take place over a wide range of temperatures, and the precise reaction temperature is not critical to the invention. In general, we find it convenient to carry out the reaction at a temperature of from 0°C to 120°C, more preferably from 10°C to 100°C. The time required for the reaction may also vary widely, depending on many factors, notably the reaction temperature and the nature of the reagents and solvent employed. However, provided that the reaction is effected under the preferred conditions outlined above, a period of from 30 minutes to 24 hours, more preferably from 1 to 16 hours, will usually suffice.

Where the tetrazolyl-protecting group is a benzyl or diphenylmethyl group, it can be removed by the catalytic reduction method described above in reaction A2(a) in relation to the removal of aralkyl groups used as carboxy-protecting groups, using a catalyst such as palladium or platinum oxide.

After completion of the reaction, the desired product of the reaction can be recovered from the reaction mixture by conventional means, for example, in a similar manner to that described in reaction A2(a) in Reaction Scheme A.

### Reaction A2(c):

The conversion of a cyano group which may be represented by R^{6a} to a tetrazolyl group in reaction A2(c) may be effected by any of the following three methods.

### A2(c-1-1) Reaction with an alkali metal azide

The reaction may be carried out by reacting the cyano compound with an alkali metal azide (such as lithium azide, sodium azide or potassium azide, preferably sodium azide). The amount of azide is not critical, although we prefer to use the azide in an amount at least equimolar with respect to the cyano compound. A suitable amount is from 1 to 5 moles (more preferably from 1 to 3 moles) of the azide per mole of the cyano compound. The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: ethers, such as dioxane and 1,2-dimethoxyethane; alcohols, such as methanol and ethanol; amides, such as N,N-dimethylformamide and N,N-dimethylacetamide; and sulphoxides, such as dimethyl sulphoxide. The reaction is preferably effected in the presence of an ammonium halide (such as ammonium fluoride, ammonium chloride or ammonium bromide, preferably ammonium chloride). The amount of ammonium halide used is not critical to the invention, although we generally prefer to use from 0.5 to 2 moles, more preferably from 1 to 1.2 moles of ammonium halide per mole of the cyano compound.

The reaction can take place over a wide range of temperatures, and the precise reaction temperature is not critical to the invention. In general, we find it convenient to carry out the reaction at a temperature of from 70°C to 150°C, more preferably from 90°C to 120°C. The time required for the reaction may also vary widely, depending on many factors, notably the reaction temperature and the nature of the reagents and solvent employed. However, provided that the reaction is effected under the preferred conditions outlined above, a period of from 10 hours to 7 days, more preferably from 1 day to 5 days will usually suffice.

After completion of the reaction, the product can be recovered by adding water and a water-immiscible organic solvent, such as ethyl acetate, to the reaction mixture, separating the resulting organic solvent layer and distilling off the solvent. If necessary, the product can be further purified by conventional means, for example, by recrystallisation, or by the various chromatography techniques, notably by column chromatography.

### A2(c-1-2) Reaction with a trialkyl- or triaryl- stannic azide

This reaction may be carried out by reacting, in a first step, the corresponding cyano compound with a trialkylstannic azide in which each alkyl group has from 1 to 6 carbon atoms (preferably trimethylstannic azide or tributylstannic azide) or a triarylstannic azide (preferably triphenylstannic azide or tritolylstannic azide) to form a stannic adduct, which is then treated, in a second step, with an acid, a base or an alkali metal fluoride. The amount of the trialkyl- or triarylstannic azide employed is not critical, although we generally find it convenient to use at least an equimolar amount of the trialkyl- or triaryl- stannic azide with respect to the cyano compound, preferably from 1 to 3 moles, more preferably from 1 to 2 moles of the trialkyl- or triaryl- stannic azide per mole of the cyano compound. The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: hydrocarbons, such as benzene, toluene, xylene and heptane; halogenated hydrocarbons, especially halogenated aliphatic hydrocarbons, such as methylene chloride and chloroform, ethers; such as dioxane and 1,2-dimethoxyethane; esters, such as ethyl acetate and butyl acetate; amides, such as N,N-dimethylformamide and N,N-dimethylacetamide; and sulphoxides, such as dimethyl sulphoxide).

The resulting stannic adduct is then treated with an acid (preferably hydrochloric acid or sulphuric acid), a base (preferably an alkali metal hydroxide, such as sodium hydroxide or potassium hydroxide, an alkali metal carbonate, such as sodium carbonate or potassium carbonate, or an alkali metal hydrogencarbonate, such as sodium hydrogencarbonate or potassium hydrogencarbonate) or an alkali metal fluoride (preferably sodium fluoride or potassium fluoride). The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: the solvents listed above for use in the first step; alcohols, such as methanol and ethanol; water; and aqueous alcohols.

These reactions can take place over a wide range of temperatures, and the precise reaction temperature is not critical to the invention. In general, we find it convenient to carry out the reaction of the first step at a temperature of from 60°C to 150°C, more preferably from 80°C to 120°C. The time required for each of the reactions may also vary widely, depending on many factors, notably the reaction temperature and the nature of the reagents and solvent employed. However, provided that the reaction is effected under the preferred conditions outlined above, a period of from 8 hours to 7 days, more preferably from 1 day to 5 days, will usually suffice. In the second step of the reaction, a suitable reaction temperature is normally about room temperature, and the reaction will generally be complete within from 30 minutes to 24 hours, more preferably from 1 hour to 6 hours.

After completion of the reaction, the product can be recovered by adding water and a water-immiscible organic solvent (such as ethyl acetate) to the reaction mixture, acidifying the aqueous layer with a mineral acid (such as hydrochloric acid), separating the resulting organic solvent layer and distilling off the solvent. The product may then, if necessary, be further purified by conventional means, for example, by recrystallisation, or by the various chromatography techniques, notably by column chromatography.

### A2(c-1-3) Reaction with a trialkyl- or triaryl- stannic halide and an alkali metal azide

This reaction may be conducted by reacting the corresponding cyano compound with a trialkylstannic halide or a triarylstannic halide (preferably trimethylstannic chloride, triethylstannic chloride, tributylstannic chloride or triphenylstannic chloride) and an alkali metal azide, instead of the trialkylstannic azide or triarylstannic azide of reaction A2(c-1-2). Examples of suitable alkali metal azides include sodium azide and lithium azide. The amounts of the trialkyl- or triarylstannic halide and the alkali metal azide employed are not critical, although we generally find it convenient to use at least an equimolar amount of the trialkyl- or triaryl- stannic halide and of the alkali metal azide with respect to the cyano compound, preferably from 1 to 3 moles, more preferably from 1 to 2 moles, of the trialkyl- or triaryl- stannic halide, and from 1 to 3 moles, more preferably from 1 to 2 moles, of the alkali metal azide per mole of the cyano compound. The reaction is carried out in two steps, each of which may be effected in a similar manner to that described above for reaction A2(c-1-2).

The conversion of an alkylcarbamoyl or carbamoyl group represented by R^{6a} to a tetrazolyl group may be effected by first converting the alkylcarbamoyl or carbamoyl group to a cyano group, and then converting the cyano group to a tetrazolyl group using the above reactions A2(c-1-1), A2(c-1-2) and A2(c-1-2). The conversion of the alkylcarbamoyl or carbamoyl group to a cyano group may be conducted by either of the following two methods.

### A2(c-2-1) Reaction with a halogenating agent, to convert an alkylcarbamoyl group to a cyano group

This reaction may be conducted by reacting the corresponding alkylcarbamoyl compound with a halogenating agent, preferably oxalyl chloride, phosphorus oxychloride or thionyl chloride. The amount of the halogenating agent employed is not critical, although we generally find it convenient to use at least an equimolar amount of the halogenating agent with respect to the alkylcarbamoyl compound, preferably from 1 to 3 moles, more preferably from 1 to 2 moles, of the halogenating agent per mole of the alkylcarbamoyl compound. The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: hydrocarbons, such as benzene, toluene, xylene and heptane; halogenated hydrocarbons, especially halogenated aliphatic hydrocarbons, such as methylene chloride and chloroform; ethers, such as diethyl ether, tetrahydrofuran and dioxane; and esters, such as ethyl acetate and butyl acetate.

The reaction can take place over a wide range of temperatures, and the precise reaction temperature is not critical to the invention. In general, we find it convenient to carry out the reaction at a temperature of from -10°C to 100°C, more preferably from 0°C to 50°C. The time required for the reaction may also vary widely, depending on many factors, notably the reaction temperature and the nature of the reagents and solvent employed. However, provided that the reaction is effected under the preferred conditions outlined above, a period of from 10 minutes to 16 hours, more preferably from 30 minutes to 6 hours, will usually suffice.

After completion of the reaction, the product can be recovered by conventional means. For example, on suitable recovery procedure comprises: adding a weakly basic aqueous solution, for example an aqueous solution of an alkali metal hydrogencarbonate (preferably sodium hydrogencarbonate), and a water-immiscible organic solvent, such as ethyl acetate, to the reaction mixture; separating the resulting organic solvent layer; and distilling off the solvent. The product may then, if necessary, be further purified by conventional means, for example, by recrystallisation, or by the various chromatography techniques, notably by column chromatography.

### A2(c-2-2) Reaction with a dehydrating agent to convert a carbamoyl group to a cyano group

This reaction may be conducted by reacting the corresponding carbamoyl compound with a dehydrating agent, preferably an acid anhydride, such as acetic anhydride, trifluoroacetic anhydride, methanesulphonic anhydride or trifluoromethanesulphonic anhydride, or thionyl chloride. The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include:
hydrocarbons, such as benzene, toluene, xylene and heptane; halogenated hydrocarbons, especially halogenated aliphatic hydrocarbons, such as methylene chloride and chloroform; ethers, such as diethyl ether, tetrahydrofuran and dioxane; and esters, such as ethyl acetate and butyl acetate. The reaction is effected in the presence of an organic amine, preferably triethylamine, pyridine or N-methylmorpholine.

The reaction can take place over a wide range of temperatures, and the precise reaction temperature is not critical to the invention. In general, we find it convenient to carry out the reaction at a temperature of from -10°C to 100°C, more preferably from 0°C to 50°C. The time required for the reaction may also vary widely, depending on many factors, notably the reaction temperature and the nature of the reagents and solvent employed. However, provided that the reaction is effected under the preferred conditions outlined above, a period of from 10 minutes to 16 hours, more preferably from 30 minutes to 6 hours, will usually suffice.

After completion of the reaction, the product can be recovered by adding a weakly basic aqueous solution (such as an aqueous solution of sodium hydrogencarbonate) and a water-immiscible organic solvent, such as ethyl acetate, to the reaction mixture, separating the resulting organic solvent layer and distilling off the solvent. The product may then, if necessary, be further purified by conventional means, for example, by recrystallisation, or by the various chromatography techniques, notably by column chromatography.

### Reaction A2(d):

The carboxy-protecting reaction in reaction A2(d) may be carried out by conventional means well known in the field of organic synthetic chemistry.

For example, the reaction may be conducted by reacting the corresponding carboxylic acid with a compound of formula R^{5a}-Z (IX) (in which: R^{5a} is as defined above; and Z represents a halogen atom, such as a chlorine, bromine or iodine atom, or a group of formula -OSO₃R^{5a}, in which R^{5a} is as defined above), preferably in the presence of a base.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: amides, such as N,N-dimethylformamide or N,N-dimethylacetamide; halogenated hydrocarbons, especially halogenated aliphatic hydrocarbons, such as methylene chloride or 1,2-dichloroethane; ketones, such as acetone or methyl ethyl ketone; and nitriles, such as acetonitrile. Of these, we prefer the amides or the ketones.

Examples of bases which may be used include: alkali metal carbonates, such as sodium carbonate and potassium carbonate; alkali metal hydrogencarbonates, such as sodium hydrogencarbonate and potassium hydrogencarbonate; alkali metal hydrides, such as lithium hydride, sodium hydride and potassium hydride; and tertiary amines, such as triethylamine, N-methyl-morpholine and diisopropylethylamine. Of these, we prefer the alkali metal carbonates or the tertiary amines.

The reaction conditions, including the reaction temperature and time, and the recovery procedure are all similar to those described above in step A1 of Reaction Scheme A.

Where the carboxy-protecting group to be introduced is an alkyl group having from 1 to 6 carbon atoms, the reaction can be conducted by reacting the corresponding carboxylic acid with an alcohol having from 1 to 6 carbon atoms (such as methanol, ethanol, propanol or hexanol) in the presence of an acid catalyst (such as hydrogen chloride or sulphuric acid), using the alcohol as solvent. The reaction can take place over a wide range of temperatures, and the precise reaction temperature is not critical to the invention. In general, we find it convenient to carry out the reaction at a temperature of from 0°C to 100°C. The time required for the reaction may also vary widely, depending on many factors, notably the reaction temperature and the nature of the reagents and solvent employed. However, provided that the reaction is effected under the preferred conditions outlined above, a period of from 1 hour to 24 hours will usually suffice. Alternatively, such protecting groups may be introduced by treating the corresponding carboxylic acid with a halogenating agent (such as phosphorus pentachloride, thionyl chloride or oxalyl chloride) in an inert solvent (preferably a halogenated hydrocarbon, such as methylene chloride or chloroform; an ether, such as tetrahydrofuran or dioxane; or an aromatic hydrocarbon, such as benzene or toluene) to give the corresponding acid halide, and then reacting this acid halide with a corresponding alcohol (when preparing the t-butyl ester, potassium t-butoxide is desirable in place of the alcohol) in the presence of a base (for example an organic amine, such as triethylamine). These reactions, likewise, can take place over a wide range of temperatures, and the precise reaction temperature is not critical to the invention. In general, we find it convenient to carry out both reactions at about room temperature. The time required for the reactions may also vary widely, depending on many factors, notably the reaction temperature and the nature of the reagents and solvent employed. However, provided that the reactions are effected under the preferred conditions outlined above, a period of from 30 minutes to 5 hours will usually suffice for the first reaction, whilst a period of from 30 minutes to 10 hours will usually suffice for the second reaction. The desired compound can then be recovered by conventional means, for example, by similar means to those described above in step A1 of Reaction Scheme A.

### Reaction A2(e):

The removal of the mercapto-protecting group represented by R^{1b} in reaction A2(e) may be effected by treating the protected compound with an acid (such as trifluoroacetic acid or a mixture of hydrobromic acid and acetic acid) and may be conducted in a similar manner to that described above for the deprotection of a carboxy-protecting group with an acid in reaction A2(a) described above.

### Reaction A2(f):

Acylation in reaction A2(f) may be conducted by reacting the compound where R^{1b} represents a hydrogen atom with: an alkanoyl halide having from 2 to 6 carbon atoms, for example acetyl chloride, propionyl chloride, butyryl bromide, valeryl chloride or hexanoyl chloride; a mixed acid anhydride, such as a mixed acid anhydride between formic acid and acetic acid; or an anhydride of a carboxylic acid having from 2 to 6 carbon atoms, such as acetic anhydride, propionic anhydride, valeric anhydride or hexanoic anhydride. The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: halogenated hydrocarbons, especially halogenated aliphatic hydrocarbons, such as methylene chloride and chloroform; esters, such as ethyl acetate; and ethers, such as tetrahydrofuran and dioxane. The reaction is effected in the presence of a base, for example an organic tertiary amine, such as triethylamine, pyridine, picoline, lutidine or N,N-diethyl-N-isopropylamine.

The reaction can take place over a wide range of temperatures, and the precise reaction temperature is not critical to the invention. In general, we find it convenient to carry out the reaction at a temperature of from 0°C to 120°C, more preferably from 0°C to 80°C. The time required for the reaction may also vary widely, depending on many factors, notably the reaction temperature and the nature of the reagents and solvent employed. However, provided that the reaction is effected under the preferred conditions outlined above, a period of from 1 hour to 24 hours, more preferably from 1 to 16 hours, will usually suffice.

After completion of the reaction, the reaction product can be recovered from the reaction mixture by conventional means, for example, in a similar manner to that described for the recovery of the compound in step A1 of Reaction Scheme A, described above.

In this reaction, in order to acylate the group of formula R^{1b}-X-R²-, where R^{1b} represents a hydrogen atom, without affecting the hydroxy group present in the substituent at the 4-position on the imidazole ring [that is in the group of formula -C(OH)R³R⁴], we prefer that the reaction should only be applied to compounds in which the groups represented by R³ and R⁴ are alkyl groups and where the hydroxy or mercapto group represented by R^{1b}-X- is linked to a primary or secondary carbon atom [such as -CH₂- or -CH(CH₃)-] in the group represented by R².

### Reaction Scheme B

In this Reaction Scheme, a compound of formula (Ic), that is a compound of formula (Ia) in which the group represented by R¹ is a hydrogen atom, is prepared.

### Step B1:

In Step B1, a compound of formula (VI) may be prepared by reacting a compound of formula (III) with a compound of formula (V). This reaction is essentially the same as, and may be carried out using the same reagents and reaction conditions as, that of Step A1 in Reaction Scheme A, described above.

### Step B2:

In Step B2, a compound of formula (IVa) can be prepared by reacting the compound of formula (VI) with a compound of formula R^{5a}OM (VII). The amount of the compound of formula (VII) employed in this reaction is not critical, but we generally prefer to employ at least an equimolar amount of the compound of formula (VII) with respect to the compound of formula (VI). More preferably, we employ from 1 to 3 moles, still more preferably from 1 to 2 moles, of the compound of formula (VII) per mole of the compound of formula (VI).

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: the alcohols represented by the formula R^{5a}OH (in which R^{5a} is as defined above); ethers, such as tetrahydrofuran and dioxane; and halogenated hydrocarbons, especially halogenated aliphatic hydrocarbons, such as methylene chloride and chloroform. A single one of these organic solvents may be employed, or a mixture of any two or more of them may be employed. Of these solvents, we prefer the alcohols or the ethers.

The reaction can take place over a wide range of temperatures, and the precise reaction temperature is not critical to the invention, although the preferred reaction temperature will depend on the nature of the compounds used as starting materials. In general, we find it convenient to carry out the reaction at a temperature of from -20°C to 80°C, more preferably from -10°C to 40°C. The time required for the reaction may also vary widely, depending on many factors, notably the reaction temperature and the nature of the reagents and solvent employed. However, provided that the reaction is effected under the preferred conditions outlined above, a period of from 30 minutes to 24 hours, more preferably from 1 hour to 16 hours, will usually suffice.

After completion of the reaction, the reaction product of formula (IVa) can be recovered from the reaction mixture by conventional means. One suitable recovery technique comprises: distilling off the solvent under reduced pressure; adding water and a water-immiscible organic solvent, such as ethyl acetate, to the residue; separating the organic solvent layer containing the desired compound; drying this over a drying agent, such as anhydrous magnesium sulphate; and distilling off the solvent. The product may, if necessary, be further purified by conventional means, for example, by recrystallisation, or by the various chromatography techniques, notably by column chromatography.

### Step B3:

In Step B3, a compound of formula (Ic) can be prepared from the compound of formula (IVa) in a similar manner to that described above in relation to reactions A2(a) to A2(d) in step A2 of Reaction Scheme A.

### Reaction Scheme C

In this Reaction Scheme, a compound of formula (Id), that is a compound of formula (Ia) in which R¹ is an alkyl group having from 1 to 6 carbon atoms or a cycloalkyl group having from 3 to 6 carbon atoms, is prepared.

### Step C1:

In Step C1, a compound of formula (IVc) is prepared by reacting a compound of formula (IVb) with a compound of formula (XI) or (XIa):

(R⁸SO₂)₂O (XI)

or

R⁸SO₂Y (XIa)

(in which R⁸ and Y are as defined above) in the presence of a base.

The amount of the compound of formula (XI) or (XIa) employed in this reaction is not critical, although we prefer to employ at least an equimolar amount of the compound of formula (XI) or (XIa) with respect to the compound of formula (IVb). More preferably, we employ from 1 to 3 moles, still more preferably from 1 to 2 moles, of the compound of formula (XI) or (XIa) per mole of the compound of formula (IVb).

The nature of the base employed in this reaction is also not critical, provided that it has no adverse effect on the reagents, and any base commonly used in a sulphonylation reaction of this type may equally be employed here. Preferred examples of bases which may be used include organic amines, such as triethylamine, N,N-diisopropyl-N-ethylamine, 4-dimethylaminopyridine, 1,5-diazabicyclo[4.3.0]-5-nonene, 1,8-diazabicyclo-[5.4.0]-7-undecene and 1,4-diazabicyclo[2.2.2]octane. Of these, we particularly prefer triethylamine or N,N-diisopropyl-N-ethylamine. The amount of the base employed in this reaction is not critical, although we prefer to employ at least an equimolar amount of the base with respect to the compound of formula (IVb). More preferably, we employ from 1 to 3 moles, still more preferably from 1 to 2 moles, of the base per mole of the compound of formula (IVb).

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: hydrocarbons, such as benzene, toluene or hexane; halogenated hydrocarbons, especially halogenated aliphatic hydrocarbons, such as methylene chloride or chloroform; ethers, such as diethyl ether, tetrahydrofuran or dioxane; and esters, such as ethyl acetate. Of these, we prefer the halogenated hydrocarbons or the ethers.

The reaction can take place over a wide range of temperatures, and the precise reaction temperature is not critical to the invention, although the preferred reaction temperature will depend on the nature of the compounds used as starting materials. In general, we find it convenient to carry out the reaction at a temperature of from -10°C to 80°C, more preferably from 0°C to 50°C. The time required for the reaction may also vary widely, depending on many factors, notably the reaction temperature and the nature of the reagents and solvent employed. However, provided that the reaction is effected under the preferred conditions outlined above, a period of from 1 to 24 hours, more preferably from 4 to 16 hours, will usually suffice.

After completion of the reaction, the reaction product of formula (IVc) can be recovered from the reaction mixture by conventional means. One suitable recovery technique comprises: adding water to the residue; extracting it with a water-immiscible organic solvent, such as ethyl acetate; drying the extract over a drying agent, such as anhydrous magnesium sulphate; and distilling off the solvent. The product may, if necessary, be further purified by conventional means, for example, by recrystallisation, or by the various chromatography techniques, notably by column chromatography.

In this reaction, in order to sulphonylate the group of formula H-O-R²- without affecting the hydroxy group present in the substituent at the 4-position on the imidazole ring [that is in the group of formula -C(OH)R³R⁴], we prefer that the reaction should only be applied to compounds in which the groups represented by R³ and R⁴ are alkyl groups and where the hydroxy group is linked to a primary or secondary carbon atom [such as -CH₂- or -CH(CH₃)-] in the group represented by R².

### Step C2:

In Step C2, a compound of formula (IVd) is prepared by reacting the compound (IVc) with a compound of formula R^{1c}XM (VIII).

The amount of the compound of formula (VIII) employed in this reaction is not critical, although we prefer to employ at least an equimolar amount of the compound of formula (VIII) with respect to the compound of formula (IVc). More preferably, we employ from 1 to 3 moles, still more preferably from 1 to 2 moles, of the compound of formula (VIII) per mole of the compound of formula (IVc).

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: hydrocarbons, such as benzene, toluene or hexane; halogenated hydrocarbons, especially halogenated aliphatic hydrocarbons, such as methylene chloride or chloroform; ethers, such as diethyl ether, tetrahydrofuran or dioxane; alcohols, such as methanol, ethanol or t-butanol [preferably, where the compound of formula (VIII) is an alkali metal alkoxide, the alcohol corresponding to this alkoxide]; ketones, such as acetone or methyl ethyl ketone; amides, such as N,N-dimethylformamide or N,N-dimethylacetamide; and sulphoxides, such as dimethyl sulphoxide. Of these, we prefer the ethers, alcohols, ketones or amides.

The reaction can take place over a wide range of temperatures, and the precise reaction temperature is not critical to the invention, although the preferred reaction temperature will depend on the nature of the compounds used as starting materials. In general, we find it convenient to carry out the reaction at a temperature of from -10°C to 120°C, more preferably from 0°C to 100°C. The time required for the reaction may also vary widely, depending on many factors, notably the reaction temperature and the nature of the reagents and solvent employed. However, provided that the reaction is effected under the preferred conditions outlined above, a period of from 30 minutes to 24 hours, more preferably from 1 to 16 hours, will usually suffice.

After completion of the reaction, the reaction product of formula (IVd) can be recovered from the reaction mixture by conventional means, for example, in a similar manner to that described in step A1 in Reaction Scheme A.

### Step C3:

In Step C3, a compound of formula (Id) is prepared from the compound of formula (IVd) in a similar manner to that described above in relation to reactions A2(a) to A2(d) in step A2 of Reaction Scheme A.

### Preparation of Starting Materials

Many of the starting materials used in these reactions are well known compounds and others can be prepared by well known reactions commonly employed for analogous compounds. The starting materials of formulae (IIa) and (V) used in Reaction Schemes A and B may be prepared as illustrated in Reaction Schemes D to G as follows:

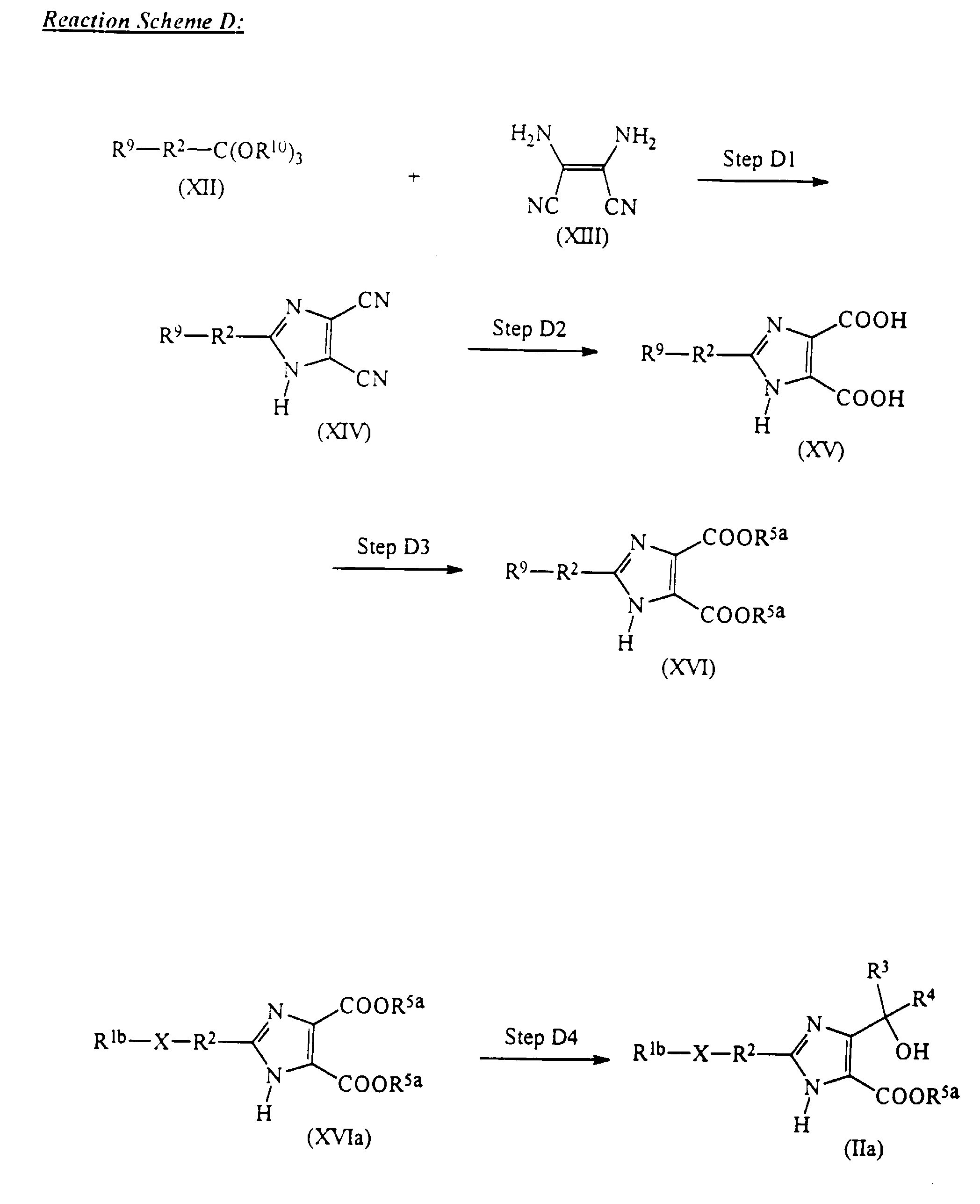

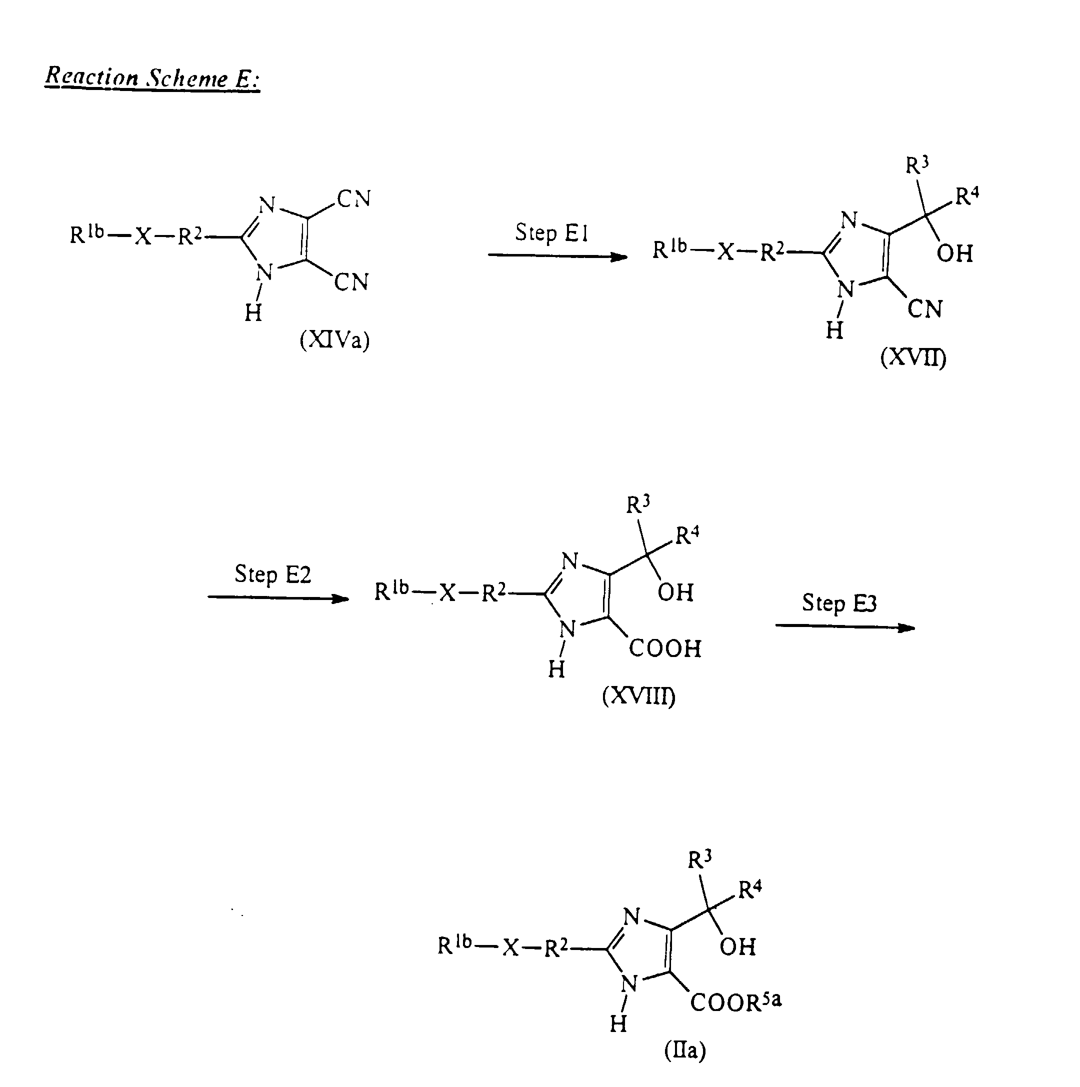

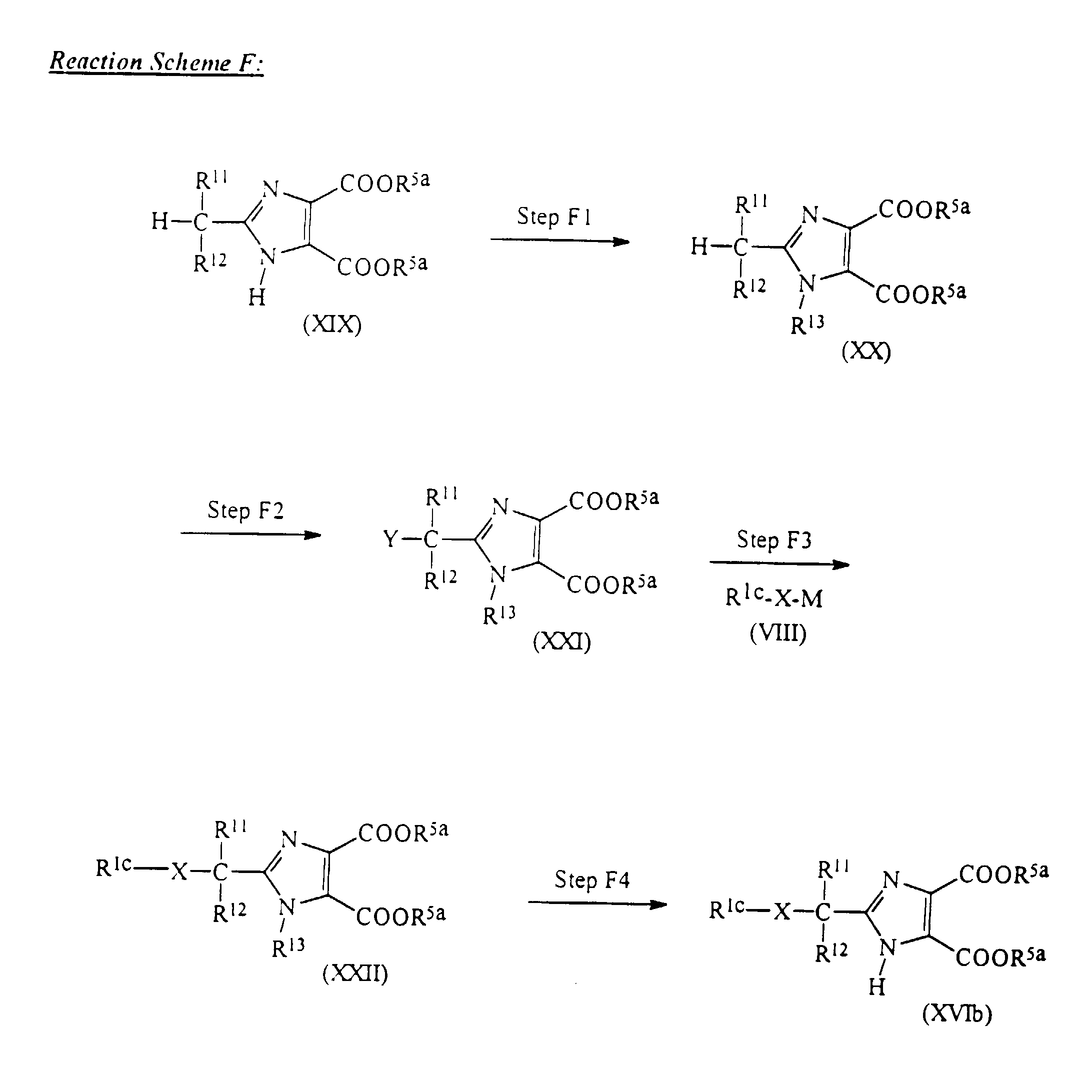

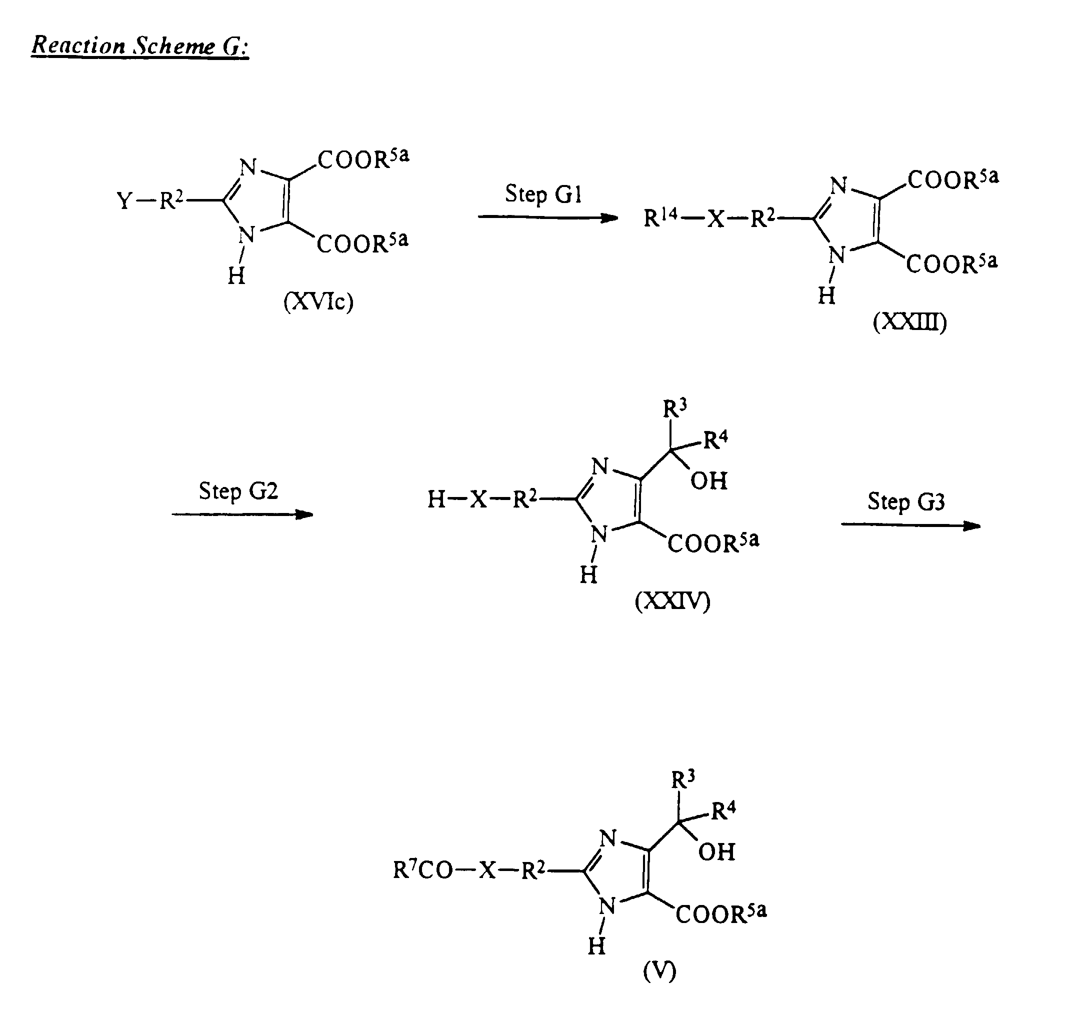

In the above formulae:
R^{1b}, R^{1c}, R², R³, R⁴, R^{5a}, R⁷, X, Y and M are as defined above;
R⁹ represents a halogen atom, preferably a chlorine, bromine or iodine atom, or a group of formula R^{1b}-X-(where R^{1b} and X are as defined above );
R¹⁰ represents an alkyl group having from 1 to 6 carbon atoms, preferably a methyl or ethyl group;
R¹¹ and R¹² are the same or different and each represents a hydrogen atom or an alkyl group having from 1 to 3 carbon atoms, provided that the total number of the carbon atoms in the atoms or groups represented by R¹¹ and R¹² is 3 or less;
R¹³ represents an imidazolyl-protecting group; and
R¹⁴ represents an alkanoyl group having from 2 to 6 carbon atoms.

Examples of imidazolyl-protecting groups which may be represented by R¹³ include: aralkyl groups in which an alkyl group having from 1 to 4 carbon atoms is substituted by at least one (preferably from 1 to 3) aryl groups, which themselves can optionally be substituted by at least one nitro group or alkoxy group having from 1 to 4 carbon atoms, for example the benzyl, p-nitrobenzyl, p-methoxybenzyl, diphenylmethyl and trityl groups; and alkoxymethyl groups, in which the alkoxy part has from 1 to 4 carbon atoms, such as the methoxymethyl, ethoxymethyl, propoxymethyl and butoxymethyl groups. Of these, we prefer the benzyl, p-nitrobenzyl, p-methoxybenzyl, trityl, methoxymethyl and ethoxymethyl groups, more preferably the benzyl or trityl groups.

### Reaction Scheme D

Reaction Scheme D consists of the preparation of a compound of formula (IIa).

### Step D1:

In Step D1, an imidazole-4,5-dicarbonitrile of formula (XIV) is prepared by reacting an orthoester compound of formula (XII) with diaminomaleonitrile of formula (XIII), which reaction may be carried out by a conventional method [such as that of R. W. Begland et al., J. Org. Chem., 39, 2341 (1974)]. In this reaction, an orthoester compound of formula (XII) is reacted with diaminomaleonitrile in an inert solvent. There is no particular restriction on the nature of the solvent to be employed, provided that it has no adverse effect on the reaction or on the reagents involved and that it can dissolve the reagents, at least to some extent. Examples of suitable solvents include: aromatic hydrocarbons, such as benzene, toluene or xylene; halogenated hydrocarbons, such as 1,2-dichloroethane and carbon tetrachloride; ethers, such as tetrahydrofuran and dioxane; and nitriles, such as acetonitrile.

The reaction can take place over a wide range of temperatures, and the precise reaction temperature is not critical to the invention. In general, we find it convenient to carry out the reaction at a temperature of from 50°C to 180°C, more preferably from 80°C to 150°C. The time required for the reaction may also vary widely, depending on many factors, notably the reaction temperature and the nature of the reagents and solvent employed. However, provided that the reaction is effected under the preferred conditions outlined above, a period of from 1 to 24 hours, more preferably from 2 to 10 hours, will usually suffice.

The reaction product of formula (XIV) can be recovered from the reaction mixture by collecting by filtration the crystals which appear or by distilling off the solvent. If necessary, the product can be further purified by conventional means, for example, by recrystallisation, or by the various chromatography techniques, notably by column chromatography.

### Step D2:

Step D2 consists of preparing a compound of formula (XV) by heating the compound of formula (XIV) under reflux for a suitable period, for example from 1 to 20 hours (more preferably from 3 to 17 hours), in the presence of an aqueous mineral acid, such as aqueous hydrochloric acid, aqueous sulphuric acid or aqueous nitric acid. The reaction product of formula (XV) can be recovered by collecting the crystals which deposit upon cooling by filtration or by distilling off the solvent.

### Step D3:

Step D3 consists of preparing a compound of formula (XVI) by protecting the carboxy group of the compound of formula (XV). This reaction is essentially the same as that of, and may be carried out in a similar manner to that described in, reaction A2(d) in step A2 of Reaction Scheme A described above.

### Step D4:

In Step D4, a compound of formula (IIa) is prepared by reacting a compound of formula (XVIa), which is a compound of formula (XVI) where R⁹ represents a group of formula R^{1b}-X- (in which R^{1b} and X are as defined above), with Grignard reagents of formulae(XXV) and (XXVa):

R^{3a}-Mg-Y (XXV)

R^{4a}-Mg-Y (XXVA)

(in which Y is as defined above and R^{3a} and R^{4a} are the same or different and each represents an alkyl group having from 1 to 6 carbon atoms).

The compound of formula (IIa) where R³ and R⁴ are the same or different and each represents an alkyl group having from 1 to 6 carbon atoms is prepared by reacting the compound of formula (XVIa) with approximately 2 moles of the Grignard reagent of formula (XXV) and then with the Grignard reagent of formula (XXVa). Further, the compound of formula (IIa) where R³ and R⁴ are the same alkyl group having from 1 to 6 carbon atoms is prepared by reacting the compound of formula (XVIa) with approximately 3 moles or more (preferably from 3 to 4 moles) of the Grignard reagent of formula (XXV) or (XXVa).

The reaction between the compound of formula (XVIa) and the Grignard reagent is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: hydrocarbons, such as hexane or toluene; ethers, such as tetrahydrofuran or diethyl ether; and halogenated hydrocarbons, such as methylene chloride. Of these, we prefer the ethers or the halogenated hydrocarbons.

The reaction can take place over a wide range of temperatures, and the precise reaction temperature is not critical to the invention. In general, we find it convenient to carry out the reaction at a temperature of from -50°C to 100°C, more preferably from -10°C to 50°C. The time required for the reaction may also vary widely, depending on many factors, notably the reaction temperature and the nature of the reagents and solvent employed. However, provided that the reaction is effected under the preferred conditions outlined above, a period of from 30 minutes to 24 hours, more preferably from 1 to 16 hours, will usually suffice.

After completion of the reaction, the desired product of each reaction can be recovered from the reaction mixture by conventional means. One suitable recovery technique comprises: adding water or an aqueous ammonium chloride solution to the reaction solution; stirring the resulting mixture at room temperature; filtering off insoluble matter, if present; extracting the mixture with a water-immiscible organic solvent, such as ethyl acetate; washing the extract with water; drying the extract over a drying agent, such as anhydrous magnesium sulphate; and distilling off the solvent. The product may, if necessary, be further purified by conventional means, for example, by recrystallisation, or by the various chromatography techniques, notably by column chromatography.

### Reaction Scheme E

Reaction Scheme E provides an alternative method of preparing the compound of formula (IIa).

### Step E1:

In Step E1, a compound of formula (XVII) is prepared by reacting a compound of formula (XIVa), which is a compound of formula (XIV) where R⁹ represents a group of formula R^{1b}-X- (in which R^{1b} and X are as defined above), with Grignard reagents of formulae (XXV) and XXVa). This reaction is essentially the same as that described in, and may be carried out in a similar manner to that described in, step D4 of Reaction Scheme D described above.

The compound of formula (XVII) where R³ and R⁴ are the same or different and each represents an alkyl group having from 1 to 6 carbon atoms is prepared by reacting the compound of formula (XIVa) with approximately 2 moles of the Grignard reagent of formula (XXV) and then with the Grignard reagent of formula (XXVa). The compound of formula (XVII) where R³ and R⁴ are the same alkyl group having from 1 to 6 carbon atoms is prepared by reacting the compound of formula (XVIa) with approximately 3 or more moles of the Grignard reagent of formula (XXV) or (XXVa).

### Step E2:

In Step E2 a compound of formula (XVIII) is prepared by hydrolyzing the cyano group of the compound of formula (XVII) with an alkali or an acid.

Hydrolysis with an alkali may be carried out by reacting the compound of formula (XVII) with a base (preferably an alkali metal hydroxide such as lithium hydroxide, sodium hydroxide or potassium hydroxide) in an inert solvent (preferably an alcohol, such as methanol or ethanol; an ether, such as tetrahydrofuran or dioxane; water; or a mixture of water and any one or more of the above organic solvents).

The reaction can take place over a wide range of temperatures, and the precise reaction temperature is not critical to the invention. In general, we find it convenient to carry out the reaction at a temperature of from 0°C to 120°C, more preferably from 20°C to 100°C. The time required for the reaction may also vary widely, depending on many factors, notably the reaction temperature and the nature of the reagents and solvent employed. However, provided that the reaction is effected under the preferred conditions outlined above, a period of from 30 minutes to 24 hours, more preferably from 1 hour to 16 hours, will usually suffice.

After completion of the reaction, the product can be recovered a conventional recovery procedure, for example as follows: neutralising the reaction mixture with a mineral acid, such as hydrochloric acid; collecting the crystals which appear in the reaction system by filtration, or distilling off the solvent. An alternative recovery procedure comprises: adding water and a water-immiscible organic solvent to the neutralised reaction mixture; separating the organic layer; washing the organic layer with water and then drying; and distilling off the solvent. If necessary, the product can be further purified by conventional means, for example, by recrystallisation, or by the various chromatography techniques, notably by column chromatography.

Hydrolysis with an acid may be carried out in a similar manner to that described in step D2 of Reaction Scheme D described above.

### Step E3:

In Step E3, a compound of formula (IIa) is prepared by protecting the carboxy group in the compound of formula (XVIII). This reaction is essentially the same as that of, and may be carried out in a similar manner to that described in, reaction A2(d) in Step A2 of Reaction Scheme A described above.

### Reaction Scheme F

Reaction Scheme F consists of an alternative method of preparing a compound of formula (XVIb), which is a compound of formula (XVI) where R⁹ represents a group of formula R^{1c}-X- (in which R^{1c} and X are as defined above) and R² represents a group of formula -C(R¹¹) (R¹²)- (in which R¹¹ and R¹² are as are as defined above and preferably R¹¹ and R¹² are both hydrogen atoms).

### Step F1:

In Step F1, a compound of formula (XX) is prepared by protecting the imidazolyl group of the compound of formula (XIX).

This reaction may be carried out by reacting a compound of formula (XIX) with a compound of formula (XXVI):

R¹³-Y (XXVI)

(in which R¹³ and Y are as defined above). The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: halogenated hydrocarbons, such as methylene chloride or chloroform; ethers, such as tetrahydrofuran or dioxane; amides, such as N,N-dimethylformamide or N,N-dimethylacetamide; and ketones, such as acetone or methyl ethyl ketone.

The reaction can take place over a wide range of temperatures, and the precise reaction temperature is not critical to the invention. In general, we find it convenient to carry out the reaction at a temperature of from 0°C to 120°C, more preferably from 20°C to 80°C. The time required for the reaction may also vary widely, depending on many factors, notably the reaction temperature and the nature of the reagents and solvent employed. However, provided that the reaction is effected under the preferred conditions outlined above, a period of from 1 to 24 hours, more preferably from 3 to 8 hours, will usually suffice.

The reaction product of formula (XX) can be recovered by adding-water to the reaction mixture, extracting the mixture with a water-immiscible organic solvent, washing the extract with water, drying it, and distilling off the solvent. The product may, if necessary, be further purified by conventional means, for example, by recrystallisation, or by the various chromatography techniques, notably by column chromatography.

### Step F2:

In Step F2, a compound of formula (XXI) is prepared by halogenating the compound of formula (XX).

This reaction may be carried out by reacting the compound of formula (XX) with a halogenating agent (preferably N-chlorosuccinimide, N-bromosuccinimide, N-iodosuccinimide or 1,3-dibromo-5,5-dimethyl-hydantoin). The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: halogenated hydrocarbons, such as methylene chloride, 1,2-dichloroethane and carbon tetrachloride. The reaction is effected in the presence of a catalyst, preferably benzoyl peroxide or azobisisobutyronitrile.

The reaction can take place over a wide range of temperatures, and the precise reaction temperature is not critical to the invention. In general, we find it convenient to carry out the reaction at a temperature of from 0°C to 100°C, more preferably from 20°C to 80°C. The time required for the reaction may also vary widely, depending on many factors, notably the reaction temperature and the nature of the reagents and solvent employed. However, provided that the reaction is effected under the preferred conditions outlined above, a period of from 10 minutes to 24 hours, more preferably from 30 minutes to 16 hours, will usually suffice.

If desired, the reaction may be remarkably accelerated by carrying it out under the irradiation of a tungsten lamp.

The reaction product of formula (XXI) can be recovered by washing the reaction mixture with water, drying over a drying agent, such as anhydrous magnesium sulphate, and distilling off the solvent. The product can, if necessary, be further purified by conventional means, for example, by recrystallisation, or by the various chromatography techniques, notably by column chromatography.

### Step F3:

In Step F3, a compound of formula (XXII) is prepared by reacting the compound of formula (XXI) with a compound of formula (VIII). This reaction is essentially the same as that of, and may be carried out in a similar manner to that described in, Step C2 of Reaction Scheme C.

### Step F4:

In Step F4, a compound of formula (XVIb) is prepared by deprotecting the imidazolyl-protecting group in the compound of formula (XXII). The reaction employed to remove the protecting group will vary depending upon the nature of the protecting group, although all are well-known methods in organic synthetic chemistry.

For example, where the imidazolyl-protecting group is a trityl or alkoxymethyl group, it may be removed by reacting the protected compound with an acid (preferably a mineral acid, such as hydrogen chloride or sulphuric acid, or an organic acid, such as formic acid, acetic acid, trifluoroacetic acid, methanesulphonic acid or p-toluenesulphonic acid). The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: alcohols, such as methanol or ethanol; ethers, such as tetrahydrofuran or dioxane; fatty acids, such as acetic acid; water; or a mixture of water and any one or more of the above organic solvents.

The reaction can take place over a wide range of temperatures, and the precise reaction temperature is not critical to the invention. In general, we find it convenient to carry out the reaction at a temperature of from 0°C to 120°C, more preferably from 10°C to 100°C. The time required for the reaction may also vary widely, depending on many factors, notably the reaction temperature and the nature of the reagents and solvent employed. However, provided that the reaction is effected under the preferred conditions outlined above, a period of from 30 minutes to 24 hours, more preferably from 1 hour to 16 hours, will usually suffice.

After completion of the reaction, the product can be recovered by conventional means. For example, one suitable recovery procedure comprises: distilling off the solvent and purifying the product, for example, by recrystallisation; or neutralising the reaction mixture with a weakly basic aqueous solution, such as an aqueous solution of sodium hydrogencarbonate, extracting the mixture with a water-immiscible organic solvent, and distilling off the solvent. If necessary, the product can be further purified by conventional means, for example, by recrystallisation, or by the various chromatography techniques, notably by column chromatography.

Where the imidazolyl-protecting group is an aralkyl group, such as a benzyl, p-nitrobenzyl or diphenylmethyl group, it may be removed by reacting a similar reaction to that described in the catalytic reduction reaction of reaction A2(a) in Step A2 of Reaction Scheme A described above. In this reaction, the reaction may often be accelerated by adding from 1 to 3 moles of aqueous hydrochloric acid or p-toluenesulphonic acid to the reaction system.

### Reaction Scheme G

In Reaction Scheme G, a compound of formula (V) is prepared.

### Step G1:

In Step G1, a compound of formula (XXIII) is prepared by reacting a compound of formula (XVIc), which is a compound of formula (XVI) where R⁹ represents a halogen atom, with a compound of formula (XXVII):

R¹⁴-X-M (XXVII)

(in which R¹⁴, X and M are as defined above). The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: amides, such as N,N-dimethylformamide or N,N-dimethylacetamide; and ketones, such as acetone or methyl ethyl ketone.

The reaction can take place over a wide range of temperatures, and the precise reaction temperature is not critical to the invention. In general, we find it convenient to carry out the reaction at a temperature of from 0°C to 120°C, more preferably from 20°C to 80°C. The time required for the reaction may also vary widely, depending on many factors, notably the reaction temperature and the nature of the reagents and solvent employed. However, provided that the reaction is effected under the preferred conditions outlined above, a period of from 30 minutes to 24 hours, more preferably from 1 to 16 hours, will usually suffice.

After completion of the reaction, the reaction product of formula (XXIII) can be recovered from the reaction mixture by conventional means, for example, in a similar manner to that described in Step A1 of Reaction Scheme A described above.

### Step G2:

In Step G2, a compound of formula (XXIV) is prepared by reacting a compound of formula (XXIII) with Grignard reagents of formulae (XXV) and (XXVa). This reaction is essentially the same as that of, and may be carried out in a similar manner to that described in, Step D4 of Reaction Scheme D described above.

### Step G3:

In Step G3, a compound of formula (V) is prepared by acylating a compound of formula (XXIV). This reaction may be carried out using an arylcarbonyl halide (such as benzoyl chloride, p-methylbenzoyl chloride, p-methoxybenzoyl chloride, p-chlorobenzoyl chloride or naphthoyl chloride) or an alkanoyl halide or an acid anhydride, as described in reaction A2(f) in Step A2 of Reaction Scheme A described above.

The product of this reaction may be recovered from the reaction mixture by conventional means, for example as described in Step A2(f) of Reaction Scheme A above.

The compounds of the present invention exhibit an excellent inhibitory effect against the elevation of blood pressure induced by angiotensin II and are therefore extremely useful for prevention or treatment of circulatory diseases as a hypotensive drug or a therapeutic drug for cardiovascular diseases.

Their biological activity was determined by the following experiment.

### Evaluation of All receptor blocking activity by Inhibition of pressor response to angiotensin II

The biological activity of each compound was assessed by determining the dose required to inhibit the pressor response to intravenous angiotensin II by fifty percent (ID₅₀) in rats. Male Wister-Imamichi rats, each weighing 300 to 400 g, were anaesthesized by intraperitoneal injection of 100 mg/Kg of sodium thiobutabarbital [Inactin (trade name)] and two cannulae were inserted: one into the femoral artery for measuring blood pressure and the other into the femoral vein for injecting drugs. Fifty ng/kg of angiotension II were intravenously administered at intervals of about 10 minutes, and the elevation of blood pressure (normally about 50 mmHg) was observed. After constant pressor responses to angiotensin II were obtained, a test compound was intravenously administered. Two minutes later, angiotension II was again injected, and the inhibitory effect of the test compound was estimated. The percent inhibitions of the pressor response to angiotensin II by progressive increase of the test compound was used to calculate the value of ID₅₀. Angiotensin II was used in this test dissolved in 0.5 % bovine serum albumin (BSA) and the test compounds were dissolved in 100% dimethyl sulphoxide (DMSO). Table 2 shows the ID₅₀ values thus determined.

The compounds of the invention are identified hereafter by the number of the one of the following Examples which illustrates their preparation.

**Table 2**

| Test compound (Compound of Example No.) | ID50 (mg/kg, i.v.) |
|---|---|
| 2 | 0.0066 |
| 6 | 0.0059 |
| 10 | 0.016 |
| 14 | 0.074 |
| 22 | 0.025 |
| 25 | 0.026 |
| 27 | 0.019 |

The compounds of the present invention can be administered, for example, orally in the form of tablets, capsules, granules, powders, syrups or the like, or parenterally by injection, suppository or the like. These pharmaceutical preparations can be produced in the conventional manner using the adjuvants generally known in the art, such as excipients, binders, disintegrating agents, lubricants, stabilisers, corrigents and the like. Although the dosage may vary depending upon the symptoms and age of the patient, the nature and severity of the disease or disorder and the route and manner of administration, in the case of oral administration to an adult human patient, the compounds of the present invention may normally be administered at a total daily dose of from 1 to 1000 mg, preferably from 5 to 300 mg, either in a single dose, or in divided doses, for example one to three times a day; in the case of intravenous injection, a dose of from 0.1 to 100 mg, preferably from 0.5 to 30 mg, may be administered from one to three times a day.

The invention is further illustrated by the following Examples, which demonstrate the preparation of various of the compounds of the invention. The preparation of certain starting materials used in these Examples is shown in the subsequent Preparations.

### EXAMPLE 1

### Methyl 4-(1-hydroxy-1-methylethyl)-2-methoxymethyl-1-{4-[2- (tetrazol-5-yl)phenyl]phenyl}methyl-imidazole-5-carboxylate

### 1(a) Methyl 4-(1-hydroxy-1-methylethyl)-2-methoxy-methyl-1-{4-[2-(trityltetrazol-5-yl)phenyl]phenyl}-methylimidazole-5-carboxylate

194 mg of potassium t-butoxide were added, whilst ice-cooling, to a solution of 359 mg of methyl 4-(1-hydroxy-1-methylethyl)-2-methoxymethylimidazole-5-carboxylate [prepared as described in Preparation 1(v)] in 5 ml of N,N-dimethylacetamide, and the resulting mixture was stirred for 15 minutes. At the end of this time, a solution of 1.32 g of 4-[2-(trityltetrazol-5-yl)phenyl]benzyl bromide in 10 ml of N,N-dimethylacetamide was added. The mixture was first stirred at room temperature for 4 hours and then at 50°C for a further 2 hours. The reaction mixture was then mixed with water and extracted with ethyl acetate. The extract was dried over anhydrous magnesium sulphate, and the solvent was removed by distillation under reduced pressure. The resulting residue was purified by column chromatography through silica gel, using a 1 : 2 by volume mixture of hexane and ethyl acetate as the eluent, to give 848 mg of the title compound as crystals, melting at 120 - 137°C (with decomposition).

Nuclear Magnetic Resonance Spectrum (CDCℓ₃), δ ppm:
1.64 (6H, singlet);
3.29 (3H, singlet);
3.63 (3H, singlet);
4.36 (2H, singlet);
5.49 (2H, singlet);
5.56 (1H, singlet);
6.76 (2H, doublet, J = 8 Hz);
6.95 (6H, doublet, J = 7 Hz);
7.09 (2H, doublet, J = 8 Hz);
7.23 - 7.53 (12H. multiplet);
7.89 (1H, doublet, J = 7 Hz).

### 1(b) Methyl 4-(1-hydroxy-1-methylethyl)-2-methoxy-methyl-1-{4-[2-(tetrazol-5-yl)phenyl]phenyl}methyl-imidazole-5-carboxylate

705 mg of methyl 4-(1-hydroxy-1-methylethyl)-2-methoxymethyl-1-{4-[2-(trityltetrazol-5-yl)phenyl]-phenyl}methylimidazole-5-carboxylate [prepared as described in step (a) above] were dissolved in 10 ml of a 25% aqueous solution of acetic acid, and the mixture was stirred at 60°C for 4 hours. At the end of this time, 10 ml of water were added, whilst ice-cooling, and the trityl alcohol which appeared as crystals was filtered off. The filtrate was concentrated by distillation under reduced pressure, and then acetic acid and water were distilled off as azeotropic mixtures with benzene, to give 460 mg of the title compound as an amorphous powder.

Nuclear Magnetic Resonance Spectrum (CDCℓ₃), δ ppm:
1.54 (6H, singlet);
3.34 (3H, singlet);
3.75 (3H, singlet);
4.45 (2H, singlet);
5.54 (2H, singlet);
6.89 (2H, doublet, J = 8 Hz);
7.09 (2H, doublet, J = 8 Hz);
7.42 - 7.62 (3H, multiplet);
7.93 (1H, doublet, J = 7 Hz).

### EXAMPLE 2

### 4-(1-Hydroxy-1-methylethyl)-2-methoxymethyl-1-{4-[2-(tetrazol-5-yl)phenyl]phenyl}methylimidazole-5-carboxylic acid

A solution of 462 mg of methyl 4-(1-hydroxy-1-methylethyl)-2-methoxymethyl-1-{4-[2-(tetrazol-5-yl)-phenyl]phenyl}methylimidazole-5-carboxylate [prepared as described in Example 1(b)] in 4 ml of a 1 N aqueous solution of sodium hydroxide was stirred at room temperature for 5 hours. At the end of this time, the insoluble matter was filtered off, and 4 ml of a 1 N aqueous solution of hydrochoric acid were added to the filtrate. The resulting crystalline powder was then collected by filtration, to give 338 mg of the title compound, melting at 187°C (with decomposition at 192 - 195°C).
Nuclear Magnetic Resonance Spectrum (hexadeuterated dimethyl sulphoxide), δ ppm:
1.54 (6H, singlet);
3.20 (3H, singlet);
4.42 (2H, singlet);
5.63 (2H, singlet);
6.96 (2H, doublet, J = 8 Hz);
7.05 (2H, doublet, J = 8 Hz);
7.52 - 7.70 (4H, multiplet).

### EXAMPLE 3

### (5-Methyl-2-oxo-1,3-dioxolen-4-yl)methyl 4-(1-hydroxy-1-methylethyl)-2-methoxymethyl-1-{4-[2-(tetrazol-5-yl)phenyl]phenyl}methylimidazole-5-carboxylate

### 3(a) (5-Methyl-2-oxo-1,3-dioxolen-4-yl)methyl 4-(1-hydroxy-1-methylethyl)-2-methoxymethyl-1-{4-[2-(trityltetrazol-5-yl)phenyl]phenyl}methylimidazole-5-carboxylate

15 ml of an aqueous solution containing 243 mg of lithium hydroxide monohydrate were added, whilst ice-cooling, to a solution of 2.72 g of methyl 4-(1-hydroxy-1-methylethyl)-2-methoxymethyl-1-{4-[2-(trityl-tetrazol-5-yl)phenyl]phenyl}methylimidazole-5-carboxylate [prepared as described in Example 1(a)] in 33 ml of dioxane, and the resulting mixture was stirred at 5 - 10°C for 16 hours. At the end of this time, a small piece of dry ice was added to the reaction solution, and the reaction solution was concentrated by distillation under reduced pressure to a volume of about 15 ml. The concentrate was mixed with ethyl acetate and a saturated aqueous solution of sodium chloride and stirred. The resulting reaction mixture was extracted with ethyl acetate, and the extract was dried over anhydrous sodium sulphate. The solvent was then removed by distillation under reduced pressure, to give a glassy salt of lithium 4-(1-hydroxy-1-methylethyl)-2-methoxy-methyl-1-{4-[2-(trityltetrazol-5-yl)phenyl]phenyl}-methylimidazole-5-carboxylate. The whole of this product was dissolved in 25 ml of N,N-dimethylacetamide, and 533 mg of potassium carbonate were added to the resulting solution, after which a solution of 1.13 g of 4-chloromethyl-5-methyl-2-oxo-1,3-dioxolene (purity degree: 74%) in 5.6 ml of N,N-dimethylacetamide was added dropwise to the mixture, whilst ice-cooling. The mixture was then stirred at 50°C for 3 hours, after which it was diluted with ethyl acetate and water. The mixture was then extracted with ethyl acetate, and the extract was dried over anhydrous magnesium sulphate and concentrated by distillation under reduced pressure. The resulting crystalline residue was washed with diethyl ether, to give 2.70 g of the title compound, melting at 144 - 146°C (with decomposition).

Nuclear Magnetic Resonance Spectrum (CDCℓ₃), δ ppm:
1.63 (6H, singlet);
1.98 (3H, singlet);
3.29 (3H, singlet);
4.37 (2H, singlet);
4.72 (2H, singlet);
5.42 (1H, singlet);
5.47 (2H, singlet);
6.70 (2H, doublet, J = 8.5 Hz);
6.96 (6H, doublet, J = 8.5 Hz);
7.09 (2H, doublet, J = 8.5 Hz);
7.24 - 7.55 (12H, multiplet);
7.88 (1H, doublet, J = 7 Hz).

### 3(b) (5-Methyl-2-oxo-1,3-dioxolen-4-yl)methyl 4-(1-hydroxy-1-methylethyl)-2-methoxymethyl-1-{4-[2-(tetrazol-5-yl)phenyl]phenyl}methylimidazole-5-carboxylate

Following a procedure similar to that described in Example 1(b), but detritylating 2.5 g of (5-methyl-2-oxo-1,3-dioxolen-4-yl)methyl 4-(1-hydroxy-1-methyl-ethyl) -2-methoxymethyl-1-{4-[2-(trityltetrazol-5-yl)-phenyl]phenyl}methylimidazole-5-carboxylate [prepared as described in step (a) above] with a 25% v/v aqueous solution of acetic acid, 916 mg of the title compound were obtained as crystals, melting at 138 - 140°C.

Nuclear Magnetic Resonance Spectrum (CDCℓ₃), δ ppm:
1.64 (6H, singlet);
2.21 (3H, singlet);
3.30 (3H, singlet);
4.44 (2H, singlet);
5.01 (2H, singlet);
5.60 (3H, singlet);
6.83 (2H, doublet, J = 8 Hz);
7.11 (2H, doublet, J = 8 Hz);
7.43 - 7.64 (3H, multiplet);
7.89 (1H, doublet, J = 8.5 Hz).

### EXAMPLE 4

### Pivaloyloxymethyl 4-(1-hydroxy-1-methylethyl)-2-methoxymethyl-1-{4-[2-(tetrazol-5-yl)phenyl]-phenyl}methylimidazole-5-carboxylate

### 4(a) Pivaloyloxymethyl 4-(1-hydroxy-1-methylethyl)-2-methoxymethyl-1-{4-[2-(trityltetrazol-5-yl)phenyl]-phenyl}methylimidazole-5-carboxylate

A solution of 41.9 mg of lithium hydroxide monohydrate in 15 ml of water was added, whilst ice-cooling, to a solution of 0.75 g of methyl 4-(1-hydroxy-1-methylethyl)-2-methoxymethyl-1-{4-[2-(trityl-tetrazol-5-yl)phenyl]phenyl}methylimidazole-5-carboxylate [prepared as described in Example 1(a)] in 15 ml of dioxane, and the mixture was stirred at room temperature overnight. At the end of this time, a small quantity of dry ice was added to the reaction solution, and the dioxane was removed by distillation under reduced pressure. The residue was then dissolved in a small quantity of an aqueous solution of sodium chloride and ethyl acetate. The ethyl acetate layer was separated, washed with an aqueous solution of sodium chloride and dried over anhydrous sodium sulphate. The solvent was removed by distillation under reduced pressure, and the resulting residue was dried in vacuo at 50°C for 1 hour. 0.25 g of potassium carbonate were then added to a solution of the resulting residue in 10 ml of N,N-dimethylacetamide, and the mixture was cooled with ice-water. A solution of 0.31 ml of pivaloyloxymethyl chloride in 3 ml of N,N-dimethylacetamide was then added dropwise to the mixture, which was then stirred at 70°C for 1.5 hours. At the end of this time, water and ethyl acetate were added to the reaction solution. The ethyl acetate layer was separated, washed with water and dried over anhydrous sodium sulphate. The solvent was removed by distillation under reduced pressure, and the resulting residue was purified by column chromatography through silica gel, using a 1 : 1 by volume mixture of hexane and ethyl acetate as the eluent, to give 0.79 g of the title compound as a foam-like solid.

Nuclear Magnetic Resonance Spectrum (CDCℓ₃), δ ppm:
1.14 (9H, singlet);
1.64 (6H, singlet);
3.28 (3H, singlet);
4.33 (2H, singlet);
5.24 (1H, singlet);
5.50 (2H, singlet);
5.71 (2H, singlet);
6.76 (2H, doublet, J = 8 Hz);
6.94 (6H, doublet, J = 7.5 Hz);
7.09 (2H, doublet, J = 8 Hz);
7.30 - 7.52 (12H, multiplet);
7.90 (1H, doublet, J = 9 Hz).

### 4(b) Pivaloyloxymethyl 4-(1-hydroxy-1-methylethyl)-2-methoxymethyl-1-{4[2-(tetrazol-5-yl)phenyl]phenyl}-methylimidazole-5-carboxylate

Following a procedure similar to that described in Example 1(b), but using a solution of 0.79 g of pivaloyloxymethyl 4-(1-hydroxy-1-ethylmethyl)-2-methoxy-methyl-1-{4- [2- (trityltetrazol-5-yl)phenyl]phenyl}-methylimidazole-5-carboxylate [prepared as described in step (a) above] as the starting material, 0.44 g of the title compound was obtained as crystals, melting at 71 - 72°C.

Nuclear Magnetic Resonance Spectrum (CDCℓ₃), δ ppm:
1.10 (9H, singlet).
1.63 (6H, singlet);
3.33 (3H, singlet);
5.40 (1H, broad singlet);
5.57 (2H, singlet);
5.82 (2H, singlet);
6.91 (2H, doublet, J = 8 Hz);
7.14 (2H, doublet, J = 8 Hz);
7.28 - 7.60 (3H, multiplet);
8.08 (1H, doublet, J = 8 Hz).

### EXAMPLE 5

### Ethyl 2-ethoxymethyl-4-(1-hydroxy-1-methylethyl)-1-{4-[2- (tetrazol-5-yl)phenyl]phenyl}methyl-imidazole-5-carboxylate

### 5(a) Ethyl 2-ethoxymethyl-4-(1-hydroxy-1-methylethyl)-1-{4-[2-(trityltetrazol-5-yl)phenyl]phenyl}methyl-imidazole-5-carboxylate

217 mg of potassium t-butoxide were added, whilst ice-cooling, to a solution of 450 mg of ethyl 2-ethoxymethyl-4-(1-hydroxy-1-methylethyl)imidazole-5-carboxylate [prepared as described in Preparation 3(iii)] in 5 ml of N,N-dimethylacetamide, and the mixture was stirred for 30 minutes. At the end of this time, a solution of 1.47 g of 4-[2-(trityltetrazol-5-yl)phenyl]benzyl bromide in 10 ml of N,N-dimethylacetamide was added dropwise to the mixture. The mixture was stirred at room temperature for 2 hours, after which it was mixed with ethyl acetate and water and shaken. The ethyl acetate layer was separated and dried over anhydrous magnesium sulphate. The solvent was removed by distillation under reduced pressure, and the resulting residue was purified by column chromatography through silica gel, using a 1 : 1 by volume mixture of hexane and ethyl acetate as the eluent, to give 1.2 g of the title compound as an amorphous powder.

Nuclear Magnetic Resonance Spectrum (CDCℓ₃), δ ppm:
1.08 (3H, triplet, J = 7 Hz);
1.13 (3H, triplet, J = 7 Hz);
1.64 (6H, singlet);
3.44 (2H, quartet, J = 7 Hz);
4.14 (2H, quartet, J = 7 Hz);
4.39 (2H, singlet);
5.54 (2H, singlet);
5.67 (1H, singlet);
6.75 (2H, doublet, J = 8 Hz);
6.96 (6H, doublet, J = 7 Hz);
7.09 (2H, doublet, J = 8 Hz);
7.23 - 7.52 (12H, multiplet);
7.88 (1H, doublet, J = 7 Hz).

### 5(b) Ethyl 2-ethoxymethyl-4-(1-hydroxy-1-methylethyl)-1-{4-[2-(tetrazol-5-yl)phenyl]phenyl}methylimidazole-5-carboxylate

A solution of 600 mg of ethyl 2-ethoxymethyl-4-(1-hydroxy-1-methylethyl)-1-{4-[2-(trityltetrazol-5-yl)-phenyl]phenyl}methylimidazole-5-carboxylate [prepared as described in step (a) above] in 10 ml of a 25% v/v aqueous solution of acetic acid was stirred at 60°C for 2 hours. 10 ml of water were then added, and the mixture was then cooled with ice. The trityl alcohol which appeared as crystals was filtered off. The filtrate was concentrated by distillation under reduced pressure, and then acetic acid and water were distilled off as azeotropic mixtures with toluene, to give 400 mg of the title compound in an amorphous powder.

Nuclear Magnetic Resonance Spectrum (CDCℓ₃), δ ppm:
1.13 (3H, triplet, J = 7 Hz);
1.15 (3H, triplet, J = 7 Hz);
3.49 (2H, quartet, J = 7 Hz);
4.40 (2H, singlet);
5.57 (2H, singlet);
6.82 (2H, doublet, J = 8 Hz);
7.05 (2H, doublet, J = 8 Hz);
7.40 - 7.61 (3H, multiplet);
7.84 (1H, doublet, J = 7 Hz).

### EXAMPLE 6

### 2-Ethoxymethyl-4-(1 -hydroxy-1-methylethyl)-1-{4-[2-(tetrazol-5-yl)phenyl]phenyl}methylimidazole-5-carboxylic acid

A solution of 400 mg of ethyl 2-ethoxymethyl-4-(1-hydroxy-1-methylethyl)-1-{4-[2-(tetrazol-5-yl)phenyl]-phenyl}methylimidazole-5-carboxylate [prepared as described in Example 5(b)] in 3.5 ml of a 1 N aqueous solution of sodium hydroxide was stirred at room temperature for 1 hour. Insoluble matter was then filtered off, and 3.5 ml of 1 N aqueous hydrochloric acid were added to the filtrate. The amorphous powder which precipitated was collected, to give 301 mg of the title compound, melting at 150°C (with softening).

Nuclear Magnetic Resonance Spectrum (hexadeuterated dimethyl sulphoxide), δ ppm:
0.96 (3H, triplet, J = 7 Hz);
1.54 (6H, singlet);
3.40 (2H, quartet, J = 7 Hz);
4.45 (2H, singlet);
5.63 (2H, singlet);
6.96 (2H, doublet, J = 8 Hz);
7.05 (2H, doublet, J = 8 Hz);
7.51 - 7.70 (4H, multiplet).

### EXAMPLE 7

### Pivaloyloxymethyl 2-ethoxymethyl-4-(1-hydroxy-1-methylethyl)-1-{4-[2-(tetrazol-5-yl)phenyl]-phenyl}methylimidazole-5-carboxylate

### 7(a) Pivaloyloxymethyl 2-ethoxymethyl-4-(1-hydroxy-1-methylethyl)-1-{4-[2- (trityltetrazol-5-yl)phenyl]-phenyl}methylimidazole-5-carboxylate

Following a procedure similar to that described in Example 4(a), but using 0.58 g of ethyl 2-ethoxymethyl-4-(1-hydroxy-1-methylethyl)-1-{4-[2-(trityltetrazol-5-yl)phenyl]phenyl}methylimidazole-5-carboxylate [prepared as described in Example 5(a)] as the starting material, 0.45 g of the title compound was obtained as a foam-like solid.

Nuclear Magnetic Resonance Spectrum (CDCℓ₃), δ ppm:
1.14 (9H, singlet);
1.14 (3H, triplet, J = 7 Hz);
1.63 (6H, singlet);
3.45 (2H, quartet, J = 7 Hz);
4.38 (2H, singlet);
5.25 (1H, singlet);
5.53 (2H, singlet);
5.71 (2H, singlet);
6.77 (2H, doublet, J = 8 Hz);
6.95 (6H, doublet, J = 7.5 Hz);
7.09 (2H, doublet, J = 8 Hz);
7.22 - 7.36 (10H, multiplet);
7.43 - 7.49 (2H, multiplet);
7.90 (1H, doublet, J = 9 Hz).

### 7(b) Pivaloyloxymethyl 2-ethoxymethyl-4-(1-hydroxy-1-methylethyl)-1-{4-[2-(tetrazol-5-yl)phenyl]phenyl}-methylimidazole-5-carboxylate

Following a procedure similar to that described in Example 1(b), but using 0.45 g of pivaloyloxymethyl 2-ethoxymethyl-4-(1-hydroxy-1-methylethyl)-1-{4-[2-(trityltetrazol-5-yl)phenyl]phenyl}methylimidazole-5-carboxylate [prepared as described in step (a) above] as the starting material, 0.28 g of the title compound was obtained as an amorphous powder, melting at 56 - 61°C.

Nuclear Magnetic Resonance Spectrum (CDCℓ₃), δ ppm:
1.07 (3H, triplet, J = 7 Hz);
1.10 (9H, singlet);
1.61 (6H, singlet);
3.48 (2H, quartet, J = 7 Hz);
4.50 (2H, singlet);
5.57 (2H, singlet);
5.80 (2H, singlet);
6.89 (2H, doublet, J = 8 Hz);
7.11 (2H, doublet, J = 8 Hz);
7.42 (1H, doublet, J = 7.5 Hz);
7.52 - 7.60 (2H, multiplet);
8.01 (1H, doublet, J = 7.5 Hz).

### EXAMPLE 8

### (5-Methyl-2-oxo-1,3-dioxolen-4-yl)methyl 2-ethoxymethyl-4-(1-hydroxy-1-methylethyl)-1-{4-[2-(tetrazol-5-yl)-phenyl]phenyl}methylimidazole-5-carboxylate

### 8(a) (5-Methyl-2-oxo-1,3-dioxolen-4-yl)methyl 2-ethoxy-methyl-4-(1-hydroxy-1-methylethyl)-1-{4-[2-(trityl-tetrazol-5-yl)phenyl]phenyl}methylimidazole-5-carboxylate

A solution of 51.5 mg of lithium hydroxide monohydrate in 8 ml of water was added to a solution of 600 mg of ethyl 2-ethoxymethyl-4-(1-hydroxy-1-methyl-ethyl)-1-{4-[2-(trityltetrazol-5-yl)phenyl]phenyl}-methylimidazole-5-carboxylate [prepared as described in Example 5(a)] in 19.5 ml of dioxane, whilst ice-cooling, and the mixture was stirred at 5 - 10°C for 16 hours. At the end of this time, a small piece of dry ice was added, and the reaction solution was concentrated by evaporation under reduced pressure down to approximately 8 ml. The concentrate was then mixed with ethyl acetate and sodium chloride and stirred. The ethyl acetate layer was separated and dried over anhydrous sodium sulphate, and the solvent was removed by distillation under reduced pressure, to give lithium 2-ethoxymethyl-4-(1-hydroxyl-methylethyl)-1-{4-[2-(trityltetrazol-5-yl)phenyl]phenyl}methylimidazole-5-carboxylate as an amorphous powder. The whole of this product was dissolved in 6 ml of N,N-dimethylacetamide, and 113 mg of potassium carbonate were added to the resulting solution, after which a solution of 240 mg of 4-chloromethyl-5-methyl-2-oxo-1,3-dioxolene (purity grade: 74%) in 2 ml of N,N-dimethylacetamide was added dropwise to the mixture. The mixture was then stirred at 50°C for 1 hour, after which it was mixed with ethyl acetate and water. The ethyl acetate layer was separated and dried over anhydrous magnesium sulphate, and then the solvent was removed by distillation under reduced pressure. The resulting residue was purified by column chromatography through silica gel, using a 3 : 1 by volume mixture of methylene chloride and ethyl acetate as the eluent, to give 548 mg of the title compound as crystals, melting at 129 - 130.5°C.

Nuclear Magnetic Resonance Spectrum (CDCℓ₃), δ ppm:
1.14 (3H, triplet, J = 7 Hz);
1.64 (6H, singlet);
1.99 (3H, singlet);
3.46 (2H, quartet, J = 7 Hz);
4.43 (2H, singlet);
4.73 (2H, singlet);
5.44 (1H, singlet);
5.51 (2H, singlet);
6.72 (2H, doublet, J = 8 Hz);
6.98 (6H, doublet, J = 7 Hz);
7.10 (2H, doublet, J = 8 Hz);
7.25 - 7.55 (12H, multiplet);
7.88 (1H, doublet, J = 8 Hz).

### 8(b) (5-Methyl-2 -oxo-1,3-dioxolen-4-yl)methyl 2-ethoxymethyl-4-(1-hydroxy-1-methylethyl)-1-{4-[2-(tetrazol-5-yl)phenyl]phenyl}methylimidazole-5-carboxylate

Following a procedure similar to that described in Example 1 (b), but detritylating 456 mg of (5-methyl-2-oxo-1,3-dioxolen-4-yl)methyl 2-ethoxymethyl-4-(1-hydroxy-1-methylethyl)-1-{4-[2-(trityltetrazol-5-yl)-phenyl]phenyl}methylimidazole-5-carboxylate [prepared as described in step (a) above] with a 25% v/v aqueous solution of acetic acid, 286 mg of the title compound were obtained as crystals, melting at 166 - 167.5°C.

Nuclear Magnetic Resonance Spectrum (CDCℓ₃), δ ppm:
1.03 (3H, triplet, J = 7 Hz);
1.64 (6H, singlet);
2.22 (3H, singlet);
3.44 (2H, quartet, J = 7 Hz);
4.48 (2H, singlet);
5.01 (2H, singlet);
5.62 (3H, singlet);
6.84 (2H, doublet, J = 8 Hz);
7.11 (2H, doublet, J = 8 Hz);
7.42 - 7.61 (3H, multiplet);
7.89 (1H, doublet, J = 8.5 Hz).

### EXAMPLE 9

### Propyl 4-(1-hydroxy-1-methylethyl)-2-propoxymethyl-1-{4-[2-(tetrazol-5-yl)phenyl]phenyl}methyl-imidazole-5-carboxylate

### 9(a) Propyl 4-(1-hydroxy-1-methylethyl)-2-propoxy-methyl-1-{4-[2-(trityltetrazol-5-yl)phenyl]phenyl}-methylimidazole-5-carboxylate

Following a procedure similar to that described in Example 1(a), but using 189 mg of propyl 4-(1-hydroxy-1-methylethyl)-2-propoxymethylimidazole-5-carboxylate [prepared as described in Preparation 4(iii)], 78 mg of potassium t-butoxide and 445 mg of 4-[2-(trityltetrazol-5-yl)phenyl]benzyl bromide as starting materials and then purifying the product by column chromatography through silica gel using a 1 : 1 mixture of hexane and ethyl acetate as the eluent, 395 mg of the title compound were obtained as a foam-like solid.

Nuclear Magnetic Resonance Spectrum (CDCℓ₃), δ ppm:
0.76 (3H, triplet, J = 7.5 Hz);
0.86 (3H, triplet, J = 7.5 Hz);
1.49 (2H, sextet, J = 7.5 Hz);
1.52 (2H, sextet. J = 7.5 Hz);
1.66 (6H, singlet);
3.34 (2H, triplet, J = 7.5 Hz);
4.06 (2H, triplet, J = 7.5 Hz);
4.37 (2H, singlet);
5.56 (2H, singlet);
5.70 (1H, singlet);
6.74 (2H, doublet, J = 8.5 Hz);
6.96 (6H, doublet, J = 7.5 Hz);
7.09 (2H, doublet, J = 8.5 Hz);
6.22 - 7.51 (12H, multiplet);
7.88 (1H, doublet, J = 8 Hz).

### 9(b) Propyl 4-(1-hydroxy-1-methylethyl)-2-propoxy-methyl-1-{4-[2-(tetrazol-5-yl)phenyl]phenyl}methyl-imidazole-5-carboxylate

Following a procedure similar to that described in Example 1(b), but using 394 mg of propyl 4-(1-hydroxy-1-methylethyl)-2-propoxymethyl-1-{4-[2-(trityltetrazol-5-yl)phenyl]phenyl}methylimidazole-5-carboxylate [prepared as described in step (a) above], 259 mg of the title compound were obtained as a foam-like solid.

Nuclear Magnetic Resonance Spectrum (CDCℓ₃), δ ppm:
0.83 (3H, triplet, J = 7 Hz);
0.85 (3H, triplet, J = 7 Hz);
1.45 - 1.60 (4H, multiplet);
1.50 (6H, singlet);
3.38 (2H, triplet, J = 6.5 Hz);
4.11 (2H, triplet, J = 7 Hz);
4.37 (2H, singlet);
5.58 (2H, singlet);
6.79 (2H, doublet, J = 8 Hz);
7.04 (2H, doublet, J = 8 Hz);
7.39 (1H, doublet, J = 8 Hz);
7.46 - 7.60 (2H, multiplet);
7.78 (1H, doublet, J = 7.5 Hz).

### EXAMPLE 10

### 4-(1-Hydroxy-1-methylethyl)-2-propoxymethyl-1-{4-[2-(tetrazol-5-yl)phenyl]phenyl}methylimidazole-5-carboxylic acid

394 mg of propyl 4-(1-hydroxy-1-methylethyl)-2-propoxymethyl-1-{4-[2-(tetrazol-5-yl)phenyl]phenyl}-methylimidazole-5-carboxylate [prepared as described in Example 9(b)] were dissolved in a solution of 88 mg of lithium hydroxide monohydrate in 10 ml of a 50% v/v aqueous solution of dioxane, and the mixture was stirred at room temperature for 3 hours. At the end of this time, the reaction solution was concentrated by distillation under reduced pressure, and the dioxane was removed by distillation under reduced pressure. The concentrate was then cooled with ice, and 2.1 ml of 1 N aqueous hydrochloric acid were added. The crystals which precipitated were collected by filtration, to give 235 mg of the title compound, melting at 166 - 168°C.

Nuclear Magnetic Resonance Spectrum (hexadeuterated dimethyl sulphoxide), δ ppm:
0.75 (3H, triplet, J = 7.5 Hz);
1.36 (2H, sextet, J = 7.5 Hz);
1.54 (6H, singlet);
3.32 (2H, triplet, J = 7.5 Hz);
4.46 (2H, singlet);
5.63 (2H, singlet);
6.96 (2H, doublet, J = 8 Hz);
7.05 (2H, doublet, J = 8 Hz);
7.50 - 7.70 (4H, multiplet).

### EXAMPLE 11

### Isopropyl 4-(1-hydroxy-1-methylethyl)-2-isopropoxy-methyl-1-{4-[2-(tetrazol-5-yl)phenyl]phenyl}-methylimidazole-5-carboxylate

### 11(a) Isopropyl 4-(1-hydroxy-1-methylethyl)-2-isopropoxymethyl-1-{4-[2-(trityltetrazol-5-yl)-phenyl]phenyl}methylimidazole-5-carboxylate

239 mg of potassium t-butoxide were added, whilst ice-cooling, to a solution of 550 mg of isopropyl 4-(1-hydroxy-1-methylethyl)-2-isopropoxymethylimidazole-5-carboxylate [prepared as described in Preparation 5(iii)] in 6 ml of N,N-dimethylacetamide, and the resulting mixture was stirred for 30 minutes. A solution of 1.62 g of 4-[2-(trityltetrazol-5-yl)phenyl]-benzyl bromide in 10 ml of N,N-dimethylacetamide was then added dropwise, and the mixture was stirred at room temperature for 2 hours. At the end of this time, the reaction mixture was mixed with water and ethyl acetate and shaken. The ethyl acetate layer was separated and dried over anhydrous magnesium sulphate, and the solvent was removed by distillation under reduced pressure. The resulting residue was purified by column chromatography through silica gel, using a 1 : 1 by volume mixture of hexane and ethyl acetate as the eluent, to give 1.47 g of the title compound as an amorphous powder.

Nuclear Magnetic Resonance Spectrum (CDCℓ₃), δ ppm:
1.06 (6H, doublet, J = 6.5 Hz);
1.10 (6H, doublet, J = 6 Hz);
3.57 (1H, septet, J = 6 Hz);
4.38 (2H, singlet); s
5.07 (1H, septet, J = 6.5 Hz);
5.56 (2H, singlet);
5.80 (1H, singlet);
6.73 (2H, doublet, J = 8 Hz);
6.96 (6H, doublet, J = 7 Hz);
7.10 (2H, doublet, J = 8 Hz);
7.23-7.52 (12H, multiplet);
7.86 (1H, doublet, J = 7 Hz).

### 11(b) Isopropyl 4-(1-hydroxy-1-methylethyl)-2-isopropoxymethyl-1-{4-[2-(tetrazol-5-yl}phenyl]-phenyl}methylimidazole-5-carboxylate

A solution of 609 mg of isopropyl 4-(1-hydroxy-1-methylethyl)-2-isopropoxymethyl-1-{4-[2-(trityltetrazol-5-yl)phenyl]phenyl}methylimidazole-5-carboxylate [prepared as described in step (a) above] in 10 ml of a 25% v/v aqueous solution of acetic acid was stirred at 60°C for 2.5 hours. 10 ml of water were then added, after which the mixture was cooled with ice. The trityl alcohol which appeared as crystals was filtered off. The filtrate was concentrated by distillation under reduced pressure, and then acetic acid and water were distilled off as azeotropic mixtures with benzene, to give 398 mg of the title compound as an amorphous powder.

Nuclear Magnetic Resonance Spectrum (CDCℓ₃), δ ppm:
1.13 (12H, doublet, J = 6 Hz);
1.51 (6H, singlet);
3.63 - 3.72 (1H, septet, J = 6 Hz);
4.37 (2H, singlet);
5.09 - 5.18 (1H, septet, J = 6 Hz);
5.62 (2H, singlet);
6.20 (1H, broad singlet);
6.85 (2H, doublet, J = 8 Hz);
7.12 (2H, doublet, J = 8 Hz);
7.39 (1H, doublet, J = 7.5 Hz);
7.51 - 7.63 (2H, multiplet);
7.92 (1H, doublet, J = 6.5 Hz).

### EXAMPLE 12

### 4-(1-Hydroxy-1-methylethyl)-2-isopropoxymethyl-1-{4-[2-(tetrazol-5-yl)phenyl]phenyl}methyl-imidazole-5-carboxylic acid

A solution of 393 mg of isopropyl 4-(1-hydroxy-1-methylethyl)-2-isopropoxymethyl-1-{4-[2-(tetrazol-5-yl)phenyl]phenyl}methylimidazole-5-carboxylate [prepared as described in Example 11(b)] in 3 ml of a 1 N aqueous solution of sodium hydroxide was stirred at room temperature for 2 hours, and then insoluble matter was filtered off. 3 ml of 1 N aqueous hydrochloric acid were added to the filtrate, and the precipitated amorphous powder was collected by filtration, to give 325 mg of the title compound, melting at 153 - 161°C (with softening).

Nuclear Magnetic Resonance Spectrum (hexadeuterated dimethyl sulphoxide), δ ppm:
1.00 (6H, doublet, J = 6 Hz);
1.54 (6H, singlet);
3.58 (1H, septet, J = 6 Hz);
4.43 (2H, singlet);
5.64 (2H, singlet);
6.96 (2H, doublet, J = 8.5 Hz);
7.05 (2H, doublet, J = 8.5 Hz);
7.50 - 7.69 (4H, multiplet).

### EXAMPLE 13

### Methyl 4-(1-hydroxy-1-methylethyl)-2-(1-methoxy-ethyl)-1-{4-[2-(tetrazol-5-yl)phenyl]phenyl}-methylimidazole-5-carboxylate

### 13(a) Methyl 4-(1-hydroxy-1-methylethyl)-2-(1 -methoxy-ethyl)-1-{4-[2-(trityltetrazol-5-yl)phenyl]phenyl}-methylimidazole-5-carboxylate

570 mg of potassium t-butoxide were added, whilst ice-cooling, to a solution of 1.12 g of methyl 4-(1-hydroxy-1-methylethyl)-2-(1-methoxyethyl)imidazole-5-carboxylate [prepared as described in Preparation 6(v)] in 11 ml of N,N-dimethylacetamide, and the mixture was stirred for 20 minutes, after which a solution of 3.86 g of 4-[2-trityltetrazol-5-yl)phenyl]benzyl bromide in 20 ml of N,N-dimethylacetamide was added dropwise to the reaction mixture. The reaction mixture was then stirred at room temperature for 2.5 hours and then mixed with ethyl acetate and water. The ethyl acetate layer was separated and dried over anhydrous magnesium sulphate, and the solvent was removed by distillation under reduced pressure. The resulting residue was purified by column chromatography through silica gel, using a 1 : 1 by volume mixture of hexane and ethyl acetate as the eluent, to give 1.69 g of the title compound as crystals, melting at 131 - 133°C.

Nuclear Magnetic Resonance Spectrum (CDCℓ₃), δ ppm:
1.44 (3H, doublet, J = 6.5 Hz);
1.63 (6H, singlet);
3.18 (3H, singlet);
3.57 (3H, singlet);
4.54 (1H, quartet, J = 6.5 Hz);
5.56 (2H, AB-quartet, Δδ = 0.17 ppm, J = 16.5 Hz);
5.59 (1H, singlet);
6.75 (2H, doublet, J = 8 Hz);
6.97 (6H, doublet, J = 7 Hz);
7.09 (2H, doublet, J = 8 Hz);
7.24 - 7.52 (12H, multiplet);
7.83 (1H, doublet, J = 7 Hz).

### 13(b) Methyl 4-(1-hydroxy-1-methylethyl)-2-(1-methoxy-ethyl)-1-{4-[2-(tetrazol-5-yl)phenyl]phenyl}methyl-imidazole-5-carboxylate

A solution of 600 mg of methyl 4-(1-hydroxy-1-methylethyl) -2-(1-methoxyethyl)-1-{4-[2-(trityltetrazol-5-yl)phenyl]phenyl}methylimidazole-5-carboxylate [prepared as described in step (a) above] in 10 ml of a 25% v/v aqueous solution of acetic acid was stirred at 60°C for 1.5 hours. The solution was then mixed with 10 ml of water and cooled with ice. The trityl alcohol which appeared as crystals was filtered off. The filtrate was concentrated by distillation under reduced pressure, and then acetic acid and water were distilled off as azeotropic mixtures with toluene, to give 331 mg of the title compound as an amorphous powder.

Nuclear Magnetic Resonance Spectrum (CDCℓ₃), δ ppm:
1.51 (3H, doublet, J = 6.5 Hz);
1.56 (6H, singlet);
3.23 (3H, singlet);
3.71 (3H, singlet);
4.63 (1H, quartet, J = 6.5 Hz);
5.61 (2H, AB-quartet, Δδ = 0.10 ppm, J = 16.5 Hz);
6.87 (2H, doublet, J = 8 Hz);
7.09 (2H, doublet, J = 8 Hz);
7.27 - 7.58 (3H, multiplet);
7.89 (1H, doublet, J = 7 Hz).

### EXAMPLE 14

### 4-(1-Hydroxy-1-methylethyl)-2-(1-methoxyethyl)-1-{4-[2- (tetrazol-5-yl)phenyl]phenyl}methyl-imidazole-5-carboxylic acid

A solution of 331 mg of methyl 4-(1-hydroxy-1-methylethyl)-2-(1-methoxyethyl)-1-{4-[2-(tetrazol-5-yl)phenyl]phenyl}methylimidazole-5-carboxylate [prepared as described in Example 13(b)] in 3 ml of a 1 N aqueous solution of sodium hydroxide was stirred at room temperature for 2.5 hours. At the end of this time, insoluble matter was filtered off and 3 ml of 1 N aqueous hydrochloric acid was added to the filtrate. The amorphous powder which precipitated was collected by filtration, to give 209 mg of the title compound, melting at 174 - 185°C (with softening).

Nuclear Magnetic Resonance Spectrum (hexadeuterated dimethyl sulphoxide), δ ppm:
1.35 (3H, doublet, J = 6.5 Hz);
1.55 (6H, singlet);
3.02 (3H, singlet);
4.54 (1H, quartet, J = 6.5 Hz);
5.70 (2H, AB-quartet, Δδ = 0.14 ppm, J = 16.5 Hz);
6.93 (2H, doublet, J = 8 Hz);
7.05 (2H, doublet, J = 8 Hz);
7.52 - 7.70 (4H, multiplet).

### EXAMPLE 15

### Methyl 1-[4-(2-carboxyphenyl)phenyl]methyl-4-(1-hydroxy-1-methylethyl)-2-methoxymethyl-imidazole-5-carboxylate

### 15(a) Methyl 1-{4-[2-(t-butoxycarbonyl)phenyl]-phenyl}methyl-4-(1-hydroxy-1-methylethyl)-2-methoxy-methylimidazole-5-carboxylate

Following a procedure similar to that described in Example 1(a), but using 230 mg of methyl 4-(1-hydroxy-1-methylethyl)-2-methoxymethylimidazole-5-carboxylate [prepared as described in Preparation 1(v)], 119 mg of potassium t-butoxide and 420 mg of 4-[2-(t-butoxy-carbonyl)phenyl]benzyl bromide and then purifying the product by column chromatography through silica gel using a 1 : 2 by volume mixture of hexane and ethyl acetate as the eluent, 468 mg of the title compound were obtained as a syrup.

Nuclear Magnetic Resonance Spectrum (CDCℓ₃), δ ppm:
1.24 (9H, singlet);
1.63 (6H, singlet);
3.38 (3H, singlet);
3.79 (3H, singlet);
4.54 (2H, singlet);
5.54 (1H, singlet);
5.62 (2H, singlet);
6.99 (2H, doublet, J = 8 Hz);
7.26 - 7.48 (5H, multiplet);
7.77 (1H, doublet, J = 7.5 Hz).

### 15(b) Methyl 1-[4-(2-carboxyphenyl)phenyl]methyl-4-(1-hydroxy-1-methylethyl)-2-methoxymethylimidazole-5-carboxylate

468 mg of methyl 1-{4-[2-(t-butoxycarbonyl)-phenyl]phenyl}methyl-4-(1-hydroxy-1-methylethyl)-2-methoxymethylimidazole-5-carboxylate [prepared as described in step (a) above] were dissolved in 10 ml of a 4 N solution of hydrogen chloride in dioxane, and the mixture was left at room temperature for 2 hours. At the end of this time, the reaction solution was concentrated and dried by evaporation under reduced pressure, to give 445 mg of the hydrochloride of the title compound as a foam-like solid.

Nuclear Magnetic Resonance Spectrum (CDCℓ₃), δ ppm:
1.72 (6H, singlet);
3.41 (3H, singlet);
3.80 (3H, singlet);
4.93 (2H, singlet);
5.65 (2H, singlet);
7.04 (2H, doublet, J = 8.5 Hz);
7.32 (3H, doublet, J = 8.5 Hz);
7.39 - 7.56 (2H, multiplet);
7.93 (1H, doublet, J = 6.5 Hz).

### EXAMPLE 16

### 1-[4-(2-Carboxyphenyl)phenyl]methyl-4-(1-hydroxy-1-methylethyl)-2-methoxymethylimidazole-5-carboxylic acid

A procedure similar to that described in Example 10 was repeated, except that 445 mg of methyl 1-[4-(2-carboxyphenyl)phenyl]methyl-4-(1-hydroxy-1-methylethyl)-2-methoxymethylimidazole-5-carboxylate hydrochloride [prepared as described in Example 15(b)] were employed, to obtain 250 mg of the title compound as crystals, melting at 164 - 165°C.

Nuclear Magnetic Resonance Spectrum (hexadeuterated dimethyl sulphoxide), δ ppm:
1.55 (6H, singlet);
3.25 (3H, singlet);
4.47 (2H, singlet);
5.67 (2H, singlet);
7.06 (2H, doublet, J = 8 Hz);
7.28 (2H, doublet, J = 8 Hz);
7.36 (1H, doublet, J = 7.5 Hz);
7.40 - 7.58 (2H, multiplet);
7.70 (1H, doublet, J = 8.5 Hz).

### EXAMPLE 17

### Ethyl 1-[4-(2-carboxyphenyl)phenyl]methyl-2-ethoxy-methyl-4-(1-hydroxy-1-methylethyl)imidazole-5-carboxylate

### 17(a) Ethyl 1-{4-[2-(t-butoxycarbonyl)phenyl]-phenyl}methyl-2-ethoxymethyl-4-(1-hydroxy-1-methyl-ethyl)imidazole-5-carboxylate

A procedure similar to that described in Example 1(a) was repeated, except that 315 mg of ethyl 4-(1-hydroxy-1-methylethyl)-2-ethoxymethylimidazole-5-carboxylate [prepared as described in Preparation 3(iii)], 145 mg of potassium t-butoxide and 510 mg of 4-[2-(t-butoxycarbonyl)phenyl]benzyl bromide were employed and the product was purified by column chromatography through silica gel using a 1 : 1 by volume mixture of hexane and ethyl acetate as the eluent, to obtain 600 mg of the title compound as a syrup.

Nuclear Magnetic Resonance Spectrum (CDCℓ₃), δ ppm:
1.18 (3H, triplet, J = 7 Hz);
1.26 (9H, singlet);
1.26 (3H, triplet, J = 7 Hz);
1.64 (6H, singlet);
3.54 (2H, quartet, J = 7 Hz);
4.27 (2H, quartet, J = 7 Hz);
4.57 (2H, singlet);
5.65 (1H, singlet);
5.67 (2H, singlet);
6.99 (2H, doublet, J = 8 Hz);
7.25 - 7.29 (3H, multiplet);
7.38 - 7.47 (2H, multiplet);
7.76 (1H, doublet, J = 7.5 Hz).

### 17(b) Ethyl 1-[4-(2-carboxyphenyl)phenyl]methyl-2-ethoxymethyl-4-(1-hydroxy-1-methylethyl)imidazole-5-carboxylate

Following a procedure similar to that described in Example 15(b), but using 600 mg of ethyl 1-{4-[2-(t-butoxycarbonyl)phenyl]phenyl}methyl-2-ethoxymethyl-4-(1-hydroxy-1-methylethyl)imidazole-5-carboxylate [prepared as described in step (a) above], 585 mg of the hydrochloride of the title compound were obtained as a foam-like solid.

Nuclear Magnetic Resonance Spectrum (CD₃OD), δ ppm:
1.15 (3H, triplet, J = 7 Hz);
1.23 (3H, triplet, J = 7 Hz);
1.69 (6H, singlet);
3.61 (2H, quartet, J = 7 Hz);
4.30 (2H, quartet, J = 7 Hz);
5.78 (2H, singlet);
5.80 (2H, singlet);
7.18 (2H, doublet, J = 8 Hz);
7.29 - 7.58 (5H, multiplet);
7.82 (1H, doublet, J = 8 Hz).

### EXAMPLE 18

### 1-[4-(2-Carboxyphenyl)phenyl]methyl-2-ethoxymethyl-4- (1-hydroxy-1-methylethyl)imidazole-5 -carboxylic acid

A procedure similar to that described in Example 10 was repeated, except that 585 mg of ethyl 1-[4-(2-carboxyphenyl)phenyl]methyl-2-ethoxymethyl-4-(1-hydroxy-1-methylethyl)imidazole-5-carboxylate hydrochloride [prepared as described in Example 17(b)] were employed, to obtain 465 mg of the title compound as a crystalline powder, melting at 166 - 169°C.

Nuclear Magnetic Resonance Spectrum (hexadeuterated dimethyl sulphoxide), δ ppm:
1.01 (3H, triplet, J = 7 Hz);
1.55 (6H, singlet);
3.44 (2H, quartet, J = 7 Hz);
4.50 (2H, singlet);
5.68 (2H, singlet);
7.06 (2H, doublet, J = 8 Hz);
7.28 (2H, doublet, J = 8 Hz);
7.35 (1H, doublet, J = 7 Hz);
7.41 - 7.58 (2H, multiplet);
7.70 (1H, doublet, J = 8.5 Hz).

### EXAMPLE 19

### Propyl 1-[4-(2-carboxyphenyl)phenyl]methyl-4-(1-hydroxy-1-methylethyl)-2-propoxymethylimidazole-5-carboxylate

### 19(a) Propyl 1-{4-[2-(t-butoxycarbonyl)phenyl]-phenyl}methyl-4-(1-hydroxy-1-methylethyl)-2-propoxy-methylimidazole-5-carboxylate

Following a procedure similar to that described in Example 1(a), but using 0.20 g of propyl 4-(1-hydroxy-1-methylethyl)-2-propoxymethylimidazole-5-carboxylate [prepared as described in Preparation 4(iii)], 82 mg of potassium t-butoxide and 290 mg of 4-[2-(t-butoxy-carbonyl)phenyl]benzyl bromide and then purifying the product by column chromatography through silica gel using a 1 : 1 by volume mixture of hexane and ethyl acetate as the eluent, 293 mg of the title compound were obtained as a syrup.

Nuclear Magnetic Resonance Spectrum (CDCℓ₃), δ ppm:
0.89 (6H, triplet, J = 7.5 Hz);
1.26 (9H, singlet);
1.53 - 2.59 (4H, multiplet);
1.64 (6H, singlet);
3.44 (2H, triplet, J = 7.5 Hz);
4.17 (2H, triplet, J = 7.5 Hz);
4.56 (2H, singlet);
5.67 (1H, singlet);
5.69 (2H, singlet);
6.98 (2H, doublet, J = 8.5 Hz);
7.27 (3H, doublet, J = 8.5 Hz);
7.38 - 7.47 (2H, multiplet);
7.76 (1H, doublet, J = 6.5 Hz).

### 19(b) Propyl 1-[4-(2-carboxyphenyl)phenyl]methyl-4-(1-hydroxy-1-methylethyl)-2-propoxymethylimidazole-5-carboxylate

A procedure similar to that described in Example 15(b) was repeated, except that 293 mg of propyl 1-[4-(2-carboxyphenyl)phenyl]methyl-4-(1-hydroxy-1-methylethyl)-2-propoxymethylimidazole-5-carboxylate [prepared as described in step (a) above] were employed, to obtain 281 mg of the hydrochloride of the title compound as a foam-like solid.

Nuclear Magnetic Resonance Spectrum (CDCℓ₃), δ ppm:
0.85 (3H, triplet, J = 7.5 Hz);
0.88 (3H, triplet, J = 7.5 Hz);
1.53 - 1.65 (4H, multiplet);
1.75 (6H, singlet);
3.54 (2H, doublet, J = 6.5 Hz);
4.19 (2H, triplet, J = 6.5 Hz);
4.98 (2H, singlet);
5.70 (2H, singlet);
7.01 (2H, doublet, J = 8 Hz);
7.24 - 7.39 (3H, multiplet);
7.41 - 7.56 (2H, multiplet);
7.92 (1H, doublet, J = 7.5 Hz).

### EXAMPLE 20

### 1-[4- (2-Carboxyphenyl)phenyl]methyl-4-(1-hydroxy-1-methylethyl)-2-propoxymethylimidazole-5-carboxylic acid

A procedure similar to that described in Example 10 was repeated, except that 281 mg of propyl 1-[4-(2-carboxyphenyl)phenyl]methyl-4-(1-hydroxy-1-methylethyl)-2-propoxymethylimidazole-5-carboxylate hydrochloride [prepared as described in Example 19(b)] were employed, to obtain 212 mg of the title compound as a crystalline powder, melting at 109 - 111°C.

Nuclear Magnetic Resonance Spectrum (hexadeuterated dimethyl sulphoxide), δ ppm:
0.78 (3H, triplet, J = 7.5 Hz);
1.41 (2H, sextet, J = 7.5 Hz);
1.56 (6H, singlet);
3.36 (2H, triplet, J = 7.5 Hz);
4.51 (2H, singlet);
5.69 (2H, singlet);
7.06 (2H, doublet, J = 8 Hz);
7.28 (2H, doublet, J = 8 Hz);
7.34 (1H, doublet, J = 7.5 Hz);
7.41 - 7.58 (2H, multiplet);
7.70 (1H, doublet, J = 6.5 Hz).

### EXAMPLE 21

### Ethyl 4-(1-hydroxy-1-methylethyl)-2 -methylthiomethyl-1-{4-[2-(tetrazol-5-yl)phenyl]phenyl}methyl-imidazole-5-carboxylate

### 21(a) Ethyl 2-acetoxymethyl-4-(1-hydroxy-1-methyl-ethyl]-1-{4-[2-(trityltetrazol-5-yl)phenyl]phenyl}-methylimidazole-5-carboxylate

Following a procedure similar to that described in Example 1(a), but using 730 mg of ethyl 2-acetoxymethyl-4-(1-hydroxy-1-methylethyl)imidazole-5-carboxylate [prepared as described in Preparation 7(iii)], 320 mg of potassium t-butoxide and 2.11 g of 4-[2-(trityltetrazol-5-yl)phenyl]benzyl bromide and then purifying the product by column chromatography through silica gel using a 2 : 1 by volume mixture of hexane and ethyl acetate, 1.23 g of the title compound were obtained as a foam-like solid.

Nuclear Magnetic Resonance Spectrum (CDCℓ₃), δ ppm:
1.08 (3H, triplet, J = 7 Hz);
1.66 (6H, singlet);
1.84 (3H, singlet);
4.15 (2H, quartet, J = 7 Hz);
5.04 (2H, singlet).
5.49 (2H, singlet);
5.58 (1H, singlet);
6.76 (2H, doublet, J = 8.5 Hz);
6.98 (6H, doublet, J = 7.5 Hz);
7.11 (2H, doublet, J = 8.5 Hz);
7.23 - 7.37 (lOH, multiplet);
7.41 - 7.53 (2H, multiplet);
7.84 (1H, doublet, J = 8 Hz).

### 21(b) Ethyl 2-hydroxymethyl-4-(1-hydroxy-1-methyl-ethyl)-1-{4-[2-(trityltetrazol-5-yl)phenyl]phenyl}-methylimidazole-5-carboxylate

0.75 ml of a 0.15 N solution of sodium ethoxide in ethanol was added to a solution of 1.69 g of ethyl 2-acetoxymethyl-4-(1-hydroxy-1-methylethyl)-1-{4-[2-(trityltetrazol-5-yl)phenyl]phenyl}methylimidazole-5-carboxylate [prepared as described in Example 21(a)] in 15 ml of ethanol, and the mixture was stirred at room temperature for 5 minutes. The reaction solution was then concentrated by evaporation under reduced pressure, ethyl acetate and water were added to the residue, and the ethyl acetate layer was separated. This ethyl acetate layer was washed with an aqueous solution of sodium chloride and then dried over anhydrous sodium sulphate, after which it was concentrated by evaporation under reduced pressure. The resulting residue was purified by recrystallisation from a mixture of diethyl ether and diisopropyl ether, to give 1.47 g of the title compound as crystals, melting at 151 - 152°C.

Nuclear Magnetic Resonance Spectrum (CDCℓ₃), δ ppm:
1.09 (3H, triplet, J = 7 Hz);
1.62 (6H, singlet);
4.17 (2H, quartet, J = 7 Hz);
4.48 (2H, singlet);
5.46 (2H, singlet);
5.66 (1H, singlet);
6.74 (2H, doublet, J = 8.5 Hz);
6.94 (6H, doublet, J = 8 Hz);
7.10 (2H, doublet, J = 8.5 Hz);
7.22 - 7.53 (12H, multiplet);
7.91 (1H, doublet, J = 9 Hz).

### 21(c) Ethyl 4-(1-hydroxy-1-methylethyl)-2 -methane-sulfonyloxymethyl-1-{4-[2- (trityltetrazol-5-yl)phenyl]-phenyl}methylimidazole-5-carboxylate

0.371 ml of N,N-diisopropyl-N-ethylamine and then 0.371 g of methanesulphonic anhydride were added, under a nitrogen atmosphere, to a solution of 500 mg of ethyl 2-hydroxymethyl-4-(1-hydroxy-1-methylethyl)-1-{4-[2-(trityltetrazol-5-yl)phenyl]phenyl}methylimidazole-5-carboxylate [prepared as described in step (b) above] in 10 ml of tetrahydrofuran. The mixture was then stirred at room temperature for 1.5 hours, after which it was mixed with ethyl acetate and an aqueous solution of sodium hydrogencarbonate. The ethyl acetate layer was separated, dried over anhydrous magnesium sulphate and concentrated by evaporation under reduced pressure, to give 610 mg of the title compound as an amorphous powder. The compound was employed in the subsequent reactions without any further purification.

Nuclear Magnetic Resonance Spectrum (CDCℓ₃), δ ppm:
1.11 (3H, triplet, J = 7 Hz);
1.65 (6H, singlet);
2.83 (3H, singlet);
4.20 (2H, quartet, J = 7 Hz);
5.09 (2H, singlet);
5.47 (1H, broad singlet);
5.53 (2H, singlet);
6.77 (2H, doublet, J = 8 Hz);
6.97 (6H, doublet, J = 7 Hz);
7.12 (2H, doublet, J = 8 Hz);
7.24 - 7.52 (12H, multiplet);
7.87 (1H, doublet, J = 7 Hz).

### 21(d) Ethyl 4-(1-hydroxy-1-methylethyl)-2-methylthiomethyl-1-{4-[2-(trityltetrazol-5-yl)phenyl]phenyl}-methylimidazole-5-carboxylate

50.3 mg of sodium methanethiolate were added to a solution of 610 mg of ethyl 4-(1-hydroxy-1-methylethyl)-2-methanesulphonyloxymethyl-1-{4-[2-(trityltetrazol-5-yl)phenyl]phenyl}methylimidazole-5-carboxylate [prepared as described in step (c) above] in 6 ml of N,N-dimethylformamide. The mixture was stirred at room temperature for 45 minutes, after which it was mixed with ethyl acetate and water. The ethyl acetate layer was separated, dried over anhydrous magnesium sulphate and then concentrated by evaporation under reduced pressure, after which the residue was purified by column chromatography through silica gel, using a 10 : 1 by volume mixture of methylene chloride and ethyl acetate as the eluent, to give 338 mg of the title compound as crystals, melting at 174.5 - 176.5°C (with decomposition).

Nuclear Magnetic Resonance Spectrum (CDCℓ₃), δ ppm:
1.10 (3H, triplet, J = 7 Hz);
1.65 (6H, singlet);
2.06 (3H, singlet);
3.46 (2H, singlet);
4.17 (2H, quartet, J = 7 Hz);
5.49 (2H, singlet);
5.72 (1H, singlet);
6.73 (2H, doublet, J = 8 Hz);
6.93 (6H, doublet, J = 7 Hz);
7.10 (2H, doublet, J = 8 Hz);
7.23 - 7.52 (12H, multiplet);
7.92 (1H, doublet, J = 7).

### 21(e) Ethyl 4-(1-hydroxy-1-methylethyl)-2-methylthiomethyl-1-{4-[2-(tetrazol-5-yl)phenyl]phenyl}methylimidazole-5-carboxylate

A mixture of 300 mg of ethyl 4-(1-hydroxy-l-methylethyl)-2-methylthiomethyl-1-{4-[2-(trityltetrazol-5-yl)phenyl]phenyl}methylimidazole-5-carboxylate [prepared as described in step (d) above] and 5 ml of a 25% v/v aqueous solution of acetic acid was stirred at 60°C for 1 hour. At the end of this time, the resulting solution was mixed with 5 ml of water and cooled with ice. The trityl alcohol which appeared as crystals was filtered off, and the filtrate was concentrated by evaporation under reduced pressure. Acetic acid and water in the residue were removed by distillation as azeotropic mixtures with toluene, to give 217 mg of the title compound as an amorphous powder.

Nuclear Magnetic Resonance Spectrum (CDCℓ₃), δ ppm:
1.18 (3H, triplet, J = 7.5 Hz);
1.55 (6H, singlet);
2.09 (3H, singlet);
3.63 (2H, singlet);
4.24 (2H, quartet, J = 7.5 Hz);
5.58 (2H, singlet);
6.89 (2H, doublet, J = 8 Hz);
7.12 (2H, doublet, J = 8 Hz);
7.41 - 7.62 (3H, multiplet);
7.95 (1H, doublet, J = 7 Hz).

### EXAMPLE 22

### 4-(1-Hydroxy-1-methylethyl)-2-methylthiomethyl-1-{4-[2-(tetrazol-5-yl)phenyl]phenyl}methyl-imidazole-5-carboxylic acid

A mixture of 217 mg of ethyl 4-(1-hydroxy-1-methyl-ethyl)-2-methylthiomethyl-1-{4-[2-(tetrazol-5-yl)-phenyl]phenyl}methylimidazole-5-carboxylate [prepared as described in Example 21(e)] and 3.2 ml of a 0.5 N aqueous solution of sodium hydroxide was stirred at room temperature for 1 hour. At the end of this time, the insoluble matter was filtered off, and the filtrate was mixed with 1.6 ml of a 1 N aqueous solution of hydrochloric acid. The amorphous powder which had precipitated was collected by filtration, to give 155 mg of the title compound, melting at 172 - 181°C (with softening).

Nuclear Magnetic Resonance Spectrum (hexadeuterated dimethyl sulphoxide), δ ppm:
1.54 (6H, singlet);
2.05 (3H, singlet);
3.73 (2H, singlet);
5.66 (2H, singlet);
6.96 (2H, doublet, J = 8 Hz);
7.06 (2H, doublet, J = 8 Hz);
7.51 - 7.69 (4H, multiplet).

### EXAMPLE 23

### Pivaloyloxymethyl 1-[4-(2-carboxyphenyl)phenyl]-methyl-2-ethoxymethyl-4-(1-hydroxy-1-methylethyl)-imidazole-5-carboxylate

### 23(a) Pivaloyloxymethyl 1-{4-[2-(t-butoxycarbonyl)-phenyl]phenyl}methyl-2-ethoxymethyl-4-(1-hydroxy-1-methylethyl)imidazole-5-carboxylate

A procedure similar to that described in Example 4(a) was repeated, except that 374 mg of ethyl 1-{4-[2-(t-butoxycarbonyl)phenyl]phenyl}methyl-2-ethoxy-methyl-4-(1-hydroxy-1-methylethyl)imidazole-5-carboxylate [prepared as described in Example 17(a)] were employed, to obtain 396 mg of the title compound as a syrup.

Nuclear Magnetic Resonance Spectrum (CDCℓ₃), δ ppm:
1.18 (9H, singlet);
1.20 (3H, triplet, J = 7.5 Hz);
1.24 (9H, singlet);
1.63 (6H, singlet);
3.56 (2H, quartet, J = 7.5 Hz);
4.58 (2H, singlet);
5.24 (1H, singlet);
5.67 (2H, singlet);
5.84 (2H, singlet);
7.03 (2H, doublet, J = 8 Hz);
7.25 - 7.29 (3H, multiplet);
7.38 - 7.48 (2H, multiplet);
7.77 (1H, doublet, J = 6 Hz).

### 23(b) Pivaloyloxymethyl 1-[4-(2-carboxyphenyl)phenyl]-methyl-2-ethoxymethyl-4-(1-hydroxy-1-methylethyl)-imidazole-5-carboxylate

Following a procedure similar to that described in Example 15(b) but using 396 mg of pivaloyloxymethyl 1-{4-[2-(t-butoxycarbonyl)phenyl]phenyl}methyl-2-ethoxymethyl-4-(1-hydroxy-1-methylethyl)imidazole-5-carboxylate [prepared as described in step (a) above], 312 mg of the hydrochloride of the title compound were obtained as an amorphous powder, melting at 65°C (with softening).

Nuclear Magnetic Resonance Spectrum (hexadeuterated dimethyl sulphoxide), δ ppm:
1.02 (3H, triplet, J = 7 Hz);
1.09 (9H, singlet);
1.55 (6H, singlet);
3.48 (2H, quartet, J = 7 Hz);
4.71 (2H, singlet);
5.62 (2H, singlet);
5.85 (2H, singlet);
7.15 (2H, doublet, J = 8 Hz);
7.29 - 7.35 (3H, multiplet);
7.43 - 7.59 (2H, multiplet);
7.73 (1H, doublet, J = 6.5 Hz).

### EXAMPLE 24

### Ethyl 4-(1-hydroxy-1-methylethyl)-2-methylthio-1-{4-[2-(tetrazol-5-yl)phenyl]phenyl}methyl-imidazole-5-carboxylate

### 24(a) Ethyl 4-(1-hydroxy-1-methylethyl)-2-methylthio-1-{4-[2-(trityltetrazol-5-yl)phenyl]phenyl}methylimidazole-5-carboxylate

242 mg of potassium t-butoxide were added, whilst ice-cooling, to a solution of 500 mg of ethyl 4-(1-hydroxy-1-methylethyl)-2-methylthioimidazole-5-carboxylate [prepared as described in Preparation 8(ii)] in 10 ml of N,N-dimethylacetamide and stirred for 30 minutes. 1.26 g of 4-[2-(trityltetrazol-5-yl)phenyl]-benzyl bromide were then added in portions to the resulting solution, and the mixture was stirred at room temperature for 4 hours. At the end of this time, the reaction mixture was mixed with ethyl acetate and water and shaken. The ethyl acetate layer was separated, washed with water and then with a saturated aqueous solution of sodium chloride and dried over anhydrous magnesium sulphate. The solvent was removed by distillation under reduced pressure, and the resulting residue was purified by column chromatography through silica gel, using a 1 : 5 by volume mixture of ethyl acetate and hexane as the eluent, to give 940 mg of the title compound as colorless crystals, melting at 125 - 127°C

Nuclear Magnetic Resonance Spectrum (CDCℓ₃), δ ppm:
1.11 (3H, triplet, J = 7.5 Hz);
1.63 (6H, singlet);
2.61 (3H, singlet);
4.16 (2H, quartet, J = 7.5 Hz);
5.34 (2H, singlet);
5.75 (1H, singlet).
6.80 - 7.90 (23H, multiplet).

### 24(b) Ethyl 4-(1-hydroxy-1-methylethyl)-2-methylthio-1-{4-[2-(tetrazol-5-yl)phenyl]phenyl}methylimidazole-5-carboxylate

900 mg of ethyl 4-(1-hydroxy-1-methylethyl)-2-methylthio-1-{4- [2- (trityltetrazol-5-yl)phenyl]-phenyl}methylimidazole-5-carboxylate [prepared as described in step (a) above] were added to 10 ml of a 25% v/v aqueous solution of acetic acid, and the mixture was stirred at 60°C for 1 hour. At the end of this time, the reaction mixture was cooled, and the crystals of trityl alcohol which appeared were filtered off. These crystals were washed with a 50% v/v aqueous solution of acetic acid, and the filtrate and the washings were mixed. The resulting mixture was concentrated by evaporation under reduced pressure, and the resulting residue was crystallized from ethyl acetate, to give 529 mg of the title compound as crystals, melting at 209 - 210°C.

Nuclear Magnetic Resonance Spectrum (hexadeuterated dimethyl sulphoxide), δ ppm:
1.07 (3H, triplet, J = 7.5 Hz);
1.49 (6H, singlet);
2.62 (3H, singlet);
4.16 (2H, quartet, J = 7.5 Hz);
5.37 (2H, singlet);
5.41 (1H, singlet);
6.95 (2H, doublet, J = 8 Hz);
7.08 (2H, doublet, J = 8 Hz);
7.50 - 7.72 (4H, multiplet).

### EXAMPLE 25

### 4-(1-Hydroxy-1-methylethyl)-2-methylthio-1-{4-[2-(tetrazol-5-yl)phenyl]phenyl}methyl-imidazole-5-carboxylic acid

500 mg of ethyl 4-(1-hydroxy-1-methylethyl)-2-methylthio-1-{4-[2-(tetrazol-5-yl)phenyl]phenyl}-methylimidazole-5-carboxylate [prepared as described in Example 24(b)] and 131 mg of lithium hydroxide monohydrate were added to a mixture of 5 ml of water and 5 ml of dioxane, and the resulting mixture was stirred at room temperature for 24 hours. At the end of this time, the reaction mixture was concentrated by evaporation under reduced pressure, and the resulting residue was dissolved in water. 3.1 ml of 1 N aqueous hydrochloric acid were then added, and the crystals which appeared were collected by filtration. These crystals were dissolved in ethyl acetate, and water was added to induce crystallisation. The crystals which appeared were collected by filtration and washed with ethyl acetate and water, to give 290 mg of the title compound as crystals, melting at 169 - 171°C (with decomposition).

Nuclear Magnetic Resonance Spectrum (hexadeuterated dimethyl sulphoxide), δ ppm:
1.55 (6H, singlet);
2.59 (3H, singlet);
5.51 (2H, singlet);
7.01 (2H, doublet, J = 8 Hz);
7.07 (2H, doublet, J = 8 Hz);
7.47 - 7.75 (4H, multiplet).

### EXAMPLE 26

### Ethyl 2-ethylthio-4-(1-hydroxy-1-methylethyl)-1-{4-[2-(tetrazol-5-yl)phenyl]phenyl}methyl-imidazole-5-carboxylate

### 26(a) Ethyl 2-ethylthio-4-(1-hydroxy-1-methylethyl)-1-{4-[2-(trityltetrazol-5-yl)phenyl]phenyl}methyl-imidazole-5-carboxylate

478 mg of potassium t-butoxide were added to a solution of 1.00 g of ethyl 2-ethylthio-4-(1-hydroxy-1-methylethyl)imidazole-5-carboxylate [prepared as described in Preparation 9(ii)] in 20 ml of N,N-dimethylacetamide, whilst ice-cooling, and the mixture was stirred for 30 minutes. At the end of this time, 2.59 g of 4-[2-(trityltetrazol-5-yl)phenyl]benzyl bromide were added in portions to the mixture. Following a procedure similar to that described in Example 24(a) and purifying the residue by column chromatography through silica gel using a 1 : 5 by volume mixture of ethyl acetate and hexane as the eluent, 2.22 g of the title compound were obtained as an amorphous powder.

Nuclear Magnetic Resonance Spectrum (CDCℓ₃), δ ppm:
1.10 (3H, triplet, J = 7.5 Hz);
1.34 (3H, triplet, J = 7.5 Hz);
1.63 (6H, singlet);
3.19 (2H, quartet, J = 7.5 Hz);
4.17 (2H, quartet, J = 7.5 Hz);
5.35 (2H, singlet);
5.78 (1H, singlet);
6.78 - 7.88 (23H, multiplet).

### 26(b) Ethyl 2-ethylthio-4-(1-hydroxy-1-methylethyl)-1-{4-[2-(tetrazol-5-yl)phenyl]phenyl}methylimidazole-5-carboxylate

2.22 g of ethyl 2-ethylthio-4-(1-hydroxy-1-methyl-ethyl)-1-{4-[2-(trityltetrazol-5-yl)phenyl]phenyl}-methylimidazole-5-carboxylate [prepared as described in step (a) above] were added to 20 ml of a 25% v/v aqueous solution of acetic acid, and the mixture was stirred at 60°C for 2 hours. The reaction mixture was then concentrated by evaporation under reduced pressure, and the resulting residue was crystallized by the addition of ethyl acetate, to give 1.22 g of the title compound as crystals, melting at 185 - 188°C.

Nuclear Magnetic Resonance Spectrum (hexadeuterated dimethyl sulphoxide), δ ppm:
1.06 (3H, triplet, J = 7.5 Hz);
1.30 (3H, triplet, J = 7.5 Hz);
1.49 (6H, singlet);
3.17 (2H, quartet, J = 7.5 Hz);
4.16 (2H, quartet, J = 7.5 Hz);
5.38 (2H, singlet);
6.95 (2H, doublet, J = 8.5 Hz);
7.08 (2H, doublet, J = 8.5 Hz);
7.50 - 7.74 (4H, multiplet).

### EXAMPLE 27

### 2-Ethylthio-4-(1-hydroxy-1-methylethyl)-1-{4-[2-(tetrazol-5-yl)phenyl]phenyl}methyl-imidazole-5-carboxylic acid

1.00 g of ethyl 2-ethylthio-4-(1-hydroxy-1-methyl-ethyl)-1-{4-[2-(tetrazol-5-yl)phenyl]phenyl}methyl-imidazole-5-carboxylate [prepared as described in Example 26(b)] and 256 mg of lithium hydroxide monohydrate were added to a mixture of 10 ml of water and 10 ml of dioxane. The mixture was then stirred at room temperature for 24 hours after which it was concentrated by evaporation under reduced pressure. The resulting residue was dissolved in water, and then 6.1 ml of 1 N aqueous hydrochloric acid were added. The oily matter which appeared was extracted with ethyl acetate. The extract was washed with a saturated aqueous solution of sodium chloride, dried over anhydrous magnesium sulphate and concentrated by evaporation under reduced pressure, to give 955 mg of the title compound as an amorphous powder.

Nuclear Magnetic Resonance Spectrum (CDCℓ₃), δ ppm:
1.29 (3H, triplet, J = 7.5 Hz);
1.60 (6H, singlet);
3.11 (2H, quartet, J = 7.5 Hz);
5.55 (2H, singlet);
6.92 (2H, doublet, J = 8.5 Hz);
6.98 (2H, doublet, J = 8.5 Hz);
7.36 - 7.60 (3H, multiplet);
7.81 (1H, doublet, J = 7.5 Hz).

### EXAMPLE 28

### Ethyl 2-hydroxymethyl-4-(1-hydroxy-1-methylethyl)-1-{4-[2-(tetrazol-5-yl)phenyl]phenyl}methyl-imidazole-5-carboxylate

A procedure similar to that described in Example 1(b) was repeated, except that 400 mg of ethyl 2-hydroxymethyl-4-(1-hydroxy-1-methylethyl)-1-{4-[2-(trityltetrazol-5-yl)phenyl]phenyl}methylimidazole-5-carboxylate [prepared as described in Example 21(b)] were used as a starting material, to obtain 264 mg of the title compound as crystals, melting at 98 - 99°C.

Nuclear Magnetic Resonance Spectrum (CDCℓ₃), δ ppm:
1.14 (3H, triplet, J = 7.5 Hz);
1.48 (6H, singlet);
4.20 (2H, quartet, J = 7.5 Hz);
4.55 (2H, singlet);
5.57 (2H, singlet);
6.77 (2H, doublet, J = 8 Hz);
6.99 (2H, doublet, J = 8 Hz);
7.28 - 7.59 (3H, multiplet);
7.83 (1H, doublet, J = 7.5 Hz).

### EXAMPLE 29

### 2-Hydroxymethyl-4-(1-hydroxy-1-methylethyl)-1-{4-[2-(tetrazol-5-yl)phenyl]phenyl}methyl-imidazole-5-carboxylic acid

Following a procedure similar to that described in Example 10, but using 200 mg of ethyl 2-hydroxymethyl-4-(1-hydroxy-1-methylethyl)-1-{4-[2-(tetrazol-5-yl)-phenyl]phenyl}methylimidazole-5-carboxylate (prepared as described in Example 28)], 169 mg of the title compound were obtained as crystals, melting at 201 - 202°C.

Nuclear Magnetic Resonance Spectrum (hexadeuterated dimethyl sulphoxide), δ ppm:
1.54 (6H, singlet);
4.46 (2H, singlet);
5.69 (2H, singlet) ;
6.98 (2H, doublet, J = 9 Hz);
7.05 (2H, doublet, J = 9 Hz);
7.52 - 7.70 (4H, multiplet).

### PREPARATION 1

### Ethyl 4-(1-hydroxy-1-methylethyl)-2-methoxymethyl-imidazole-5-carboxylate

### 1(i) Diethyl 1-benzyl-2-methylimidazole-4,5-dicarboxylate

5.21 g of potassium t-butoxide were added to a solution of 10.0 g of diethyl 2-methylimidazole-4,5-dicarboxylate in 100 ml of N,N-dimethylacetamide, whilst ice-cooling and under a nitrogen atmosphere. The mixture was stirred for 30 minutes, until a homogeneous solution was obtained, and then 5.78 ml of benzyl bromide were added dropwise to this solution, whilst ice-cooling. The resulting mixture was stirred at room temperature for 1 hour, after which it was mixed with ethyl acetate and water and shaken. The ethyl acetate layer was separated, washed with an aqueous solution of sodium chloride and then dried over anhydrous magnesium sulphate. The solvent was removed by distillation under reduced pressure, and the resulting residue was purified by column chromatography through silica gel, using a 3 : 1 by volume mixture of hexane and ethyl acetate as the eluent, to give 12.38 g of the title compound as a syrup.

Nuclear Magnetic Resonance Spectrum (CDCℓ₃), δ ppm:
1.26 (3H, triplet, J = 7.5 Hz);
1.39 (3H, triplet, J = 7.5 Hz);
2.39 (3H, singlet);
4.28 (2H, quartet, J = 7.5 Hz);
4.39 (3H, quartet, J = 7.5 Hz);
5.39 (2H, singlet);
7.01 (2H, doublet, J = 6 Hz);
7.24 - 7.34 (3H, multiplet).

### 1 (ii) Diethyl 1-benzyl-2-bromomethylimidazole-4,5-dicarboxylate

2.52 g of N-bromosuccinimide and 0.42 of benzoyl peroxide were added to a solution of 4.07 g of diethyl 1-benzyl-2-methylimidazole-4,5-dicarboxylate [prepared as described in step (i) above] in 80 ml of carbon tetrachloride, and the mixture was irradiated by a 375 W tungsten lamp for 50 minutes, whilst stirring. At the end of this time, the reaction solution was washed with a 5% w/v aqueous solution of sodium thiosulphate and with a saturated aqueous solution of sodium hydrogencarbonate, in that order, after which it was concentrated by evaporation under reduced pressure. The resulting residue was purified by column chromatography through silica gel, using a 3 : 2 by volume mixture of hexane and ethyl acetate as the eluent, to give 3.81 g of the title compound as a syrup.

Nuclear Magnetic Resonance Spectrum (CDCℓ₃), δ ppm:
1.25 (3H, triplet, J = 7.5 Hz);
1.39 (3H, triplet, J = 7.5 Hz);
4.28 (2H, quartet, J = 7.5 Hz);
4.39 (2H, singlet);
4.40 (2H, quartet, J = 7.5 Hz);
5.52 (2H, singlet);
7.10 (2H, doublet, J = 5.5 Hz);
7.27 - 7.39 (3H, multiplet).

### 1(iii) Dimethyl 1-benzyl-2-methoxymethylimidazole-4,5-dicarboxylate

492 mg of a 28% w/v solution of sodium methoxide in methanol were added to a solution of 655 mg of diethyl 1-benzyl-2-bromomethylimidazole-4,5-dicarboxylate [prepared as described in step (ii) above] in 7 ml of methanol, and the mixture was allowed to stand at room temperature for 13 hours. At the end of this time, 2.5 ml of 1 N aqueous hydrochloric acid were added to the reaction solution, and the methanol was removed by distillation under reduced pressure. The concentrate was mixed with ethyl acetate and water and then shaken. The ethyl acetate layer was separated, washed with a saturated aqueous solution of sodium hydrogencarbonate and with a saturated aqueous solution of sodium chloride, in that order, and dried over anhydrous magnesium sulphate. The solvent was removed by distillation under reduced pressure, and then the residue was purified by column chromatography through silica gel, using a 5 : 1 by volume mixture of methylene chloride and ethyl acetate as the eluent, to give 391 mg of the title compound as a syrup.

Nuclear Magnetic Resonance Spectrum (CDCℓ₃), δ ppm:
3.34 (3H, singlet);
3.81 (3H, singlet);
3.92 (3H, singlet);
4.51 (2H, singlet);
5.52 (2H, singlet);
7.05 (2H, doublet, J = 8 Hz);
7.25 - 7.34 (3H, multiplet).

### 1(iv) Dimethyl 2 -methoxymethylimidazole-4,5-dicarboxylate

650 mg of 10% w/w palladium-on-carbon and 6.1 ml of a 4 N solution of hydrogen chloride in dioxane were added to a solution of 6.5 g of dimethyl 1-benzyl-2-methoxymethylimidazole-4,5-dicarboxylate [prepared as described in step (iii) above] in 65 ml of methanol. The mixture was then stirred at room temperature for 1.5 hours under a hydrogen atmosphere. At the end of this time, the catalyst was filtered off and the filtrate was concentrated by evaporation under reduced pressure, to give a crystalline compound. This crystalline compound was washed with ethyl acetate, to give 5.13 g of the hydrochloride of the title compound, melting at 108 - 111°C.

Nuclear Magnetic Resonance Spectrum (hexadeuterated dimethyl sulphoxide), δ ppm:
3.29 (3H, singlet);
3.82 (6H, singlet);
4.43 (2H, singlet);
7.28 (2H, broad singlet).

### 1(v) Methyl 4-(1-hydroxy-1-methylethyl)-2-methoxy-methylimidazole-5-carboxylate

8.87 ml of a 0.98M solution of methylmagnesium iodide in diethyl ether were added dropwise at 4 - 6°C to a solution of 575 mg of dimethyl 2-methoxymethyl-imidazole-4,5-carboxylate hydrochloride [prepared as described in step (iv) above] in 40 ml of methylene chloride, under a nitrogen atmosphere. The mixture was then stirred at room temperature for 1 hour, after which it was mixed with ethyl acetate and then with an aqueous solution of ammonium chloride, whilst ice-cooling. Sodium chloride was added to the aqueous layer until it was saturated, and then the mixture was further shaken. The ethyl acetate layer was separated and dried over anhydrous magnesium sulphate. The solvent was removed by distillation under reduced pressure, and the resulting residue was purified by column chromatography through silica gel, using a 1 : 20 by volume mixture of methanol and methylene chloride as the eluent, to give 391 mg of the title compound as crystals, melting at 94.5 - 96.0°C.

Nuclear Magnetic Resonance Spectrum (CDCℓ₃), δ ppm:
1.63 (6H, singlet);
3.46 (3H, singlet);
3.92 (3H, singlet);
4.55 (2H, singlet).

### PREPARATION 2

### Dimethyl 2-methoxymethylimidazole-4,5-dicarboxylate

### 2(i) Diethyl 2-methyl-1-(4-nitrobenzyl)imidazole-4,5-dicarboxylate

Following a procedure similar to that described in Preparation 1(i), but using 6.65 g of diethyl 2-methylimidazole-4,5-dicarboxylate and 6.35 g of p-nitrobenzyl bromide as starting materials, 8.57 g of the title compound were obtained as crystals, melting at 109°C.

Nuclear Magnetic Resonance Spectrum (CDCℓ₃), δ ppm:
1.28 (3H, triplet, J = 7.5 Hz);
1.41 (3H, triplet, J = 7.5 Hz);
2.40 (3H, singlet);
4.28 (2H, quartet, J = 7.5 Hz);
4.41 (2H, quartet, J = 7.5 Hz);
5.53 (2H, singlet);
7.19 (2H, doublet, J = 9 Hz);
8.21 (2H, doublet, J = 9 Hz).

### 2(ii) Diethyl 2-bromomethyl-1-(4-nitrobenzyl)imidazole-4,5-dicarboxylate

Following a procedure similar to that described in Preparation 1(ii), but brominating 6.6 g of diethyl 2-methyl-1-(4-nitrobenzyl)imidazole-4,5-dicarboxylate [prepared as described in step (i) above] with 3.9 g of N-bromosuccinimide, 5.75 g of the title compound were obtained as a syrup.

Nuclear Magnetic Resonance Spectrum (CDCℓ₃), δ ppm:
1.26 (3H, triplet, J = 7.5 Hz);
1.41 (3H, triplet, J = 7.5 Hz);
4.27 (2H, quartet, J = 7.5 Hz);
4.42 (2H, quartet, J = 7.5 Hz);
5.66 (2H, singlet);
7.27 (2H, doublet, J = 8.5 Hz);
8.22 (2H, doublet, J = 8.5 Hz).

### 2(iii) Dimethyl 2-methoxymethyl-1-(4-nitrobenzyl)-imidazole-4,5-dicarboxylate

Following a procedure similar to that described in Preparation 1(iii), but using 2.63 g of diethyl 2-bromomethyl-1-(4-nitrobenzyl)imidazole-4,5-dicarboxylate [prepared as described in step (ii) above], 1.38 g of the title compound were obtained as crystals, melting at 107 - 110°C.

Nuclear Magnetic Resonance Spectrum (CDCℓ₃), δ ppm:
3.82 (3H, singlet);
3.94 (3H, singlet);
4.28 (3H, singlet);
4.54 (2H, singlet);
5.56 (2H, singlet);
7.23 (2H, doublet, J = 8.5 Hz);
8.19 (2H, doublet, J = 8.5 Hz).

### 2(iv) Dimethyl 2-methoxymethylimidazole-4,5-dicarboxylate

Following a procedure similar to that described in Preparation 1(iv), but catalytically reducing 1.25 g of dimethyl 2-methoxymethyl-1-(4-nitrobenzyl)imidazole-4,5-dicarboxylate [prepared as described in step (iii) above], a mixture of the hydrochlorides of the title compound and of p-toluidine was obtained. This was mixed with ethyl acetate and with a saturated aqueous solution of sodium hydrogencarbonate to neutralize it, and then the ethyl acetate layer was separated. This layer was dried over anhydrous magnesium sulphate, and the solvent was removed by distillation under reduced pressure. The resulting syrup was left in diisopropyl ether and the crystals which appeared were collected by filtration, to give 563 mg of the title compound, melting at 93 - 95°C.

Nuclear Magnetic Resonance Spectrum (CDCℓ₃), δ ppm:
3.43 (3H, singlet);
3.93 (6H, singlet);
4.59 (2H, singlet).

### PREPARATION 3

### Ethyl 2-ethoxymethyl-4-(1-hydroxy-1-methylethyl)-imidazole-5-carboxylate

### 3(i) Diethyl 1-benzyl-2-ethoxymethylimidazole-4,5-dicarboxylate

A solution of 1.80 g of diethyl 1-benzyl-2-bromo-methylimidazole-4,5-dicarboxylate [prepared as described in Preparation 1(ii)] in 50 ml of ethanol was added dropwise to a solution of sodium ethoxide in ethanol (prepared from 0.18 g of sodium and 50 ml of ethanol), and the resulting mixture was left at room temperature for 13 hours. At the end of this time, a procedure similar to that described in Preparation 1(iii) was repeated, and the residue was purified by column chromatography through silica gel, using a 1 : 1 by volume mixture of hexane and ethyl acetate as the eluent, to give 1.14 g of the title compound as a syrup.

Nuclear Magnetic Resonance Spectrum (CDCℓ₃), δ ppm:
1.13 (3H, triplet, J = 7 Hz);
1.22 (3H, triplet, J = 7 Hz);
1.38 (3H, triplet, J = 7 Hz);
3.50 (2H, quartet, J = 7 Hz);
4.25 (2H, quartet, J = 7 Hz);
4.38 (2H, quartet, J = 7 Hz);
4.56 (2H, singlet);
5.53 (2H, singlet) ;
7.06 (2H, doublet, J = 6 Hz);
7.26 - 7.39 (3H, multiplet).

### 3(ii) Diethyl 2-ethoxymethylimidazole-4,5-dicarboxylate

Following a procedure similar to that described in Preparation 1(iv), but using 4.37 g of diethyl 1-benzyl-2-ethoxymethylimidazole-4,5-dicarboxylate [prepared as described in step (i) above], 3.49 g of the hydrochloride of the title compound were obtained as crystals, melting at 60 - 61°C.

Nuclear Magnetic Resonance Spectrum (CDCℓ₃), δ ppm:
1.16 (3H, triplet, J = 7 Hz);
1.38 (6H, triplet, J = 7 Hz);
3.65 (2H, quartet, J = 7 Hz);
4.40 (4H, quartet, J = 7 Hz);
4.96 (2H, singlet).

A solution of the diethyl 2-ethoxymethylimidazole-4,5-dicarboxylate hydrochloride thus obtained in ethyl acetate was neutralized by the addition of a saturated aqueous solution of sodium hydrocarbonate. The ethyl acetate layer was separated, dried over anhydrous magnesium sulphate and concentrated by evaporation under reduced pressure, to give the title compound as crystals, melting at 71 - 74°C

Nuclear Magnetic Resonance Spectrum (CDCℓ₃), δ ppm:
1.26 (3H, triplet, J = 7 Hz);
1.39 (4H, triplet, J = 7 Hz);
3.63 (2H, quartet, J = 7 Hz);
4.41 (4H, quartet, J = 7 Hz);
4.64 (2H, singlet).

### 3(iii) Ethyl 2-methoxymethyl-4-(1-hydroxy-1-methylethyl)imidazole-5-carboxylate

A solution of 800 mg of diethyl 2-ethoxymethyl-imidazole-4,5-dicarboxylate hydrochloride [prepared as described in step (ii) above] in 20 ml of methylene chloride were added dropwise at 4 - 8°C to 8.6 ml of a solution of methylmagnesium iodide in diethyl ether (prepared from 285 mg of magnesium and 0.731 ml of methyl iodide), under a nitrogen atmosphere. The reaction solution was then stirred at room temperature for 1.5 hours, after which it was concentrated by evaporation under reduced pressure. The resulting residue was dissolved in ethyl acetate, and a saturated aqueous solution of ammonium chloride was added, whilst ice-cooling. The mixture was stirred for 30 minutes, and then the ethyl acetate layer was separated and dried over anhydrous magnesium sulphate. It was then concentrated by evaporation under reduced pressure, and the residue was purified by column chromatography through silica gel, using a 20 : 1 by volume mixture of methylene chloride and methanol as the eluent, to give 495 mg of the title compound as crystals, melting at 112 - 113°C.

Nuclear Magnetic Resonance Spectrum (hexadeuterated dimethyl sulphoxide), δ ppm:
1.12 (3H, triplet, J = 7 Hz);
1.29 (3H, triplet, J = 7 Hz);
1.52 (6H, singlet);
3.48 (2H, quartet, J = 7 Hz);
4.25 (2H, quartet, J = 7 Hz);
5.79 (1H, broad singlet).

### PREPARATION 4

### Propyl 4-(1-hydroxy-1-methylethyl)-2-propoxymethyl-imidazole-5-carboxylate

### 4(i) Dipropyl 1-benzyl-2-propoxymethylimidazole-4,5-dicarboxylate

A solution of 2.59 g of diethyl 1-benzyl-2-bromo-methylimidazole-4,5-dicarboxylate [prepared as described in Preparation 1(ii)] in 10 ml of propanol and 5 ml of tetrahydrofuran was added dropwise to a solution of sodium propoxide in propanol (prepared from 0.23 g of sodium and 20 ml of propanol), and the resulting mixture was left at room temperature for 3 hours. At the end of this time, following a procedure similar to that described in Preparation 1(iii), the residue was purified by column chromatography through silica gel, using a 3 : 1 by volume mixture of hexane and ethyl acetate as the eluent, to give 0.99 g of the title compound as a syrup.

Nuclear Magnetic Resonance Spectrum (CDCℓ₃), δ ppm:
0.87 (6H, triplet, J = 7 Hz);
0.98 (3H, triplet, J = 7 Hz);
1.53 (2H, quartet, J = 7 Hz);
1.60 (2H, quartet, J = 7 Hz);
1.77 (2H, quartet, J = 7 Hz);
3.40 (2H, triplet, J = 7 Hz);
4.14 (2H, triplet, J = 7 Hz);
4.28 (2H, triplet, J = 7 Hz);
4.56 (2H, singlet);
5.53 (2H, singlet);
7.06 (2H, doublet, J = 7 Hz);
7.23 - 7.39 (3H, multiplet).

### 4(ii) Dipropyl 2-propoxymethylimidazole-4,5-dicarboxylate

Following a procedure similar to that described in Preparation 1(iv), but using 0.99 g of dipropyl 1-benzyl-2-propoxymethylimidazole-4,5-dicarboxylate [prepared as described in step (i) above] as the starting material, 0.83 g of the hydrochloride of the title compound was obtained as a syrup.

Nuclear Magnetic Resonance Spectrum (CDCℓ₃), δ ppm:
0.85 (3H, triplet, J = 7 Hz);
0.98 (6H, triplet, J = 7 Hz);
1.57 (2H, sextet, J = 7 Hz);
1.79 (4H, sextet, J = 7 Hz);
3.59 (2H, triplet, J = 7 Hz);
4.30 (4H, triplet, J = 7 Hz);
5.11 (2H, singlet).

### 4 (iii) Propyl 4-(1-hydroxy-1-methylethyl)-2-propoxy-methylimidazole-5-carboxylate

Following a procedure similar to that described in Preparation 3(iii), but using 0.83 g of dipropyl 2-propoxymethylimidazole-4,5-dicarboxylate hydrochloride [prepared as described in step (ii) above] and then purifying the product by column chromatography through silica gel using a 1 : 20 by volume mixture of methanol and methylene chloride as the eluent, 0.63 g of the title compound was obtained as crystals, melting at 72 - 73°C.

Nuclear Magnetic Resonance Spectrum (CDCℓ₃), δ ppm:
0.94 (3H, triplet, J = 7 Hz);
0.99 (3H, triplet, J = 7 Hz);
1.54 - 1.68 (2H, multiplet);
1.62 (6H, singlet);
1.78 (2H, sextet, J = 7 Hz);
3.50 (2H, triplet, J = 7 Hz);
4.28 (2H, doublet, J = 7 Hz);
4.58 (2H, singlet);
5.74 (1H, singlet).

### PREPARATION 5

### Isopropyl 4-(1-hydroxy-1-methylethyl)-2-isopropoxy-methylimidazole-5-carboxylate

### 5(i) Diisopropyl 1-benzyl-2-isopropoxymethylimidazole-4,5-dicarboxylate

A solution of 5.19 g of diethyl 1-benzyl-2-bromo-methylimidazole-4,5-dicarboxylate [prepared as described in Preparation 1(ii)] in 20 ml of isopropanol and 25 ml of tetrahydrofuran was added dropwise to a solution of sodium isopropoxide in isopropanol (prepared from 0.77 g of sodium and 100 ml of isopropanol), and then, the resulting mixture was heated under reflux for 5 hours. The reaction solution was then treated following a procedure similar to that described in Preparation 1(iii). The residue was purified by column chromatography through silica gel, using a 3 : 2 by volume mixture of hexane and ethyl acetate as the eluent, to give 1.47 g of the title compound as a syrup.

Nuclear Magnetic Resonance Spectrum (CDCℓ₃), δ ppm:
1.13 (6H, doublet, J = 6 Hz);
1.19 (6H, doublet, J = 6.5 Hz);
1.38 (6H, doublet, J = 6.5 Hz);
3.65 (1H, septet, J = 6 Hz);
4.54 (2H, singlet);
5.08 (2H, septet, J = 6.5 Hz);
5.25 (2H, septet, J = 6.5 Hz);
5.52 (2H, singlet);
7.06 (2H, doublet, J = 6 Hz);
7.25 - 7.33 (3H, multiplet).

### 5(ii) Diisopropyl 2-isopropoxymethylimidazole-4,5-dicarboxylate

Following a procedure similar to that described in Preparation 1(iv), but using 1.47 g of diisopropyl 1-benzyl-2-isopropoxymethylimidazole-4,5-dicarboxylate [prepared as described in step (i) above] and then crystallizing the product from diisopropyl ether, 1.0 g of the hydrochloride of the title compound was obtained as crystals, melting at 85 - 89°C

Nuclear Magnetic Resonance Spectrum (CDCℓ₃), δ ppm:
1.20 (3H, doublet, J = 6 Hz);
1.40 (6H, doublet, J = 6.5 Hz);
3.91 (1H, septet, J = 6 Hz);
5.09 (2H, singlet);
5.24 (2H, doublet, J = 6.5 Hz).

### 5(iii) Isopropyl 4-(1-hydroxy-1-methylethyl)-2-isopropoxymethylimidazole-5-carboxylate

950 mg of diisopropyl 2-isopropoxymethylimidazole-4,5-dicarboxylate hydrochloride [prepared as described in step (ii) above] in 10 ml of methylene chloride were added dropwise, whilst keeping the temperature at 7°C or less, to a solution of 4.5 ml of methylmagnesium iodide in diethyl ether (prepared from 298 mg of magnesium and 0.763 ml of methyl iodide) under a nitrogen atmosphere. The resulting mixture was stirred at room temperature for 2 hours, and then the reaction solution was concentrated by evaporation under reduced pressure. The resulting residue was dissolved in ethyl acetate, and a saturated aqueous solution of ammonium chloride was added, whilst ice-cooling. The mixture was stirred for 30 minutes, and then the ethyl acetate layer was separated. The extract was dried over anhydrous magnesium sulphate, after which it was concentrated by evaporation under reduced pressure. The resulting residue was purified by column chromatography through silica gel, using a 20 : 1 by volume mixture of methylene chloride and methanol as the eluent, to give 603 mg of the title compound as crystals, melting at 153.5 - 155°C

Nuclear Magnetic Resonance Spectrum (CDCℓ₃), δ ppm:
1.24 (6H, doublet, J = 6 Hz);
1.38 (6H, doublet, J = 6 Hz);
1.60 (6H, singlet);
3.75 (1H, septet, J = 6 Hz);
4.61 (2H, singlet);
5.26 (1H, septet, J = 6 Hz);
5.71 (1H, singlet).

### PREPARATION 6

### Methyl 4-(1-hydroxy-1-methylethyl)-2-(1-methoxyethyl)imidazole-5-carboxylate

### 6(i) Diethyl 1-benzyl-2-ethylimidazole-4,5-dicarboxylate

Following a procedure similar to that described in Preparation 1(i), 4.00 g of diethyl 2-ethylimidazole-4,5-dicarboxylate were benzylated, using 2.20 ml of benzyl bromide. The product was purified by column chromatography through silica gel, using a 1 : 1 by volume mixture of methylene chloride and ethyl acetate as the eluent, to give 5.19 g of the title compound as a syrup.

Nuclear Magnetic Resonance Spectrum (CDCℓ₃), δ ppm:
1.24 (3H, triplet, J = 7 Hz);
1.28 (3H, triplet, J = 7 Hz);
1.40 (3H, triplet, J = 7 Hz);
2.70 (2H, quartet, J = 7 Hz);
4.26 (2H, quartet, J = 7 Hz);
4.40 (2H, quartet, J = 7 Hz);
5.41 (2H, singlet);
7.01 (2H, doublet, J = 6 Hz);
7.27 - 7.35 (3H, multiplet).

### 6(ii) Diethyl 1-benzyl-2-(1-bromoethyl)imidazole-4,5-dicarboxylate

3.08 g of N-bromosuccinimide and 0.51 g of benzoyl peroxide were added to a solution of 5.19 g of diethyl 1-benzyl-2-ethylimidazole-4,5-dicarboxylate [prepared as described in step (i) above] in 100 ml of carbon tetrachloride, and the resulting mixture was heated under reflux for 1 hour. Following a procedure similar to that described in Preparation 1(ii), 6.29 g of the title compound were obtained as a syrup from the resulting reaction solution.

Nuclear Magnetic Resonance Spectrum (CDCℓ₃), δ ppm:
1.24 (3H, triplet, J = 7 Hz);
1.38 (3H, triplet, J = 7 Hz);
2.12 (3H, doublet, J = 6.5 Hz);
4.26 (2H, quartet, J = 7 Hz);
4.40 (2H, quartet, J = 7 Hz);
4.92 (1H, quartet, J = 6.5 Hz);
5.35 (1H, doublet, J = 16 Hz);
5.74 (1H, doublet, J = 16 Hz);
7.06 (2H, doublet, J = 6 Hz);
7.26 - 7.50 (3H, multiplet).

### 6(iii) Dimethyl 1-benzyl-2-(1-methoxyethyl)imidazole-4,5-dicarboxylate

Following a procedure similar to that described in Preparation 1(iii), but using 7.60 g of diethyl 1-benzyl-2-(1-bromoethyl)imidazole-4,5-dicarboxylate [prepared as described in step (ii) above] and purifying the product by column chromatography through silica gel, using a 3 : 2 by volume mixture of hexane and ethyl acetate as the eluent, 4.36 g of the title compound were obtained as a syrup.

Nuclear Magnetic Resonance Spectrum (CDCℓ₃), δ ppm:
1.51 (3H, doublet, J = 7 Hz);
3.23 (3H, singlet);
3.73 (3H, singlet);
3.83 (3H, singlet);
4.68 (1H, quartet, J = 7 Hz);
5.56 (1H, doublet, J = 16 Hz);
5.65 (1H, doublet, J = 16 Hz);
7.00 (2H, doublet, J = 7 Hz);
7.23 - 7.33 (3H, multiplet).

### 6(iv) Dimethyl 2-(1-methoxyethyl)imidazole-4,5-dicarboxylate

Following a procedure similar to that described in Preparation 1(iv), but using 3.30 g of dimethyl 1-benzyl-2-(1-methoxyethyl)imidazole-4,5-dicarboxylate [prepared as described in step (iii) above], 2.02 g of the hydrochloride of the title compound were obtained as a syrup.

Nuclear Magnetic Resonance Spectrum (CDCℓ₃), δ ppm:
1.74 (3H, doublet, J = 6.5 Hz);
3.42 (3H, singlet);
3.52 (3H, singlet);
4.00 (3H, singlet);
5.31 (1H, quartet, J = 6.5 Hz).

### 6(v) Methyl 2-(1-methoxyethyl)-4-(1-hydroxy-1-methylethyl)imidazole-5-carboxylate

A suspension of 1.9 g of dimethyl 2-(1-methoxyethyl) imidazole-4,5-dicarboxylate hydrochloride [prepared as described in step (iv) above] in 30 ml of methylene chloride was added dropwise, whilst keeping the temperature at 5°C or less, to a solution of 30 ml of methylmagnesium iodide in diethyl ether (prepared from 746 mg of magnesium and 1.91 ml of methyl iodide), under a nitrogen atmosphere. The resulting mixture was stirred at room temperature for 1 hour, after which it was concentrated by evaporation under reduced pressure. The residue was dissolved in ethyl acetate, and a saturated aqueous solution of ammonium chloride solution was added, whilst ice-cooling. The mixture was stirred for 30 minutes, and then the ethyl acetate layer was separated. The extract was dried over anhydrous magnesium sulphate, and then concentrated by evaporation under reduced pressure. The residue was purified by column chromatography through silica gel, using a 20 : 1 by volume mixture of methylene chloride and methanol as the eluent, to give 1.12 g of the title compound as a syrup.

Nuclear Magnetic Resonance Spectrum (CDCℓ₃), δ ppm:
1.52 (3H, doublet, J = 6 Hz);
1.61 & 1.67 (total 6H, each singlet);
3.36 & 3.40 (total 3H, each singlet);
3.92 & 3.94 (total 3H, each singlet);
4.53 (1H, quartet, J = 6 Hz);
5.51 & 5.62 (total 1H, each singlet).

### PREPARATION 7

### Ethyl 2-acetoxymethyl-4-(1-hydroxy-1-methylethyl)-imidazole-5-carboxylate

### 7(i) Diethyl 2-acetoxymethyl-1-benzylimidazole-4,5-dicarboxylate

1.11 g of sodium acetate were added to a solution of 2.67 g of diethyl 1-benzyl-2-bromomethylimidazole-4,5-dicarboxylate [prepared as described in Preparation 1(ii)] in 30 ml of dimethylformamide, and the resulting mixture was heated at 40°C for 5 hours. At the end of this time, the reaction solution was mixed with ethyl acetate and water, and then the ethyl acetate layer was separated. The resulting ethyl acetate extract was washed with an aqueous solution of sodium chloride and then dried over anhydrous sodium sulphate. The solvent was then removed by distillation under reduced pressure. The residue was purified by column chromatography through silica gel, using a 1 : 1 by volume mixture of hexane and ethyl acetate as the eluent, to give 1.52 g of the title compound as a syrup.

Nuclear Magnetic Resonance Spectrum (CDCℓ₃), δ ppm:
1.23 (3H, triplet, J = 7 Hz);
1.39 (3H, triplet, J = 7 Hz);
1.89 (3H, singlet) ;
4.27 (2H, quartet, J = 7 Hz);
4.40 (2H, quartet, J = 7 Hz);
5.15 (2H, singlet);
5.47 (2H, singlet);
7.01 (2H, doublet, J = 6 Hz);
7.29 - 7.34 (3H, multiplet).

### 7(ii) Diethyl 2-acetoxymethylimidazole-4,5-dicarboxylate

Following a procedure similar to that described in Preparation 1(iv), but using 2.00 g of diethyl 2-acetoxymethyl-1-benzylimidazole-4,5-dicarboxylate [prepared as described in step (i) above] as a starting material, 1.70 g of the hydrochloride of the title compound were obtained as a syrup.

Nuclear Magnetic Resonance Spectrum (CDCℓ₃), δ ppm:
1.39 (6H, triplet, J = 7 Hz);
2.12 (3H, singlet);
4.40 (4H, quartet, J = 7 Hz);
5.64 (2H, singlet);
13.1 (3H, broad singlet).

1.70 g of the hydrochloride of the title compound prepared as described above were dissolved in a mixture of ethyl acetate and water, and the resulting solution was mixed with 0.47 g of sodium hydrogencarbonate. The ethyl acetate layer was then separated, washed with an aqueous solution of sodium chloride and dried over anhydrous sodium sulphate, after which the solvent was removed by distillation under reduced pressure, to give 1.49 g of the title compound as a syrup.

Nuclear Magnetic Resonance Spectrum (CDCℓ₃), δ ppm:
1.34 (6H, doublet, J = 7 Hz);
2.06 (3H, singlet);
4.36 (4H, quartet, J = 7 Hz);
5.20 (2H, singlet).

### 7(iii) Ethyl 2-acetoxymethyl-4-(1-hydroxy-1-methylethyl)imidazole-5-carboxylate

Following a procedure similar to that described in Preparation 3(iii), 1.54 g of diethyl 2-acetoxymethylimidazole-4,5-dicarboxylate were reacted with 6.5 equivalents of methylmagnesium iodide. Ethyl acetate was then added to the reaction solution, whilst ice-cooling, and the reaction solution was concentrated by evaporation under reduced pressure. The resulting residue was mixed with 50 ml of pyridine and 25 ml of acetic anhydride, and left at room temperature overnight. At the end of this time, 10 ml of methanol were added to the reaction solution, which was then stirred for 30 minutes. The solution was then concentrated by evaporation under reduced pressure. The residue was mixed with water and ethyl acetate, and the ethyl acetate layer was separated, washed twice with a saturated aqueous solution of sodium hydrogencarbonate and then once with an aqueous solution of sodium chloride, and then dried over anhydrous sodium sulphate. The solvent was removed by distillation under reduced pressure, and the resulting residue was purified by column chromatography through silica gel, using a 1 : 4 by volume mixture of hexane and ethyl acetate as the eluent, to give 1.46 g of the title compound as a syrup.

Nuclear Magnetic Resonance Spectrum (CDCℓ₃), δ ppm:
1.33 (3H, triplet, J = 6.5 Hz);
1.64 (6H, singlet);
2.06 (3H, singlet);
4.37 (2H, quartet, J = 6.5 Hz);
5.10 (2H, singlet);
5.83 (1H, broad singlet).

### PREPARATION 8

### Ethyl 4-(1-hydroxy-1-methylethyl)-2-methylthioimidazole-5-carboxylate

### 8(i) Diethyl 2-methylthioimidazole-4,5-dicarboxylate

1.14 g of potassium carbonate and 1.17 g of methyl iodide were added to a solution of 2.00 g of diethyl 2-mercaptoimidazole-4,5-dicarboxylate in 100 ml of acetone, and the mixture was heated under reflux, whilst stirring, for 30 minutes. At the end of this time, the insoluble matter was removed from the reaction mixture by filtration, and the filtrate was concentrated by evaporation under reduced pressure. The residue was purified by column chromatography through silica gel, using ethyl acetate as the eluent, to give 1.72 g of the title compound as crystals, melting at 119 - 121°C.

Nuclear Magnetic Resonance Spectrum (CDCℓ₃), δ ppm:
1.37 (6H, triplet, J = 7.5 Hz);
2.67 (3H, singlet);
4.39 (4H, quartet, J = 7.5 Hz).

### 8(ii) Ethyl 4-(1-hydroxy-1-methylethyl)-2-methylthioimidazole-5-carboxylate

A solution of 3.30 g of methyl iodide in 5 ml of diethyl ether was added dropwise to a mixture of 565 mg of magnesium in 30 ml of diethyl ether, under a nitrogen atmosphere, and the resulting solution was heated under reflux, whilst stirring, for 30 minutes. At the end of this time, a solution of 1.50 g of diethyl 2-methylthioimidazole-4,5-dicarboxylate[prepared as described in step (i) above] in 10 ml of methylene chloride was added dropwise to the reaction solution, and then the solution was stirred at room temperature for 1 hour. 50 ml of a saturated aqueous solution of ammonium chloride were then added to the reaction mixture, after which the mixture was stirred, and then the product was extracted with ethyl acetate. The extract was washed with a saturated aqueous solution of sodium chloride and then dried over anhydrous magnesium sulphate. The solvent was removed by distillation under reduced pressure, and the resulting crystalline residue was washed with hexane, to give 1.00 g of the title compound, melting at 128 - 129°C.

Nuclear Magnetic Resonance Spectrum (CDCℓ₃), δ ppm:
1.36 (3H, triplet, J = 7.5 Hz);
1.62 (6H, singlet);
2.62 (3H, singlet);
4.35 (2H, quartet, J = 7.5 Hz);
5.74 (1H, singlet).

### PREPARATION 9

### Ethyl 2-ethylthio-4-(1-hydroxy-1-methylethyl)imidazole-5-carboxylate

### 9(i) Diethyl 2-ethylthioimidazole-4,5-dicarboxylate

1.19 g of potassium carbonate and 1.34 g of ethyl iodide were added to a solution of 2.00 g of diethyl 2-mercaptoimidazole-4,5-dicarboxylate in 40 ml of acetone, and the resulting mixture was heated under reflux, whilst stirring, for 2 hours. At the end of this time, the mixture was treated in a similar manner to that described in Preparation 8(i). The residue was purified by column chromatography through silica gel, using ethyl acetate as the eluent, to give 2.03 g of the title compound as an oil.

Nuclear Magnetic Resonance Spectrum (CDCℓ₃), δ ppm:
1.30 - 1.40 (9H, multiplet);
3.20 (2H, quartet, J = 7.5 Hz);
4.39 (4H, quartet, J = 7.5 Hz).

### 9(ii) Ethyl 2-ethylthio-4-(1-hydroxy-1-methylethyl)imidazole-5-carboxylate

4.20 g of methyl iodide were added dropwise to a mixture of 714 mg of magnesium in 30 ml of diethyl ether, under a nitrogen atmosphere, and the resulting solution was heated under reflux, whilst stirring, for 30 minutes. At the end of this time, a solution of 2.00 g of diethyl 2-ethylthioimidazole-4,5-dicarboxylate [prepared as described in step (i) above] in 20 ml of methylene chloride was added dropwise to the reaction solution, and the mixture was treated in a similar manner to that described in Preparation 8(ii). The resulting crystalline residue was washed with a mixture of hexane and diisopropyl ether, to give 1.32 g of the title compound, melting at 82 - 85°C.

Nuclear Magnetic Resonance Spectrum (CDCℓ₃), δ ppm:
1.30-1.42 (6H, multiplet);
1.62 (6H, singlet);
3.14 (2H, quartet, J = 7.5 Hz);
4.37 (2H, quartet, J = 7.5 Hz);
5.64 (1H, singlet).

## Claims

1. A compound of formula (I): 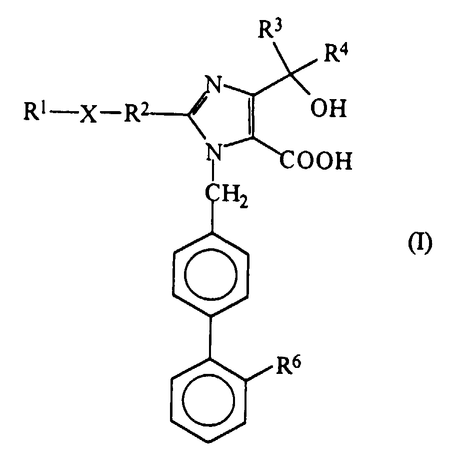 in which:
R¹ represents a hydrogen atom, an alkyl group having from 1 to 6 carbon atoms, a cycloalkyl group having from 3 to 6 ring carbon atoms or an alkanoyl group having from 1 to 6 carbon atoms;
R² represents a single bond or an alkylene or alkylidene group having from 1 to 4 carbon atoms;
R³ and R⁴ are the same or different and each represents an alkyl group having from 1 to 6 carbon atoms;
R⁶ represents a carboxy group or a tetrazol-5-yl group; and
X represents an oxygen or sulphur atom;
and pharmaceutically acceptable salts and esters thereof.

2. The compound of Claim 1, wherein said compound has the formula (Ia): 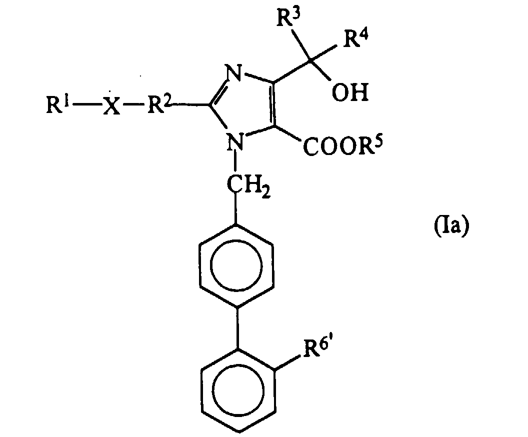 in which: R¹, R², R³, R⁴ and X are as defined in Claim 1, R⁵ represents a hydrogen atom or an ester group and R^{6'} represents a carboxy group, an esterified carboxy group or a tetrazol-5-yl group.

3. The compound of Claim 1, wherein R¹ represents a hydrogen atom, a methyl group, an ethyl group, a cyclopropyl group or an acetyl group.

4. The compound of Claim 1, wherein R² represents a single bond, a methylene group, an ethylene group or an ethylidene group.

5. The compound of Claim 1, wherein R³ and R⁴ are the same or different and each represents a methyl group or an ethyl group.

6. The compound of Claim 2, wherein R⁵ represents
a hydrogen atom,
an alkyl group having from 1 to 4 carbon atoms,
a phenyl group,
a phenyl group substituted by at least one substituent selected from methyl groups, ethyl groups, methoxy groups, ethoxy groups, fluorine atoms and chlorine atoms,
a naphthyl group,
a benzyl group,
a benzyl group substituted by at least one substituent selected from methyl groups, ethyl groups, methoxy groups, ethoxy groups, fluorine atoms and chlorine atoms,
a diphenylmethyl group,
a naphthylmethyl group,
an alkanoyloxyalkyl group in which the alkanoyl part has from 1 to 5 carbon atoms and the alkyl part has from 1 to 4 carbon atoms,
a cycloalkanecarbonyloxyalkyl group in which the cycloalkane part has 5 or 6 carbon atoms and the alkyl part has from 1 to 4 carbon atoms,
an alkoxycarbonyloxyalkyl group in which the alkoxy and alkyl parts each have from 1 to 4 carbon atoms,
a cycloalkyloxycarbonyloxyalkyl group in which the cycloalkyl part has 5 or 6 carbon atoms and the alkyl part has from 1 to 4 carbon atoms,
a (5-phenyl-2-oxo-1,3-dioxolen-4-yl)methyl group,
a (5-alkyl-2-oxo-1,3-dioxolen-4-yl)methyl group,
in which the alkyl part has from 1 to 4 carbon atoms, or
a phthalidyl group.

7. The compound of Claim 2, wherein R^{6'} represents a carboxy group or a tetrazol-5-yl group.

8. The compound of Claim 2, wherein:
R¹ represents a hydrogen atom, a methyl group, an ethyl group, a cyclopropyl group or an acetyl group;
R² represents a single bond, a methylene group, an ethylene group or an ethylidene group;
R³ and R⁴ are the same or different and each represents a methyl group or an ethyl group;
R⁵ represents
a hydrogen atom,
an alkyl group having from 1 to 4 carbon atoms,
a phenyl group,
a phenyl group substituted by at least one substituent selected from methyl groups, ethyl groups, methoxy groups, ethoxy groups, fluorine atoms and chlorine atoms,
a naphthyl group,
a benzyl group,
a benzyl group substituted by at least one substituent selected from methyl groups, ethyl groups, methoxy groups, ethoxy groups, fluorine atoms and chlorine atoms,
a diphenylmethyl group,
a naphthylmethyl group,
an alkanoyloxyalkyl group in which the alkanoyl part has from 1 to 5 carbon atoms and the alkyl part has from 1 to 4 carbon atoms,
a cycloalkanecarbonyloxyalkyl group in which the cycloalkane part has 5 or 6 carbon atoms and the alkyl part has from 1 to 4 carbon atoms,
an alkoxycarbonyloxyalkyl group in which the alkoxy and alkyl parts each have from 1 to 4 carbon atoms,
a cycloalkyloxycarbonyloxyalkyl group in which the cycloalkyl part has 5 or 6 carbon atoms and the alkyl part has from 1 to 4 carbon atoms,
a (5-phenyl-2-oxo-1,3-dioxolen-4-yl)methyl group,
a (5-alkyl-2-oxo-1,3-dioxolen-4-yl)methyl group, in which the alkyl part has from 1 to 4 carbon atoms, or
a phthalidyl group; and
R^{6'} represents a carboxy group or a tetrazol-5-yl group.

9. The compound of Claim 1, wherein the group of formula R¹-X-R²- represents a methoxymethyl group, an ethoxymethyl group, a 1-methoxyethyl group, a 2-methoxyethyl group, a 2-ethoxyethyl group, a methylthiomethyl group, an ethylthiomethyl group, a 1-methylthioethyl group, 2-methylthioethyl, a 2-ethylthioethyl group, a methylthio group or an ethylthio group.

10. The compound of Claim 2, wherein R⁵ represents a hydrogen atom, an alkyl group having from 1 to 4 carbon atoms, a benzyl group, an alkanoyloxyalkyl group in which the alkanoyl part has from 1 to 5 carbon atoms and the alkyl part has 1 or 2 carbon atoms, a cycloalkanecarbonyloxyalkyl group in which the cycloalkane part has 5 or 6 carbon atoms and the alkyl part has 1 or 2 carbon atoms, an alkoxycarbonyloxyalkyl group in which the alkoxy part has from 1 to 4 carbon atoms and the alkyl part has 1 or 2 carbon atoms, a cycloalkyloxycarbonyloxyalkyl group in which the cycloalkyl part has 5 or 6 carbon atoms and the alkyl part has 1 or 2 carbon atoms, a (5-phenyl-, 5-methyl- or 5-ethyl- 2-oxo-1,3-dioxolen-4-yl)methyl group, or a phthalidyl group.

11. The compound of Claim 2, wherein:
the group of formula R¹-X-R²- represents a methoxymethyl group, an ethoxymethyl group, a 1-methoxyethyl group, a 2-methoxyethyl group, a 2-ethoxyethyl group, a methylthiomethyl group, an ethylthiomethyl group, a 1-methylthioethyl group, 2-methylthioethyl, a 2-ethylthioethyl group, a methylthio group or an ethylthio group;
R³ and R⁴ are the same or different and each represents a methyl or ethyl group;
R⁵ represents a hydrogen atom, an alkyl group having from 1 to 4 carbon atoms, a benzyl group, an alkanoyloxyalkyl group in which the alkanoyl part has from 1 to 5 carbon atoms and the alkyl part has 1 or 2 carbon atoms, a cycloalkanecarbonyloxyalkyl group in which the cycloalkane part has 5 or 6 carbon atoms and the alkyl part has 1 or 2 carbon atoms, an alkoxycarbonyloxyalkyl group in which the alkoxy part has from 1 to 4 carbon atoms and the alkyl part has 1 or 2 carbon atoms, a cycloalkyloxycarbonyloxyalkyl group in which the cycloalkyl part has 5 or 6 carbon atoms and the alkyl part has 1 or 2 carbon atoms, a (5-phenyl-, 5-methyl- or 5-ethyl- 2-oxo-1,3-dioxolen-4-yl)methyl group, or a phthalidyl group; and
R^{6'} represents a carboxy group or a tetrazol-5-yl group.

12. The compound of Claim 1, wherein the group of formula R¹-X-R²- represents a methoxymethyl group, an ethoxymethyl group, a methylthiomethyl group, a methylthio group or an ethylthio group.

13. The compound of Claim 1, wherein R³ and R⁴ both represent methyl groups.

14. The compound of Claim 2, wherein R⁵ represents a hydrogen atom, a pivaloyloxymethyl group, an ethoxycarbonyloxymethyl group, a 1-(ethoxycarbonyloxy)ethyl group, an isopropoxycarbonyloxymethyl group, a 1-(isopropoxycarbonyloxy)ethyl group, a (5-methyl-2-oxo-1,3-dioxolen-4-yl)methyl group or a phthalidyl group.

15. The compound of Claim 2, wherein:
the group of formula R¹-X-R²- represents a methoxymethyl group, an ethoxymethyl group, a methylthiomethyl group, a methylthio group or an ethylthio group;
R³ and R⁴ both represent methyl groups;
R⁵ represents a hydrogen atom, a pivaloyloxymethyl group, an ethoxycarbonyloxymethyl group, a 1-(ethoxy-carbonyloxy)ethyl group, an isopropoxycarbonyloxymethyl group, a 1-(isopropoxycarbonyloxy)ethyl group, a (5-methyl-2-oxo-1,3-dioxolen-4-yl)methyl group or a phthalidyl group; and
R^{6'} represents a carboxy group or a tetrazol-5-yl group.

16. The compound of Claim 1, selected from:
4-(1-hydroxy-1-methylethyl)-2-methoxymethyl-1-{4-[2-(tetrazol-5-yl)phenyl]phenyl}methylimidazole-5-carboxylic acid;
2-ethoxymethyl-4-(1-hydroxy-1-methylethyl)-1-{4-[2-(tetrazol-5-yl)phenyl]phenyl}methylimidazole-5-carboxylic acid;
2-ethylthio-4-(1-hydroxy-1-methylethyl)-1-{4-[2-(tetrazol-5-yl)phenyl]phenyl}methylimidazole-5-carboxylic acid;
pivaloyloxymethyl 4-(1-hydroxy-1-methylethyl)-2-methoxy-methyl-1-{4-[2-(tetrazol-5-yl)phenyl]phenyl}methyl-imidazole-5-carboxylate;
pivaloyloxymethyl 2-ethoxymethyl-4-(1-hydroxy-1-methyl-ethyl)-1-{4-[2-(tetrazol-5-yl)phenyl]phenyl}methyl-imidazole-5-carboxylate;
pivaloyloxymethyl 2-ethylthio-4-(1-hydroxy-1-methyl-ethyl)-1-{4- [2- (tetrazol-5-yl)phenyl]phenyl}methyl-imidazole-5-carboxylate;
(5-methyl-2-oxo-1,3-dioxolen-4-yl)methyl 4-(1-hydroxy-1-methylethyl) -2-methoxymethyl-1-{4-[2-(tetrazol-5-yl)-phenyl]phenyl}methylimidazole-5-carboxylate;
(5-methyl-2-oxo-1,3-dioxolen-4-yl)methyl 2-ethoxymethyl-4-(1-hydroxy-1-methylethyl)-1-{4-[2-(tetrazol-5-yl)-phenyl]phenyl}methylimidazole-5-carboxylate;
(5-methyl-2-oxo-1,3-dioxolen-4-yl)methyl 2-ethylthio-4-(1-hydroxy-1-methylethyl)-1-{4-[2-(tetrazol-5-yl)-phenyl]phenyl}methylimidazole-5-carboxylate;
ethoxycarbonyloxymethyl 4-(1-hydroxy-1-methylethyl)-2-methoxymethyl-1-{4-[2-(tetrazol-5-yl)phenyl]phenyl}-methylimidazole-5-carboxylate;
ethoxycarbonyloxymethyl 2-ethoxymethyl-4-(1-hydroxy-1-methylethyl)-1-{4-[2-(tetrazol-5-yl)phenyl]phenyl}-methylimidazole-5-carboxylate;
ethoxycarbonyloxymethyl 2-ethylthio-4-(1-hydroxy-1-methylethyl)-1-{4-[2-(tetrazol-5-yl)phenyl]phenyl}-methylimidazole-5-carboxylate;
isopropoxycarbonyloxymethyl 4-(1-hydroxy-1-methyl-ethyl)-2-methoxymethyl-1-{4-[2-(tetrazol-5-yl)phenyl]-phenyl}methylimidazole-5-carboxylate;
isopropoxycarbonyloxymethyl 2-ethoxymethyl-4-(1-hydroxy-1-methylethyl)-1-{4-[2-(tetrazol-5-yl)phenyl]phenyl}-methylimidazole-5-carboxylate;
isopropoxycarbonyloxymethyl 2-ethylthio-4-(1-hydroxy-1-methylethyl)-1-{4-[2-(tetrazol-5-yl)phenyl]phenyl}-methylimidazole-5-carboxylate;
and pharmaceutically acceptable salts and esters thereof.

17. A pharmaceutical composition for the treatment or prophylaxis of hypertension or of a cardiovascular disease, which comprises an effective amount of an anti-hypertensive agent in admixture with a pharmaceutically acceptable carrier or diluent, wherein the anti-hypertensive agent is selected from compounds of formula (I) and pharmaceutically acceptable salts and esters thereof, as claimed in any one of Claims 1 to 16.

18. A pharmaceutical composition for the treatment or prophylaxis of hypertension or of a cardiovascular disease, which comprises an effective amount of an anti-hypertensive agent in admixture with a pharmaceutically acceptable carrier or diluent, wherein the anti-hypertensive agent is selected from compounds of formula (Ia) and pharmaceutically acceptable salts and esters thereof, as claimed in Claim 2.

19. Compounds of formula (I) and pharmaceutically acceptable salts and esters thereof, as claimed in any one of Claims 1 to 16, for use as a pharmaeutical.

20. Compounds of formula (Ia) and pharmaceutically acceptable salts and esters thereof, as claimed in Claim 2, for use as a pharmaceutical.

21. Compounds of formula (I) and pharmaceutically acceptable salts and esters thereof as defined in any one of claims 1 to 16 for use in the treatment or prophylaxis of hypertension or of a cardiovascular disease.

22. The use of a compound of formula (I) as defined in any one of claims 1 to 16, or a pharmaceutically acceptable salt or ester thereof, in the manufacture of a medicament for the treatment or prophylaxis of hypertension or of a cardiovascular disease.

23. A process for the preparation of a compound of formula (I), as defined in any one of Claims 1 to 16, which process comprises reacting a compound of formula (II): 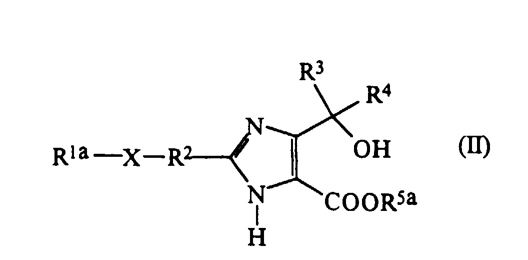 (in which:
R², R³, R⁴ and X are as defined above;
R^{1a} represents
when X represents an oxygen atom: a hydrogen atom, an alkyl group having from 1 to 6 carbon atoms, a cycloalkyl group having from 3 to 6 carbon atoms or a group of formula R⁷CO-, where R⁷ represents a hydrogen atom, an alkyl group having from 1 to 6 carbon atoms or an aryl group having from 6 to 10 ring carbon atoms; or
when X represents a sulphur atom: an alkyl group having from 1 to 6 carbon atoms, a cycloalkyl group having from 3 to 6 carbon atoms, a mercapto-protecting group or said group of formula R⁷CO-; and
R^{5a} represents a carboxy-protecting group) with a compound of formula (III): 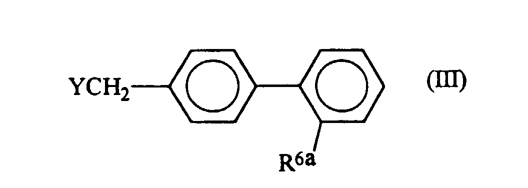 (in which Y represents a halogen atom; and R^{6a} represents a protected carboxy group, a protected tetrazol-5-yl group, a cyano group, a carbamoyl group or an alkylcarbamoyl group in which the alkyl part has from 1 to 6 carbon atoms) to give a compound of formula (Ib): 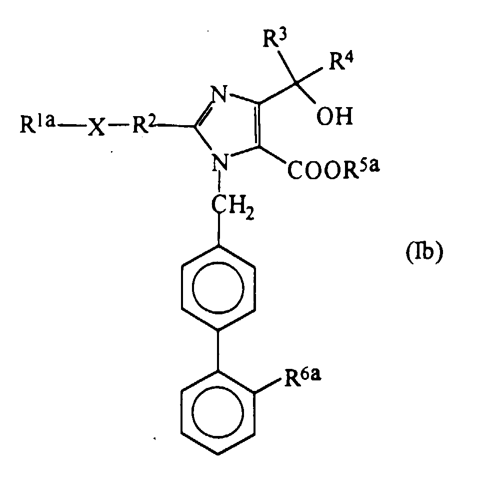 (in which R^{1a}, R², R³, R⁴, R^{5a}, R^{6a}, and X are as defined above);
and, if necessary, converting any group represented by R^{1a} or R^{6a} to a group represented by R¹ or R⁶, respectively;
and, optionally, removing any carboxy-protecting group, salifying and/or esterifying the resulting compound.

## Patentansprüche

1. Verbindung der Formel (I) 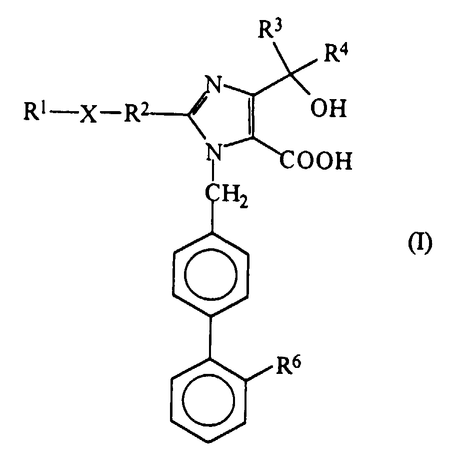 in der
R¹ ein Wasserstoffatom, einen Alkylrest mit 1 bis 6 Kohlenstoffatomen, einen Cycloalkylrest mit 3 bis 6 Ringkohlenstoffatomen oder einen Alkanoylrest mit 1 bis 6 Kohlenstoffatomen bedeutet;
R² eine Einfachbindung oder einen Alkylen- oder Alkylidenrest mit 1 bis 4 Kohlenstoffatomen bedeutet;
R³ und R⁴ gleich oder verschieden sind und jeweils einen Alkylrest mit 1 bis 6 Kohlenstoffatomen bedeuten;
R⁶ einen Carboxylrest oder einen Tetrazol-5-ylrest bedeutet; und
X ein Sauerstoff- oder Schwefelatom bedeutet;
und pharmazeutisch verträgliche Salze und Ester davon.

2. Verbindung nach Anspruch 1, wobei die Verbindung die Formel (Ia) aufweist 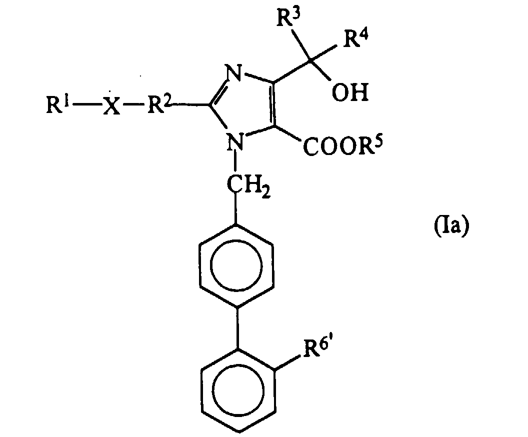 in der R¹, R², R³, R⁴ und X die in Anspruch 1 definierten Bedeutungen haben, R⁵ ein Wasserstoffatom oder eine Estergruppe bedeutet und R^{6'} eine Carboxylgruppe, eine veresterte Carboxylgruppe oder eine Tetrazol -5-ylgruppe bedeutet.

3. Verbindung nach Anspruch 1, wobei R¹ ein Wasserstoffatom, einen Methylrest, einen Ethylrest, einen Cyclopropylrest oder einen Acetylrest bedeutet.

4. Verbindung nach Anspruch 1, wobei R² eine Einfachbindung, einen Methylenrest, einen Ethylenrest oder einen Ethylidenrest bedeutet.

5. Verbindung nach Anspruch 1, wobei R³ und R⁴ gleich oder verschieden sind und jeweils einen Methylrest oder einen Ethylrest bedeuten.

6. Verbindung nach Anspruch 2, wobei R⁵ ein Wasserstoffatom, einen Alkylrest mit 1 bis 4 Kohlenstoffatomen, einen Phenylrest, einen durch mindestens einen unter Methylresten, Ethylresten, Methoxyresten, Ethoxyresten, Fluoratomen und Chloratomen ausgewählten Substituenten substituierten Phenylrest, einen Naphthylrest, einen Benzylrest, einen durch mindestens einen unter Methylresten, Ethylresten, Methoxyresten, Ethoxyresten, Fluoratomen und Chloratomen ausgewählten Substituenten substituierten Benzylrest, einen Diphenylmethylrest, einen Naphthylmethylrest, einen Alkanoyloxyalkylrest, in dem der Alkanoylteil 1 bis 5 Kohlenstoffatome und der Alkylteil 1 bis 4 Kohlenstoffatome aufweist, einen Cycloalkancarbonyloxyalkylrest, in dem der Cycloalkanteil 5 oder 6 Kohlenstoffatome und der Alkylteil 1 bis 4 Kohlenstoffatome aufweist, einen Alkoxycarbonyloxyalkylrest, in dem der Alkoxy- und Alkylteil jeweils 1 bis 4 Kohlenstoffatome aufweist, einen Cycloalkyloxycarbonyloxyalkylrest, in dem der Cycloalkylteil 5 oder 6 Kohlenstoffatome und der Alkyteil 1 bis 4 Kohlenstoffatome aufweist, einen (5-Phenyl-2-oxo-1,3-dioxolen-4-yl)-methylrest, einen (5-Alkyl-2-oxo-1,3-dioxolen-4-yl)-methylrest, in dem der Alkylteil 1 bis 4 Kohlenstoffatome aufweist, oder einen Phthalidylrest bedeutet.

7. Verbindung nach Anspruch 2, wobei R^{6'} einen Carboxylrest oder einen Tetrazol-5-ylrest bedeutet.

8. Verbindung nach Anspruch 2, wobei
R¹ ein Wasserstoffatom, einen Methylrest, einen Ethylrest, einen Cyclopropylrest oder einen Acetylrest bedeutet;
R² eine Einfachbindung, einen Methylenrest, einen Ethylenrest oder einen Ethylidenrest bedeutet;
R³ und R⁴ gleich oder verschieden sind und jeweils einen Methylrest oder einen Ethylrest bedeuten;
R⁵ ein Wasserstoffatom, einen Alkylrest mit 1 bis 4 Kohlenstoffatomen, einen Phenylrest, einen durch mindestens einen unter Methylresten, Ethylresten, Methoxyresten, Ethoxyresten, Fluoratomen und Chloratomen ausgewählten Substituenten substituierten Phenylrest, einen Naphthylrest, einen Benzylrest, einen durch mindestens einen unter Methylresten, Ethylresten, Methoxyresten, Ethoxyresten, Fluoratomen und Chloratomen ausgewählten Sutstituenten substituierten Benzylrest, einen Diphenylmethylrest, einen Naphthylmethylrest, einen Alkanoyloxyalkylrest, in dem der Alkanoylteil 1 bis 5 Kohlenstoffatome und der Alkylteil 1 bis 4 Kohlenstoffatome aufweist, einen Cycloalkancarbonyloxyalkylrest, in dem der Cycloalkanteil 5 oder 6 Kohlenstoffatome und der Alkylteil 1 bis 4 Kohlenstoffatome aufweist, einen Alkoxycarbonyloxyalkylrest, in dem der Alkoxy- und Alkylteil jeweils 1 bis 4 Kohlenstoffatome aufweist, einen Cycloalkyloxycarbonyloxyalkylrest, in dem der Cycloalkylteil 5 oder 6 Kohlenstoffatome und der Alkyteil 1 bis 4 Kohlenstoffatome aufweist, einen (5-Phenyl-oxo-1,3dioxolen-4-yl)-methylrest, einen (5-Alkyl-2-oxo-1,3-dioxolen-4-yl)-methylrest, in dem der Alkylteil 1 bis 4 Kohlenstoffatome aufweist, oder einen Phthalidylrest bedeutet; und
R^{6'} einen Carboxylrest oder einen Tetrazol-5-ylrest bedeutet.

9. Verbindung nach Anspruch 1, wobei der Rest der Formel R¹-X-R²- einen Methoxymethylrest, einen Ethoxymethylrest, einen 1-Methoxyethylrest, einen 2-Methoxyethylrest, einen 2-Ethoxyethylrest, einen Methylthiomethylrest, einen Ethylthiomethylrest, einen 1-Methylthioethylrest, einen 2-Methylthioethylrest, einen 2-Ethylthioethylrest, einen Methylthiorest oder einen Ethylthiorest bedeutet.

10. Verbindung nach Anspruch 2, wobei R⁵ ein Wasserstoffatom, einen Alkylrest mit 1 bis 4 Kohlenstoffatomen, einen Benzylrest, einen Alkanoyloxyalkylrest, in dem der Alkanoylteil 1 bis 5 Kohlenstoffatome und der Alkylteil 1 oder 2 Kohlenstoffatome aufweist, einen Cycloalkancarbonyloxyalkylrest, in dem der Cycloalkanteil 5 oder 6 Kohlenstoffatome und der Alkylteil 1 oder 2 Kohlenstoffatome aufweist, einen Alkoxycarbonyloxyalkylrest, in dem der Alkoxyteil 1 bis 4 Kohlenstoffatome und der Alkylteil 1 oder 2 Kohlenstoffatome aufweist, einen Cycloalkyloxycarbonyloxyalkylrest, in dem der Cycloalkylteil 5 oder 6 Kohlenstoffatome und der Alkylteil 1 oder 2 Kohlenstoffatome aufweist, einen (5-Phenyl-, 5-Methyl- oder 5-Ethyl-2-oxo-1,3-dioxolen-4-yl)-methylrest oder einen Phthalidylrest bedeutet.

11. Verbindung nach Anspruch 2, wobei
der Rest der Formel R¹-X-R²⁻ einen Methoxymethylrest, einen Ethoxymethylrest, einen 1-Methoxyethylrest, einen 2-Methoxyethylrest, einen 2-Ethoxyethylrest, einen Methylthiomethylrest, einen Ethylthiomethylrest, einen 1-Methylthioethylrest, einen 2-Methylthioethylrest, einen 2-Ethylthioethylrest, einen Methylthiorest oder einen Ethylthiorest bedeutet;
R³ und R⁴ gleich oder verschieden sind und jeweils einen Methyl- oder Ethylrest bedeuten;
R⁵ ein Wasserstoffatom, einen Alkylrest mit 1 bis 4 Kohlenstoffatomen, einen Benzylrest, einen Alkanoyloxyalkylrest, in dem der Alkanoylteil 1 bis 5 Kohlenstoffatome und der Alkylteil 1 oder 2 Kohlenstoffatome aufweist, einen Cycloalkancarbonyloxyalkylrest, in dem der Cycloalkanteil 5 oder 6 Kohlenstoffatome und der Alkylteil 1 oder 2 Kohlenstoffatome aufweist, einen Alkoxycarbonyloxyalkylrest, in dem der Alkoxyteil 1 bis 4 Kohlenstoffatome und der Alkylteil 1 oder 2 Kohlenstoffatome aufweist, einen Cycloalkyloxycarbonyloxyalkylrest, in dem der Cycloalkylteil 5 oder 6 Kohlenstoffatome und der Alkylteil 1 oder 2 Kohlenstoffatome aufweist, einen (5-Phenyl-, 5-Methyl - oder 5-Ethyl-2-oxo-1,3-dioxolen-4-yl)-methylrest oder einen Phthalidylrest bedeutet; und
R^{6'} einen Carboxylrest oder einen Tetrazol-5-ylrest bedeutet.

12. Verbindung nach Anspruch 1, wobei der Rest der Formel R¹-X-R²⁻ einen Methoxymethylrest, einen Ethoxymethylrest, einen Methylthiomethylrest, einen Methylthiorest oder einen Ethylthiorest bedeutet.

13. Verbindung nach Anspruch 1, wobei R³ und R⁴ jeweils Methylreste bedeuten.

14. Verbindung nach Anspruch 2, wobei R⁵ ein Wasserstoffatom, einen Pivaloyloxymethylrest, einen Ethoxycarbonyloxymethylrest, einen 1-(Ethoxycarbonyloxy)-ethylrest, einen Isopropoxycarbonyloxymethylrest, einen 1-(Isopropoxycarbonyloxy)-ethylrest, einen (5-Methyl-2-oxo-1,3-dioxolen-4-yl)-methylrest oder einen Phthalidylrest bedeutet.

15. Verbindung nach Anspruch 2, wobei
der Rest der Formel R¹-X-R²⁻ einen Methoxymethylrest, einen Ethoxymethylrest, einen Methylthiomethylrest, einen Methylthiorest oder einen Ethylthiorest bedeutet;
R³ und R⁴ jeweils Methylreste bedeuten;
R⁵ ein Wasserstoffatom, einen Pivaloyloxymethylrest, einen Ethoxycarbonyloxymethylrest, einen 1-(Ethoxycarbonyloxy)-ethylrest, einen Isopropoxycarbonyloxymethylrest, einen 1-(Isopropoxycarbonyloxy)-ethylrest, einen (5-Methyl-2-oxo-1,3-dioxolen-4-yl)-methylrest oder einen Phthalidylrest bedeutet; und
R^{6'} einen Carboxylrest oder einen Tetrazol-5-ylrest bedeutet.

16. Verbindung nach Anspruch 1, ausgewählt unter:
4-(1-Hydroxy-1-methylethyl)-2-methoxymethyl-1-{4-(2-(tetrazol-5-yl)-phenyl]-phenyl}-methylimidazol-5-carbonsäure;
2-Ethoxymethyl-4-(1-hydroxy-1-methylethyl)-1-{4-[2-(tetrazol-5-yl)-phenyl]-phenyl}-methylimidazol-5-carbonsäure;
2-Ethylthio-4-(1-hydroxy-1-methylethyl)-1-{4-[2-(tetrazol-5-yl)-phenyl]-phenyl}-methylimidazol-5-carbonsäure;
Pivaloyloxymethyl-4-{1-hydroxy-1-methylethyl)-2-methoxymethyl-1-{4-[2-(tetrazol-5-yl)-phenyl]-phenyl}-methylimidazol-5-carboxylat;
Pivaloyloxymethyl-2-ethoxymethyl-4-(1-hydroxy-1-methylethyl)-2-methoxymethyl-1-{4-[2-(tetrazol-5-yl)-phenyl]-phenyl}-methylimidazol-5-carboxylat;
Pivaloyloxymethyl-2-ethylthio-4-(1-hydroxy-1-methylethyl)-1-{4-[2-(tetrazol-5-yl)-phenyl]-phenyl}-methylimidazol-5-carboxylat;
(5-Methyl-2-oxo-1,3-dioxolen-4-yl)-methyl-(1-hydroxy-1-methylethyl)-2-methoxymethyl-1-{4-[2-(tetrazol-5-yl)-phenyl]-phenyl}-methylimidazol-5-carboxylat;
(5-Methyl-2-oxo-1,3-dioxolen-4-yl)-methyl-2-ethoxymethyl-4-(1-hydroxy-1-methylethyl)-1-{4-[2-(tetrazol-5-yl)-phenyl]-phenyl}-methylimidazol-5-carboxylat;
(5-Methyl-2-oxo-1,3-dioxolen-4-yl)-methyl-2-ethylthio-4-(1-hydroxy-1-methylethyl)-1-{4-[2-(tetrazol-5-yl)-phenyl]-phenyl}-methylimidazol-5-carboxylat;
Ethoxycarbonyloxymethyl-4-(1-hydroxy-1-methylethyl)-2-methoxymethyl-1-{4-(2-(tetrazol-5-yl)-phenyl]-phenyl}-methylimidazol-5-carboxylat;
Ethoxycarbonyloxymethyl-2-ethoxymethyl-4-(1-hydroxy-1-methylethyl)-1-{4-[2-(tetrazol-5-yl)-phenyl]-phenyl}-methylimidazol-5-carboxylat;
Ethoxycarbonyloxymethyl-2-ethylthio-4-(1-hydroxy-1-methylethyl)-1-{4-[2-(tetrazol-5-yl)-phenyl]-phenyl}-methylimidazol-5-carboxylat;
Isopropoxycarbonyloxymethyl-4-(1-hydroxy-1-methylethyl)-2-methoxymethyl-1-{4-[2-(tetrazol-5-yl)-phenyl]-phenyl}-methylimidazol-5-carboxylat;
Isopropoxycarbonyloxymethyl-2-ethoxymethyl-4-(1-hydroxy-1-methylethyl)-1-{4-[2-(tetrazol-5-yl)]-phenyl]-phenyl}-methylimidazol-5-carboxylat;
Isopropoxycarbonyloxymethyl-2-ethylthio-4-(1-hydroxy-1-methylethyl)-1-{4-[2-(tetrazol-5-yl)-phenyl]-phenyl}-methylimidazol-5-carboxylat;
und pharmazeutisch verträgliche Salze und Ester davon.

17. Pharmazeutische Zusammensetzung zur Therapie oder Prophylaxe von Hochdruck oder einer kardiovaskulären Krankheit, die eine wirksame Menge eines antihypertonischen Mittels im Gemisch mit einem pharmazeutisch verträglichen Trägerstoff oder Verdünnungsmittel enthält, wobei das antihypertonische Mittel unter Verbindungen der Formel (I) und pharmazeutisch verträglichen Salzen und Estern davon gemäß einem der Ansprüche 1 bis 16 ausgewählt ist.

18. Pharmazeutische Zusammensetzung zur Therapie oder Prophylaxe von Hochdruck oder einer kardiovaskulären Krankheit, die eine wirksame Menge eines antihypertonischen Mittels im Gemisch mit einem pharmazeutisch verträglichen Trägerstoff oder Verdünnungsmittel enthält, wobei das antihypertonische Mittel unter Verbindungen der Formel (Ia) und pharmazeutisch verträglichen Salzen und Estern davon gemäß Anspruch 2 ausgewählt ist.

19. Verbindungen der Formel (I) und pharmazeutisch verträgliche Salze und Ester davon nach einem der Ansprüche 1 bis 16 zur Verwendung als Arzneimittel.

20. Verbindungen der Formel (Ia) und pharmazeutisch verträgliche Salze und Ester davon nach Anspruch 2 zur Verwendung als Arzneimittel.

21. Verbindungen der Formel (I) und pharmazeutisch verträgliche Salze und Ester davon nach einem der Ansprüche 1 bis 16 zur Verwendung bei der Therapie oder Prophylaxe von Hochdruck oder einer kardiovaskulären Krankheit.

22. Verwendung einer Verbindung der Formel (I) nach einem der Ansprüche 1 bis 16 oder eines pharmazeutisch verträglichen Salzes oder Esters davon bei der Herstellung eines Arzneimittels zur Therapie oder Prophylaxe von Hochdruck oder einer kardiovaskulären Krankheit.

23. Verfahren zur Herstellung einer Verbindung der Formel (I) nach einem der Ansprüche 1 bis 16, umfassend die Umsetzung einer Verbindung der Formel (II) 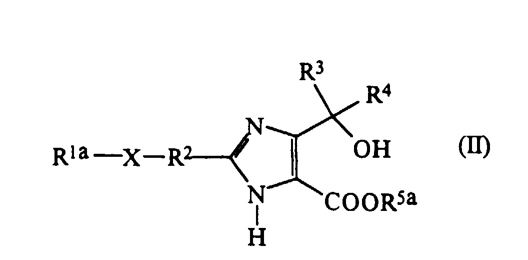 (in der
R^{2,} R³, R⁴ und X die vorstehend definierte Bedeutung haben,
R^{1a} folgende Bedeutungen hat:
wenn X ein Sauerstoffatom darstellt: ein Wasserstoffatom, einen Alkylrest mit 1 bis 6 Kohlenstoffatomen, einen Cycloalkylrest mit 3 bis 6 Kohlenstoffatomen oder einen Rest der Formel R⁷CO-, wobei R⁷ ein Wasserstoffatom, einen Alkylrest mit 1 bis 6 Kohlenstoffatomen oder einen Arylrest mit 6 bis 10 Ringkohlenstoffatomen bedeutet; oder
wenn X ein Schwefelatom bedeutet: einen Alkylrest mit 1 bis 6 Kohlenstoffatomen, einen Cycloalkylrest mit 3 bis 6 Kohlenstoffatomen, eine Mercaptoschutzgruppe oder den Rest der Formel R⁷CO-; und
R^{5a} eine Carboxylschutzgruppe bedeutet) mit einer Verbindung der Formel (III) : 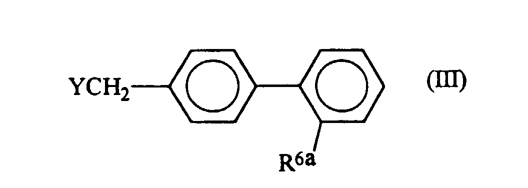 (in der Y ein Halogenatom bedeutet; und R^{6a} einen geschützten Carboxylrest, einen geschützten Tetrazol-5-ylrest, einen Cyanorest, einen Carbamoylrest oder einen Alkylcarbamoylrest bedeutet, wobei der Alkylteil 1 bis 6 Kohlenstoffatome aufweist)
unter Bildung einer Verbindung der Formel (Ib) 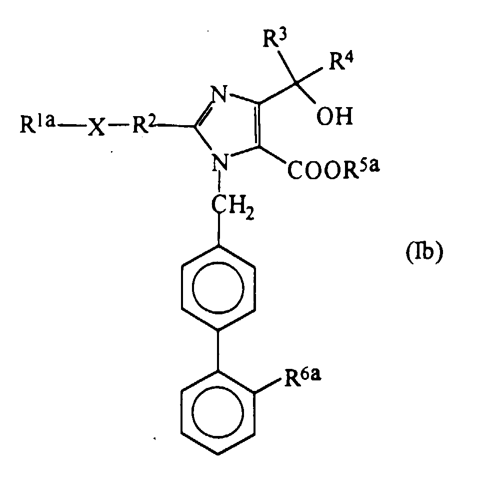 (in der R^{1a}, R², R³, R⁴, R^{5a}, R^{6a} und X die vorstehend definierten Bedeutungen haben);
und gegebenenfalls Umwandeln von etwaigen, durch R^{1a} oder durch R^{6a} wiedergegebenen Resten in einen durch R¹ bzw. R⁶ wiedergegebenen Rest;
und gegebenenfalls Entfernen von etwaigen Carboxylschutzgruppen und Salzbildung und/oder Veresterung der erhaltenen Verbindung.

## Revendications

1. Composé de formule (I) : 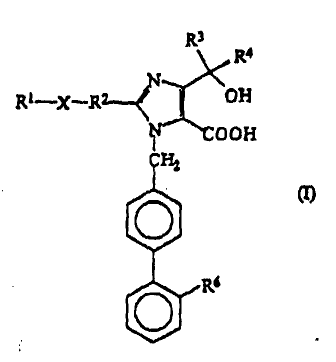 dans laquelle :
R¹ représente un atome d'hydrogène, un groupe alkyle ayant 1 à 6 atomes de carbone, un groupe cycloalkyle ayant 3 à 6 atomes de carbone cycliques ou un groupe alcanoyle ayant 1 à 6 atomes de carbone cycliques ;
R² représente une liaison simple ou un groupe alkylène ou alkylidène ayant 1 à 4 atomes de carbone ;
R³ et R⁴ sont identiques ou différents et chacun représente un groupe alkyle ayant 1 à 6 atomes de carbone ;
R⁶ représente un groupe carboxyle ou un groupe tétrazole-5-yle ; et
X représente un atome d'oxygène ou de soufre ; et ses sels et esters pharmaceutiquement acceptables.

2. Composé de la revendication 1, ledit composé ayant la formule (Ia) : 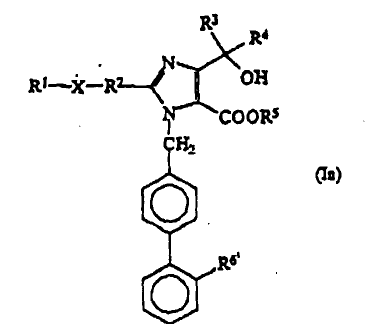 dans laquelle : R¹, R², R³, R⁴ et X sont tels que définis dans la revendication 1, R⁵ représente un atome d'hydrogène ou un groupe ester et R^{6'} représente un groupe carboxyle, un groupe carboxyle estérifié ou un groupe tétrazole-5-yle.

3. Composé de la revendication 1, dans lequel R¹ représente un atome d'hydrogène, un groupe méthyle, un groupe éthyle, un groupe cyclopropyle ou un groupe acétyle.

4. Composé de la revendication 1, dans lequel R² représente une liaison simple, un groupe méthylène, un groupe éthylène ou un groupe éthylidène.

5. Composé de la revendication 1, dans lequel R³ et R⁴ sont identiques ou différents et chacun représente un groupe méthyle ou un groupe éthyle.

6. Composé de la revendication 2, dans lequel R⁵ représente
un atome d'hydrogène,
un groupe alkyle ayant 1 à 4 atomes de carbone,
un groupe phényle,
un groupe phényle substitué par au moins un substituant choisi parmi les groupes méthyle, les groupes éthyle, les groupes méthoxy, les groupes éthoxy, les atomes de fluor et les atomes de chlore,
un groupe naphtyle,
un groupe benzyle,
un groupe benzyle substitué par au moins un substituant choisi parmi les groupes méthyle, les groupes éthyle, les groupes méthoxy, les groupes éthoxy, les atomes de fluor et les atomes de chlore,
un groupe diphénylméthyle,
un groupe naphtylméthyle,
un groupe alcanoyloxyalkyle dans lequel la portion alcanoyle compte 1 à 5 atomes de carbone et la portion alkyle compte 1 à 4 atomes de carbone,
un groupe cycloalcanecarbonyloxyalkyle dans lequel la portion cycloalcane compte 5 ou 6 atomes de carbone et la portion alkyle compte 1 à 4 atomes de carbone,
un groupe alcoxycarbonyloxyalkyle dans lequel les portions alcoxy et alkyle ont chacune 1 à 4 atomes de carbone,
un groupe cycloalkyloxycarbonyloxyalkyle dans lequel la portion cycloalkyle compte 5 ou 6 atomes de carbone et la portion alkyle compte 1 à 4 atomes de carbone,
un groupe (5-phényl-2-oxo-1,3-dioxolène-4-yl)méthyle,
un groupe (5-alkyl-2-oxo-1,3-dioxolène-4-yl)méthyle dans lequel la portion alkyle compte 1 à 4 atomes de carbone, ou
un groupe phtalidyle.

7. Composé de la revendication 2, dans lequel R^{6'} représente un groupe carboxyle ou un groupe tétrazole-5-yle.

8. Composé de la revendication 2, dans lequel :
R¹ représente un atome d'hydrogène, un groupe méthyle, un groupe éthyle, un groupe cyclopropyle ou un groupe acétyle ;
R² représente une liaison simple, un groupe méthylène, un groupe éthylène ou un groupe éthylidène ;
R³ et R⁴ sont identiques ou différents et chacun représente un groupe méthyle ou un groupe éthyle ;
R⁵ représente
un atome d'hydrogène,
un groupe alkyle ayant 1 à 4 atomes de carbone,
un groupe phényle,
un groupe phényle substitué par au moins un substituant choisi parmi les groupes méthyle, les groupes éthyle, les groupes méthoxy, les groupes éthoxy, les atomes de fluor et les atomes de chlore,
un groupe naphtyle,
un groupe benzyle,
un groupe benzyle substitué par au moins un substituant choisi parmi les groupes méthyle, les groupes éthyle, les groupes méthoxy, les groupes éthoxy, les atomes de fluor et les atomes de chlore,
un groupe diphénylméthyle,
un groupe naphtylméthyle,
un groupe alcanoyloxyalkyle dans lequel la portion alcanoyle compte 1 à 5 atomes de carbone et la portion alkyle compte 1 à 4 atomes de carbone,
un groupe cycloalcanecarbonyloxyalkyle dans lequel la portion cycloalcane compte 5 ou 6 atomes de carbone et la portion alkyle compte 1 à 4 atomes de carbone,
un groupe alcoxycarbonyloxyalkyle dans lequel les portions alcoxy et alkyle ont chacune 1 à 4 atomes de carbone,
un groupe cycloalkyloxycarbonyloxyalkyle dans lequel la portion cycloalkyle compte 5 ou 6 atomes de carbone et la portion alkyle compte 1 à 4 atomes de carbone,
un groupe (5-phényl-2-oxo-1,3-dioxolène-4-yl)méthyle,
un groupe (5-alkyl-2-oxo-1,3-dioxolène-4-yl)méthyle dans lequel la portion alkyle compte 1 à 4 atomes de carbone, ou
un groupe phtalidyle ; et
R^{6'} représente un groupe carboxyle ou un groupe tétrazole-5-yle.

9. Composé de la revendication 1, dans lequel le groupe de formule R¹-X-R²- représente un groupe méthoxyméthyle, un groupe éthoxyméthyle, un groupe 1-méthoxyéthyle, un groupe 2-méthoxyéthyle, un groupe 2-éthoxyéthyle, un groupe méthylthiométhyle, un groupe éthylthiométhyle, un groupe 1-méthylthioéthyle, un groupe 2-méthylthioéthyle, un groupe 2-éthylthioéthyle, un groupe méthylthio ou un groupe éthylthio.

10. Composé de la revendication 2, dans lequel R⁵ représente un atome d'hydrogène, un groupe alkyle ayant 1 à 4 atomes de carbone, un groupe benzyle, un groupe alcanoyloxyalkyle dans lequel la portion alcanoyle compte 1 à 5 atomes de carbone et la portion alkyle compte 1 ou 2 atomes de carbone, un groupe cycloalcanecarbonyloxyalkyle dans lequel la portion cycloalcane compte 5 ou 6 atomes de carbone et la portion alkyle compte 1 ou 2 atomes de carbone, un groupe alcoxycarbonyloxyalkyle dans lequel la portion alcoxy compte 1 à 4 atomes de carbone et la portion alkyle compte 1 ou 2 atomes de carbone, un groupe cycloalkyloxycarbonyloxyalkyle dans lequel la portion cycloalkyle compte 5 ou 6 atomes de carbone et la portion alkyle compte 1 ou 2 atomes de carbone, un groupe (5-phényl-, 5-méthyl- ou 5-éthyl-2-oxo-1,3-dioxolène-4-yl)méthyle ou un groupe phtalidyle.

11. Composé de la revendication 2, dans lequel :
le groupe de formule R¹-X-R²- représente un groupe méthoxy-méthyle, un groupe éthoxyméthyle, un groupe 1-méthoxy-éthyle, un groupe 2-méthoxyéthyle, un groupe 2-éthoxy-éthyle, un groupe méthylthiométhyle, un groupe éthyl-thiométhyle, un groupe 1-méthylthioéthyle, un groupe 2-méthylthioéthyle, un groupe 2-éthylthioéthyle, un groupe méthylthio ou un groupe éthylthio ;
R³ et R⁴ sont identiques ou différents et chacun représente un groupe méthyle ou éthyle ;
R⁵ représente un atome d'hydrogène, un groupe alkyle ayant 1 à 4 atomes de carbone, un groupe benzyle, un groupe alcanoyloxyalkyle dans lequel la portion alcanoyle compte 1 à 5 atomes de carbone et la portion alkyle compte 1 ou 2 atomes de carbone, un groupe cycloalcanecarbonyloxyalkyle dans lequel la portion cycloalcane compte 5 ou 6 atomes de carbone et la portion alkyle compte 1 ou 2 atomes de carbone, un groupe alcoxycarbonyloxyalkyle dans lequel la portion alcoxy compte 1 à 4 atomes de carbone et la portion alkyle compte 1 ou 2 atomes de carbone, un groupe cycloalkyloxycarbonyloxyalkyle dans lequel la portion cycloalkyle compte 5 ou 6 atomes de carbone et la portion alkyle compte 1 ou 2 atomes de carbone, un groupe (5-phényl-, 5-méthyl- ou 5-éthyl-2-oxo-1,3-dioxolène-4-yl)méthyle ou un groupe phtalidyle ; et
R^{6'} représente un groupe carboxyle ou un groupe tétrazole-5-yle.

12. Composé de la revendication 1, dans lequel le groupe de formule R¹-X-R²- représente un groupe méthoxy-méthyle, un groupe éthoxyméthyle, un groupe méthylthio-méthyle, un groupe méthylthio ou un groupe éthylthio.

13. Composé de la revendication 1, dans lequel R³ et R⁴ représentent tous deux des groupes méthyle.

14. Composé de la revendication 2, dans lequel R⁵ représente un atome d'hydrogène, un groupe pivaloyloxy-méthyle, un groupe éthoxycarbonyloxyméthyle, un groupe 1-(éthoxycarbonyloxy)éthyle, un groupe isopropoxycarbonyl-oxyméthyle, un groupe 1-(isopropoxycarbonyloxy)éthyle, un groupe (5-méthyl-2-oxo-1,3-dioxolène-4-yl)méthyle ou un groupe phtalidyle.

15. Composé de la revendication 2, dans lequel :
le groupe de formule R¹-X-R²- représente un groupe méthoxy-méthyle, un groupe éthoxyméthyle, un groupe méthylthio-méthyle, un groupe méthylthio ou un groupe éthylthio ;
R³ et R⁴ représentent tous deux des groupes méthyle ;
R⁵ représente un atome d'hydrogène, un groupe pivaloyloxy-méthyle, un groupe éthoxycarbonyloxyméthyle, un groupe 1-(éthoxycarbonyloxy)éthyle, un groupe isopropoxy-carbonyloxyméthyle, un groupe 1-(isopropoxycarbonyloxy)-éthyle, un groupe (5-méthyl-2-oxo-1,3-dioxolène-4-yl)-méthyle ou un groupe phtalidyle ; et
R^{6'} représente un groupe carboxyle ou un groupe tétrazole-5-yle.

16. Composé de la revendication 1, choisi parmi les suivants :
- acide 4-(1-hydroxy-1-méthyléthyl)-2-méthoxyméthyl-1-{4-[2-(tétrazole-5-yl)phényl]phényl}méthylimidazole-5-carboxylique ;
- acide 2-éthoxyméthyl-4-(1-hydroxy-1-méthyléthyl)-1-{4-[2-(tétrazole-5-yl)phényl]phényl}méthylimidazole-5-carboxylique ;
- acide 2-éthylthio-4-(1-hydroxy-1-méthyléthyl)-1-{4-[2-(tétrazole-5-yl)phényl]phényl}méthylimidazole-5-carboxylique ;
- 4-(1-hydroxy-1-méthyléthyl)-2-méthoxyméthyl-1-{4-[2-(tétrazole-5-yl)phényl]phényl}méthylimidazole-5-carboxylate de pivaloyloxyméthyle ;
- 2-éthoxyméthyl-4-(1-hydroxy-1-méthyléthyl)-1-{4-[2-(tétrazole-5-yl)phényl]phényl}méthylimidazole-5-carboxylate de pivaloyloxyméthyle ;
- 2-éthylthio-4-(1-hydroxy-1-méthyléthyl)-1-{4-[2-(tétrazole-5-yl)phényl]phényl}méthylimidazole-5-carboxylate de pivaloyloxyméthyle ;
- 4-(1-hydroxy-1-méthyléthyl)-2-méthoxyméthyl-1-{4-[2-(tétrazole-5-yl)phényl]phényl}méthylimidazole-5-carboxylate de (5-méthyl-2-oxo-1,3-dioxolène-4-yl)-méthyle ;
- 2-éthoxyméthyl-4-(1-hydroxy-1-méthyléthyl)-1-{4-[2-(tétrazole-5-yl)phényl]phényl}méthylimidazole-5-carboxylate de (5-méthyl-2-oxo-1,3-dioxolène-4-yl)-méthyle ;
- 2-éthylthio-4-(1-hydroxy-1-méthyléthyl)-1-{4-[2-(tétrazole-5-yl)phényl]phényl}méthylimidazole-5-carboxylate de (5-méthyl-2-oxo-1,3-dioxolène-4-yl)-méthyle ;
- 4-(1-hydroxy-1-méthyléthyl)-2-méthoxyméthyl-1-{4-[2-(tétrazole-5-yl)phényl]phényl}méthylimidazole-5-carboxylate d'éthoxycarbonyloxyméthyle ;
- 2-éthoxyméthyl-4-(1-hydroxy-1-méthyléthyl)-1-{4-[2-(tétrazole-5-yl)phényl]phényl}méthylimidazole-5-carboxylate d'éthoxycarbonyloxyméthyle ;
- 2-éthylthio-4-(1-hydroxy-1-méthyléthyl)-1-{4-[2-(tétrazole-5-yl)phényl]phényl}méthylimidazole-5-carboxylate d'éthoxycarbonyloxyméthyle ;
- 4-(1-hydroxy-1-méthyléthyl)-2-méthoxyméthyl-1-{4-[2-(tétrazole-5-yl)phényl]phényl}méthylimidazole-5-carboxylate d'isopropoxycarbonyloxyméthyle ;
- 2-éthoxyméthyl-4-(1-hydroxy-1-méthyléthyl)-1-{4-[2-(tétrazole-5-yl)phényl]phényl}méthylimidazole-5-carboxylate d'isopropoxycarbonyloxyméthyle ;
- 2-éthylthio-4-(1-hydroxy-1-méthyléthyl)-1-{4-[2-(tétrazole-5-yl)phényl]phényl}méthylimidazole-5-carboxylate d'isopropoxycarbonyloxyméthyle ;
et leurs sels et esters pharmaceutiquement acceptables.

17. Composition pharmaceutique pour le traitement ou la prophylaxie de l'hypertension ou d'une maladie cardiovasculaire, qui comprend une quantité efficace d'un agent antihypertenseur en mélange avec un support ou diluant pharmaceutiquement acceptable, dans laquelle l'agent antihypertenseur est choisi parmi les composés de formule (I) et leurs sels et esters pharmaceutiquement acceptables, tels que revendiqués dans l'une quelconque des revendications 1 à 16.

18. Composition pharmaceutique pour le traitement ou la prophylaxie de l'hypertension ou d'une maladie cardiovasculaire, qui comprend une quantité efficace d'un agent antihypertenseur en mélange avec un support ou diluant pharmaceutiquement acceptables, dans laquelle l'agent antihypertenseur est choisi parmi les composés de formule (la) et leurs sels et esters pharmaceutiquement acceptables, tels que revendiqués dans la revendication 2.

19. Composés de formule (I) et leurs sels et esters pharmaceutiquement acceptables, tels que revendiqués dans l'une quelconque des revendications 1 à 16, pour leur utilisation comme produit pharmaceutique.

20. Composés de formule (la) et leurs sels et esters pharmaceutiquement acceptables, tels que revendiqués dans la revendication 2, pour leur utilisation comme produit pharmaceutique.

21. Composés de formule (I) et leurs sels et esters pharmaceutiquement acceptables tels que définis dans l'une quelconque des revendications 1 à 16, pour leur utilisation dans le traitement ou la prophylaxie de l'hypertension ou d'une maladie cardiovasculaire.

22. Utilisation d'un composé de formule (I) tel que défini dans l'une quelconque des revendications 1 à 16, ou d'un sel ou ester pharmaceutiquement acceptable de celui-ci, dans la fabrication d'un médicament destiné au traitement ou à la prophylaxie de l'hypertension ou d'une maladie cardiovasculaire.

23. Procédé pour la préparation d'un composé de formule (I), tel que défini dans l'une quelconque des revendications 1 à 16, procédé qui comprend la réaction d'un composé de formule (II) : 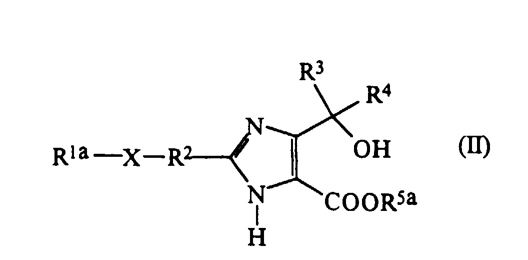 (dans laquelle :
R², R³, R⁴ et X sont tels que définis ci-dessus ;
R^{1a} représente,
lorsque X représente un atome d'oxygène : un atome d'hydrogène, un groupe alkyle ayant 1 à 6 atomes de carbone, un groupe cycloalkyle ayant 3 à 6 atomes de carbone ou un groupe de formule R⁷CO-, où R⁷ représente un atome d'hydrogène, un groupe alkyle ayant 1 à 6 atomes de carbone ou un groupe aryle ayant 6 à 10 atomes de carbone cycliques ; ou
lorsque X représente un atome de soufre : un groupe alkyle ayant 1 à 6 atomes de carbone, un groupe cycloalkyle ayant 3 à 6 atomes de carbone, un groupe protecteur de thiol ou ledit groupe de formule R⁷CO- ; et
R^{5a} représente un groupe protecteur de carboxyle) avec un composé de formule (III) : 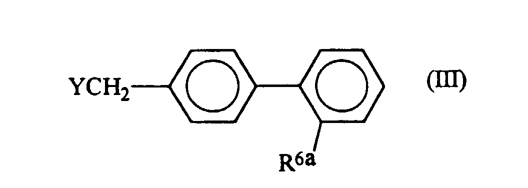 (dans laquelle Y représente un atome d'halogène ; et R^{6a} représente un groupe carboxyle protégé, un groupe tétrazole-5-yle protégé, un groupe cyano, un groupe carbamoyle ou un groupe alkylcarbamoyle dans lequel la portion alkyle compte 1 à 6 atomes de carbone) pour donner un composé de formule (Ib) : 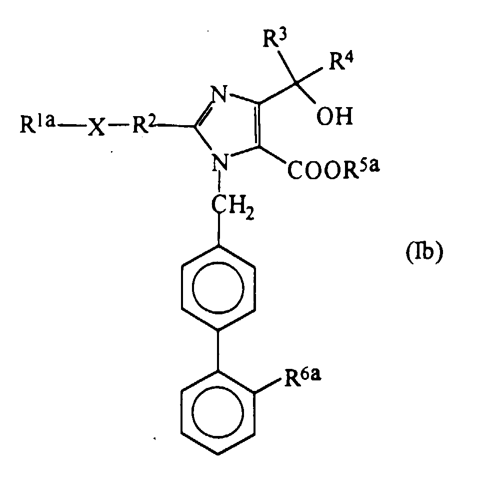 (dans laquelle R^{1a}, R², R³, R⁴, R^{5a}, R^{6a} et X sont tels que définis ci-dessus) ;
et, si nécessaire, la conversion de tout groupe représenté par R^{1a} ou R^{6a} en un groupe représenté par R¹ ou R⁶, respectivement ;
et, facultativement, l'élimination de tout groupe protecteur de carboxyle, une salification et/ou une estérification du composé résultant.
